# EUROPEAN PATENT APPLICATION

(11) **EP 4 640 699 A2**
(43) Date of publication of application: **29.10.2025**
(21) Application number: 25196505.9
(22) Date of filing: 17.06.2022
(51) Int. Cl.: C07K 14/81

(54) **RETINAL DISORDERS**

(30) Priority: 18.06.2021 GB 202108759; 25.03.2022 GB 202204231
(62) Divisional of application: 22735972.6
(71) Applicant: Ikarovec Limited, Colney Lane, Norwich NR4 7GJ (GB)
(72) Inventor: WIDDOWSON, Peter, Norwich, NR4 7GJ (GB); BINLEY, Katie, Norwich, NR4 7GJ (GB)
(74) Representative: Hutter, Anton

(57) **Abstract**

The invention relates to retinal disorders, and to genetic constructs and recombinant vectors comprising such constructs, and their use in gene therapy methods for treating, preventing or ameliorating a wide range of retinal disorders. The constructs and vectors are particularly useful for treating geographic atrophy (GA) and dry age-related macular degeneration (dry-AMD). The invention extends to the use of the constructs and vectors for reducing complement activation and retinal cell damage and loss. The invention also extends to pharmaceutical compositions *per se,* and their use in treating, preventing or ameliorating retinal disorders, and reducing complement activation and retinal cell damage and loss.

## Description

The present invention relates to retinal disorders, and to genetic constructs and recombinant vectors comprising such constructs, and their use in gene therapy methods for treating, preventing or ameliorating a wide range of retinal disorders. The constructs and vectors are particularly, although not exclusively, useful for treating geographic atrophy (GA) and dry age-related macular degeneration (dry-AMD). The invention also extends to the use of the constructs and vectors for reducing complement activation and retinal cell damage and loss. The invention also extends to pharmaceutical compositions *per se,* and their use in treating, preventing or ameliorating retinal disorders, and reducing complement activation and retinal cell damage and loss.

Worldwide, there are estimated to be around five million people with geographic atrophy (GA) [1], with over one million in the U.S. alone [2], equating to an average of 1 in every 29 people over the age of 75. GA is a chronic progressive degeneration of the macula, as part of late-stage age-related macular degeneration (AMD). The disease is characterised by localised atrophy of retinal tissue and chorio-capillaris, leading to central scotomas and permanent loss of visual acuity. Increasing age and a family history of AMD are the most widely recognised risk factors for GA [3]. Smoking, past or present, also significantly increases an individual's risk of developing GA [3,4] and to date, no studies have found any gender difference in GA disease prevalence [3,5]. The Age-Related Eye Disease Study found an increased risk of GA in users of thyroid hormones or antacids, and other studies have shown that patients with coronary heart disease, lens opacities, or previous cataract surgery have a greater risk of developing GA [6]. Nevertheless, the pathogenesis of GA remains unclear.

The natural course of AMD begins with early stages that are characterised by pigment displacement and the presence of drusen, a yellow deposit that forms between the retinal pigment epithelium (RPE) and Bruch's membrane [7,8]. Reticular pseudo-drusen is particularly associated with the development of GA [9,10]. Later stages of AMD are characterised by either choroidal neovascularization, or GA. GA is recognised as a sharply defined area in the posterior pole, with atrophy of the RPE, the overlying photoreceptors and the chorio-capillaris.

One major component of drusen is the accumulated auto-fluorescent membrane-bound lipofuscin [10]. Lipofuscin is often found in aging retinas and is notably elevated in GA patients within the RPE cell layer [11-13]. Lipofuscin is primarily responsible for the intrinsic fluorescence of the human ocular fundus, both of the normal aged retina and more intensely in patients with GA. [14,15]. Around the age of 90 years old, lipofuscin granules occupy approximately 20% of the area of the macular RPE cell [12]. As the atrophic area expands, visual function decreases [10,12,13,16]. Clinically, exudative and non-exudative AMD are very different, but these late stages of AMD are far from exclusive. Individuals with GA carry a higher risk of developing choroidal neovascularization, and patients with exudative AMD are at high risk of developing atrophic areas.

Lipofuscin contains a complex mixture of pigments, but the major component has been shown to be A2E (*N*-retinylidene-*N*-retinylethanolamine; and iso-A2E, which readily interconvert), formed from two molecules of all-trans-retinal or 11-cis-retinal and ethanolamine which are found in the photoreceptor outer segment membrane [17-22]. It has been shown that lipofuscin levels directly correlate with histopathologic damage of GA [12,13,22], indicating that lipofuscin is one of the major factors of GA. Not only does A2E act as a detergent, thereby interfering with normal RPE lysosomal activity, but A2E can interfere with cholesterol metabolism [23] and lead to oxidative damage.

Several unsuccessful approaches to reduce levels of A2E and drusen accumulation have been attempted using drugs that slow the biochemical visual cycle. For example, Fenretinide (ReVision Therapeutics), an oral Retinal-Binding Protein (RBP4)-transthyretin-retinol complex inhibitor, failed to slow GA progression in a Phase-2 clinical study. In addition, the RPE65 isomerase inhibitor, ACU-4426 (emixustat hydrochloride, Acucela), whilst reducing A2E accumulation in the ABCA4/RDH8 double knockout mouse model [24,25], failed to halt GA progression in Phase-2b/3 clinical studies [26].

The complement system (CS) is part of the innate immune system to defend against foreign pathogens, such as microbes [27-29], and may play a role in drusen reduction. For example, Complement factor H variants, Y402H and ARMS2, have been associated with increased risk of GA development [30,31] and drusen has been shown to contain multiple complement components in addition to lipofuscin [27-33]. This indicates that localised inflammation, mediated by the complement system, may be an important element in AMD.

The CS consists of three biochemical pathways: classical pathway (CP), lectin pathway (LP) and alternative pathway (AP), each of which has a distinct trigger [27-29]. Whilst each pathway can be activated by separate components, the pathways converge to involve a key protein component called complement factor 3 (C3).

The classical pathway (CP) is activated by antigen-antibody complexes, specifically via the interaction between C1q and antibodies. After activation of the C1 complex, complement C2 and C4 are cleaved into C2a, C2b, C4a and C4b. The C4b and C2a proteins then combine into the C4b2a complex, which is a C3 convertase. The C4b2a complex further binds to C3b to form C5 convertase.

The lectin pathway (LP) is usually activated by binding mannose residues on the microbial surface, either via mannose-binding lectin or ficolin, which resemble C1q, C1r and C1s, to form Mannan-binding lectin-associated serine proteases (MASPs). Three MASPs have been characterised to date; MASP-1, MASP-2 and MASP-3. MASP-2 is similar to C1s in catalytic specificity and is able to cleave C2 and C4.

The alternative pathway (AP) is implicated closely with the pathogenesis of AMD. In the AP, Complement Factors B, P (properdin) and D are involved in the activation of C3, via C3b and activator factor B (Bb), which form the C3 convertase complex. C3 then divides into C3a and C3b. C3b can bind to the C3a receptor on immune cells causing inflammation, thus allowing the infiltration of immune cells to further neutralise the pathogen. The C3b component further amplifies the C3 convertase activity and also forms a complex with either C4b and C2a (C4b-C2a-C3b) or Bb (C3b-Bb-C3b), which both act as a C5 convertase to generate C5a and C5b from the C5 protein. The C5a can then bind to the C5b receptor on immune cells, similar to C3a, whilst the C5b forms a complex with C6, C7, C8 and C9 (S5b-9), forming a membrane attack complex (MAC) or terminal complement complex (TCC). The TCC is capable of destroying the invading microbe through cell swelling and eventual lysis.

Activity of the AP can be modulated by Complement factor I (CFI) [34,35], which is also called the C3b/C4b inactivator because it cleaves the cell-bound or fluid phase of C3b and C4b. Another modulating factor is CD55 or decay-accelerating factor (DAF) [36-39], which is a membrane bound protein that protects cells from complement-mediated lysis. CD55's primary function is to inactivate the C3 convertases by dissociating them into their constituent proteins [36-39]. A further modulatory protein called complement factor H-related protein-1 (CFHR1), which is a splice variant of complement factor H [40-43], is also involved in reducing complement activation. The prominent regulator is complement factor H and its spliced product FHL-1, which regulate complement activity in the fluid phase and on surfaces by decay acceleration and cofactor activities. Factor H and FHL-1 target and decay the C3 convertase, composed of C3b and factor B. Another factor capable of reducing complement activation is called Membrane Cofactor Protein or CD46 [44,46] which is membrane bound. Once bound to C3b, factor H and CFHL-1 occupy the factor B binding site in C3b, accelerate the decay and prevent the formation of new C3 convertases. Furthermore, factor H, CFHL-1 and CD46 act as cofactors for the protease factor I that cleaves C3b to iC3b as well as for C4b in the case of CD46. CFHR-1 does not mediate decay-accelerating or factor I cofactor activity. Like factor H, CFHR-1 binds to C3b and recognises self-surfaces by binding to glycosaminoglycan and inhibits the C5 convertase and terminal complement complex formation [43].

Despite evidence for a key role of the complement system in mediating GA, trials of agents which modulate the complement system have shown mixed results. For example, the monoclonal antibodies eculizumab (Aexion) and lampalizumab [44] (Genentech/Roche) were unsuccessful in slowing GA progression in the clinic. Additionally, Gyroscope Therapeutics generated a Complement factor I gene therapy into a clinical trial for GA (GT005) [47]. However, whilst boosting CFI in patients with low or absent CFI would theoretically reduce complement activation, all three pathways would be affected with this treatment, including the CP and LP, which are responsible for anti-microbial activity. Therefore, a systemic reduction in all of the complement system pathways may leave eyes vulnerable to bacterial infections, or it would take longer for infected eyes to clear. Additionally, early clinical trials with anti-complement monotherapy, e.g. avacincaptad pegol and the complement factor I gene therapy have witnessed some GA patients developing wet-AMD, which has required them to need monthly anti-VEGF injections of ranibizumab or aflibercept, to prevent rapid vision loss.

PEDF is a 50 kDa protein released apicolaterally in large quantities from RPE cells [48,49] and displays retinal neuroprotective properties through interaction with the PEDF receptor [50,51]. PEDF has several functions in the retina, namely it is anti-angiogenic and displays anti-tumorigenic, and neurotrophic properties [50-56], partly through inhibition of endothelial cell migration [54]. There is a high density of PEDF receptors in the RPE cell layer [57], suggesting an autocrine and paracrine neuroprotective role in the retina. In addition, PEDF has been demonstrated to protect human RPE cells against oxidative stress (a phenomenon proposed in retinas of GA patients as a result of lipofuscin accumulation) via up-regulation of Uncoupling Protein-2 (UCP-2) [55,58]. Accordingly, the inventors deduced that a reduction in retinal concentrations of PEDF in GA patients would leave the retinal pigment epithelium and photoreceptors extremely vulnerable to damage from oxidative stress and complement attack.

There is, therefore, a significant need for an improved therapy for the treatment of retinal disorders, such as geographic atrophy (GA) and dry age-related macular degeneration (dry-AMD), which can activate PEDF receptors and neutralise or attenuate the complement system.

Using significant inventive endeavour, the inventors have carefully designed and constructed a novel genetic construct, which encodes a PEDF receptor agonist and an anti-complement protein, under the control of a single promoter, i.e. it is bicistronic. The promoter of the construct may be used to ensure that both the agonist of the PEDF receptor and the anti-complement protein are maximally expressed to reduce retinal cell damage and loss through two independent pathophysiological pathways, namely increasing activity at PEDF receptors and reducing complement activation.

Thus, according to a first aspect of the invention, there is provided a genetic construct comprising a promoter operably linked to a first coding sequence, which encodes an agonist of the PEDF receptor, and a second coding sequence, which encodes an anti-complement protein.

The PEDF receptor agonist restores concentrations of PEDF, thereby reducing inflammation, reducing levels of toxic lipofuscin components and preserving RPE and photoreceptor cells. Additionally, the anti-complement protein is capable of neutralising or attenuating the alternative complement pathway, thereby arresting further RPE cell loss. Advantageously, the genetic construct of the invention targets the AP pathway, meaning the classical and lectin pathways of the complement system are preserved, such that the anti-microbial defence system, which can facilitate destruction of an invading pathogen, is maintained. Importantly, the combination of anti-complement with PEDF receptor activation will limit retinal VEGF release, which would otherwise have the potential of converting dry-AMD to the much more aggressive wet-AMD pathophysiology. Furthermore, the promoter of the construct may be used to ensure that both the PEDF receptor agonist and the anti-complement protein are maximally expressed to restore concentrations of PEDF and reduce inflammation, and neutralise the complement pathway to arrest further RPE cell loss.

The inventors have surprisingly demonstrated in the Examples that it is possible to combine the open reading frames (ORFs) which code for both the PEDF receptor agonist and anti-complement protein, in a single genetic construct. This was especially challenging given the large size of the PEDF receptor agonist and anti-complement protein. It could not have been predicted that it would have been possible to co-express both of these large proteins in physiologically useful concentrations from a single expression cassette, under the control of a single promoter, and for that expression cassette to be accommodated by an AAV vector (such as an rAAV-2 vector). Advantageously, with the construct of the invention, there is no need to inject a recombinant protein, as described in the prior art. Furthermore, in the prior art, it is still necessary to perform regular injections of protein into the eye, which is clearly disadvantageous, whereas the construct of the invention surprisingly only requires a single administration to achieve long-term therapeutic effect, thereby providing significant benefits to patients.

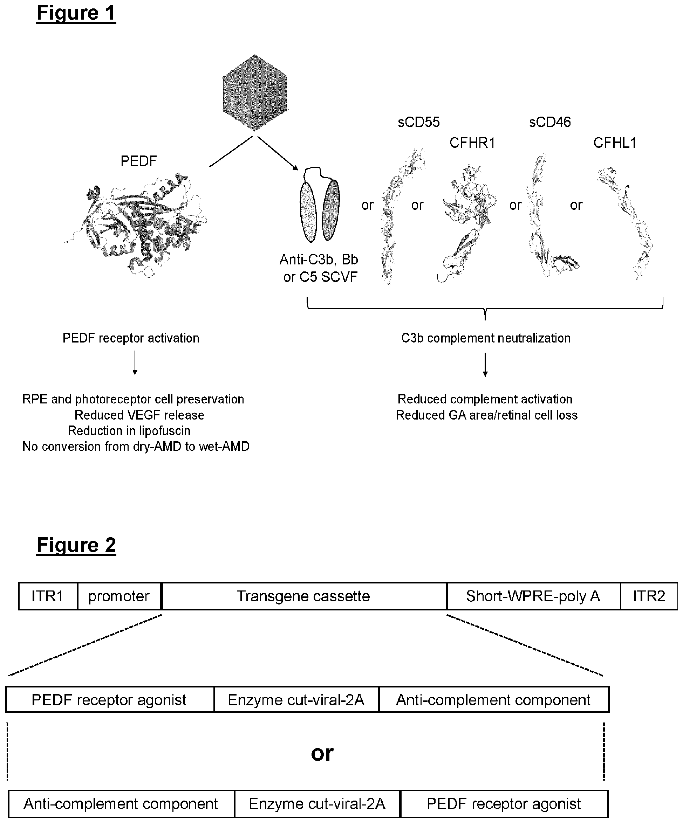

Preferably, the genetic construct of the invention comprises an expression cassette, one embodiment of which is shown in Figure 2. As can be seen in Figure 2, in one embodiment, the construct comprises the promoter, the first nucleotide sequence encoding the PEDF receptor agonist, and the second nucleotide sequence encoding the anti-complement protein. Thus, preferably the genetic construct and expression cassette may be referred to as being bi-cistronic.

As illustrated in Figure 2, the first and second coding sequences encoding the PEDF receptor agonist and anti-complement protein may be disposed in any order from the 5' to the 3'. For example, in one embodiment, the coding sequence for the PEDF receptor agonist is disposed 5' of the coding sequence for the anti-complement protein, preferably with a spacer sequencer therebetween. Alternatively, in another embodiment, the coding sequence for the anti-complement protein may be disposed 5' of the coding sequence for the PEDF receptor agonist, preferably with a spacer sequence therebetween.

The promoter in the genetic construct of the first aspect may be any nucleotide sequence that is capable of inducing RNA polymerase to bind to and transcribe the first and second coding sequence.

The promoter may be constitutive or tissue-specific.

A suitable constitutive promoter may be the cytomegalovirus promoter. One embodiment of the nucleotide sequence (508 bp) encoding the cytomegalovirus (CMV) promoter is referred to herein as SEQ ID No: 1, as follows:

CGTTACATAACTTACGGTAAATGGCCCGCCTGGCTGACCGCCCAACGACCCCCGCCCATTGACGTCAATAATGACGTA
TGTTCCCATAGTAACGCCAATAGGGACTTTCCATTGACGTCAATGGGTGGAGTATTTACGGTAAACTGCCCACTTGGC
AGTACATCAAGTGTATCATATGCCAAGTACGCCCCCTATTGACGTCAATGACGGTAAATGGCCCGCCTGGCATTATGC
CCAGTACATGACCTTATGGGACTTTCCTACTTGGCAGTACATCTACGTATTAGTCATCGCTATTACCATGGTGATGCG
GTTTTGGCAGTACATCAATGGGCGTGGATAGCGGTTTGACTCACGGGGATTTCCAAGTCTCCACCCCATTGACGTCAA
TGGGAGTTTGTTTTGGCACCAAAATCAACGGGACTTTCCAAAATGTCGTAACAACTCCGCCCCATTGACGCAAATGGG
CGGTAGGCGTGTACGGTGGGAGGTCTATATAAGCAGAGCT
[SEQ ID No: 1]

In another embodiment, the promoter is preferably a truncated form of the CMV promoter. One embodiment of the nucleotide sequence (60 bp) encoding the truncated form of the CMV promoter is referred to herein as SEQ ID No: 2, as follows:
AGGTAGGCGTGTACGGTGGGAGGTCTATATAAGCAGAGCTGGTTTAGTGAACCGTCAGAT
[SEQ ID No: 2]

In another embodiment, the promoter is a fusion of the cytomegalovirus (CMV) early enhancer element and the first intron of the chicken beta-actin gene (CAG). One embodiment of the nucleotide sequence (583 bp) encoding the cytomegalovirus early enhancer element and the first intron of chicken beta-actin gene is referred to herein as SEQ ID No: 3, as follows:

CGTTACATAACTTACGGTAAATGGCCCGCCTGGCTGACCGCCCAACGACCCCCGCCCATTGACGTCAATAATGACGTATGT
TCCCATAGTAACGCCAATAGGGACTTTCCATTGACGTCAATGGGTGGACTATTTACGGTAAACTGCCCACTTGGCAGTACA
TCAAGTGTATCATATGCCAAGTACGCCCCCTATTGACGTCAATGACGGTAAATGGCCCGCCTGGCATTATGCCCAGTACAT
GACCTTATGGGACTTTCCTACTTGGCAGTACATCTACGTATTAGTCATCGCTATTACCATGGTCGAGGTGAGCCCCACGTT
CTGCTTCACTCTCCCCATCTCCCCCCCCTCCCCACCCCCAATTTTGTATTTATTTATTTTTTAATTATTTTGTGCAGCGAT

GGGGGCGGGGGGGGGGGGGGGGCGCGCGCCAGGCGGGGCGGGGCGGGGCGAGGGGCGGGGCGGGGCGAGGCGGAGAGGTGC
GGCGGCAGCCAATCAGAGCGGCGCGCTCCGAAAGTTTCCTTTTATGGCGAGGCGGCGGCGGCGGCGGCCCTATAAAAAGCG
AAGCGCGCGGCGGGCG
[SEQ ID No: 3]

A suitable tissue-specific promoter may be the vitelliform macular dystrophy protein-2 (VMD2) promoter (sometimes referred to as the bestrophin-1). Advantageously, this promoter restricts transgene expression to the RPE cells. One embodiment of the nucleotide sequence (2039 bp) encoding the VMD2 promoter is referred to herein as SEQ ID No: 4, as follows:

AATTCTGTCATTTTACTAGGGTGATGAAATTCCCAAGCAACACCATCCTTTTCAGATAAGGGCACTGAGGCTGAGAGA
GGAGCTGAAACCTACCCGGCGTCACCACACACAGGTGGCAAGGCTGGGACCAGAAACCAGGACTGTTGACTGCAGCCC
GGTATTCATTCTTTCCATAGCCCACAGGGCTGTCAAAGACCCCAGGGCCTAGTCAGAGGCTCCTCCTTCCTGGAGAGT
TCCTGGCACAGAAGTTGAAGCTCAGCACAGCCCCCTAACCCCCAACTCTCTCTGCAAGGCCTCAGGGGTCAGAACACT
GGTGGAGCAGATCCTTTAGCCTCTGGATTTTAGGGCCATGGTAGAGGGGGTGTTGCCCTAAATTCCAGCCCTGGTCTC
AGCCCAACACCCTCCAAGAAGAAATTAGAGGGGCCATGGCCAGGCTGTGCTAGCCGTTGCTTCTGAGCAGATTACAAG
AAGGGACTAAGACAAGGACTCCTTTGTGGAGGTCCTGGCTTAGGGAGTCAAGTGACGGCGGCTCAGCACTCACGTGGG
CAGTGCCAGCCTCTAAGAGTGGGCAGGGGCACTGGCCACAGAGTCCCAGGGAGTCCCACCAGCCTAGTCGCCAGACCT
TCTGTGGGATCATCGGACCCACCTGGAACCCCACCTGTGAGTACAAGGTGCCCCAGGTGGACTGGGCTGGGGCTTTGA
GGCCTTCAGGGTTGGATGGCCATCTTGCGTATTTGTGTGGGATATGCACACACAGGCAGCACATGCGCAGGTGTGTGG
GCACCTGTGTGTCTGTGCAAATGCCCTGAGGTGGGAATGAGCTTGGTGTGCATCAGGCACAGCCAGCCAGTGTGGCTG
CAGCAAAACACACAGGGAAAGAATGGAGGGGGCATCAATCACTGCTTCAGTAAATTTTTATTGAGCGCCTTCTACGAG
AACACAAGAGGAGCTTCCATTCTGAGGAGGAAACAGGCAGGAAACAGGCAGATATCCTGTATAATTTCAAGTAGTGAT
AAGTGCTCTCTAGAAATATCAAGCAAGGTGAGGAGACACAGAGCACCGGTGGCAGTGGGGCTCTATTTCCAGGTTGGA
TGGTTGGGAACATCCTTTCTAAAGGGAACCTGGAGTGGGAAGGAACCATGCAGGTATCTCAGGAAGAGCTTCCTCCAG
GCAGGAAGATCAGCAGGTGGAAAGGCCCTGGAGCCACCATTCAGTAAACATCATTTGAGCATCTCTACCAGCTAGGTT
CCATTATGGGAATGGGAATATGGTGGTGGACAGGGCTGCCTGGTCCCTTCCATACTTCTCACACTAGGGTGGTTGAGA
GAGCTTGGGAGCTAACGAACAAGATGGGCTGAGAACACTGCCTAGCCCAGAGGACCTGAGCTTAGTGTGTAGACATTG
CTGCTGTTACTGCCTTTGTCATTGTATTATTTATTTATTTATTTATTTTAGACAGAGTTTTGCTCTTCTTACC
CAGGCTGGAGTGCAATGGCGTGATCTCAGCTCACTGCAACCTCCACCTCCTGGGTTCAAGCGATTCTCCTGCCTCAGC
CTCCTGAGTAGCTGGGATTACAGGCACCCGCACCACGCCTGGATAATTTTTTTGTATTTTTAGTAGAGACAGGGTTTC
ACCATGTTGGCCAGGCTGGTCTCGAACTCCTGACCTTAGGTGATCCACCTGCCTCGACTTCCCAAAGTGCTGGGATTA
TAGGCATGAGCCACTGCGCCCAGTGATTATAGAAAGTTAAAGGCACATGGCAATGCACACGCCTATCTACGTCTTCCC
TGCCAAAGCAAAGGGCAGCCTCTGGGCTCACTTTCTTGCGTTTCTACTTCCAAAAGGCAGTCAGAACTGGCAGGGCCT
TGGAGACCACTTCATCCACCTCCTAGGGTCCCTATGGGAGAGTTGAGGTCCAGAGCAGGGAAGGGTCCTGACAGGCTC
TGACCAGGGCCTCTGATCCCTACAAACCCCCAATCGGTGTCCCTCTCTACCAGGACCCAAGCCCACCTGCTGCAGCCC
ACTGCCTGGCC
[SEQ ID No: 4]

In yet a further preferred embodiment, the promoter is a truncated form of the VMD2 promoter. One embodiment of the nucleotide sequence (623 bp) encoding the truncated form of the VMD2 promoter is referred to herein as SEQ ID No: 5, as follows:

AATTCTGTCATTTTACTAGGGTGATGAAATTCCCAAGCAACACCATCCTTTTCAGATAAGGGCACTGAGGCTGAGAGA
GGAGCTGAAACCTACCCGGCGTCACCACACACAGGTGGCAAGGCTGGGACCAGAAACCAGGACTGTTGACTGCAGCCC
GGTATTCATTCTTTCCATAGCCCACAGGGCTGTCAAAGACCCCAGGGCCTAGTCAGAGGCTCCTCCTTCCTGGAGAGT
TCCTGGCACAGAAGTTGAAGCTCAGCACAGCCCCCTAACCCCCAACTCTCTCTGCAAGGCCTCAGGGGTCAGAACACT
GGTGGAGCAGATCCTTTAGCCTCTGGATTTTAGGGCCATGGTAGAGGGGGTGTTGCCCTAAATTCCAGCCCTGGTCTC
AGCCCAACACCCTCCAAGAAGAAATTAGAGGGGCCATGGCCAGGCTGTGCTAGCCGTTGCTTCTGAGCAGATTACAAG
AAGGGACTAAGACAAGGACTCCTTTGTGGAGGTCCTGGCTTAGGGAGTCAAGTGACGGCGGCTCAGCACTCACGTGGG
CAGTGCCAGCCTCTAAGAGTGGGCAGGGGCACTGGCCACAGAGTCCCAGGGAGTCCCACCAGCCTAGTCGCCAGACC
[SEQ ID No: 5]

In yet another preferred embodiment, the nucleotide sequence (462 bp) encoding the truncated form of the VMD2 promoter is referred to herein as SEQ ID No: 6, as follows:

TCATTCTTTCCATAGCCCACAGGGCTGTCAAAGACCCCAGGGCCTAGTCAGAGGCTCCTCCTTCCTGGAGAGTTCCTG
GCACAGAAGTTGAAGCTCAGCACAGCCCCCTAACCCCCAACTCTCTCTGCAAGGCCTCAGGGGTCAGAACACTGGTGG
AGCAGATCCTTTAGCCTCTGGATTTTAGGGCCATGGTAGAGGGGGTGTTGCCCTAAATTCCAGCCCTGGTCTCAGCCC
AACACCCTCCAAGAAGAAATTAGAGGGGCCATGGCCAGGCTGTGCTAGCCGTTGCTTCTGAGCAGATTACAAGAAGGG
ACTAAGACAAGGACTCCTTTGTGGAGGTCCTGGCTTAGGGAGTCAAGTGACGGCGGCTCAGCACTCACGTGGGCAGTG
CCAGCCTCTAAGAGTGGGCAGGGGCACTGGCCACAGAGTCCCAGGGAGTCCCACCAGCCTAGTCGCCAGACC
[SEQ ID No: 6]

In another embodiment, the promoter is the human phosphoglycerate kinase-1 (PGK) promoter. One embodiment of the nucleotide sequence (500 bp) encoding the human PGK-1 promoter is referred to herein as SEQ ID No: 7, as follows:

GGGTAGGGGAGGCGCTTTTCCCAAGGCAGTCTGGAGCATGCGCTTTAGCAGCCCCGCTGGGCACTTGGCGCTACACAAGTG
GCCTCTGGCCTCGCACACATTCCACATCCACCGGTAGGCGCCAACCGGCTCCGTTCTTTGGTGGCCCCTTCGCGCCACCTT
CTACTCCTCCCCTAGTCAGGAAGTTCCCCCCCGCCCCGCAGCTCGCGTCGTGCAGGACGTGACAAATGGAAGTAGCACGTC
TCACTAGTCTCGTGCAGATGGACAGCACCGCTGAGCAATGGAAGCGGGTAGGCCTTTGGGGCAGCGGCCAATAGCAGCTTT
GCTCCTTCGCTTTCTGGGCTCAGAGGCTGGGAAGGGGTGGGTCCGGGGGCGGGCTCAGGGGCGGGCTCAGGGGCGGGGCGG
GCGCCCGAAGGTCCTCCGGAGGCCCGGCATTCTGCACGCTTCAAAAGCGCACGTCTGCCGCGCTGTTCTCCTCTTCCTCAT
CTCCGGGCCTTTCG
[SEQ ID No: 7]

In a further embodiment, the promoter is EF1α derived from the human *EEF1A1* gene that expresses the alpha subunit of eukaryotic elongation factor 1. One embodiment of the nucleotide sequence (1182 bp) encoding the EF1α promoter is referred to herein as SEQ ID No: 8, as follows:

GCTCCGGTGCCCGTCAGTGGGCAGAGCGCACATCGCCCACAGTCCCCGAGAAGTTGGGGGGAGGGGTCGGCAATTGAACCG
GTGCCTAGAGAAGGTGGCGCGGGGTAAACTGGGAAAGTGATGTCGTGTACTGGCTCCGCCTTTTTCCCGAGGGTGGGGGAG
AACCGTATATAAGTGCAGTAGTCGCCGTGAACGTTCTTTTTCGCAACGGGTTTGCCGCCAGAACACAGGTAAGTGCCGTGT
GTGGTTCCCGCGGGCCTGGCCTCTTTACGGGTTATGGCCCTTGCGTGCCTTGAATTACTTCCACGCCCCTGGCTGCAGTAC
GTGATTCTTGATCCCGAGCTTCGGGTTGGAAGTGGGTGGGAGAGTTCGAGGCCTTGCGCTTAAGGAGCCCCTTCGCCTCGT
GCTTGAGTTGAGGCCTGGCTTGGGCGCTGGGGCCGCCGCGTGCGAATCTGGTGGCACCTTCGCGCCTGTCTCGCTGCTTTC
GATAAGTCTCTAGCCATTTAAAATTTTTGATGACCTGCTGCGACGCTTTTTTTCTGGCAAGATAGTCTTGTAAATGCGGGC
CAAGATCTGCACACTGGTATTTCGGTTTTTGGGGCCGCGGGCGGCGACGGGGCCCGTGCGTCCCAGCGCACATGTTCGGCG
AGGCGGGGCCTGCGAGCGCGGCCACCGAGAATCGGACGGGGGTAGTCTCAAGCTGGCCGGCCTGCTCTGGTGCCTGGCCTC
GCGCCGCCGTGTATCGCCCCGCCCTGGGCGGCAAGGCTGGCCCGGTCGGCACCAGTTGCGTGAGCGGAAAGATGGCCGCTT
CCCGGCCCTGCTGCAGGGAGCTCAAAATGGAGGACGCGGCGCTCGGGAGAGCGGGCGGGTGAGTCACCCACACAAAGGAAA
AGGGCCTTTCCGTCCTCAGCCGTCGCTTCATGTGACTCCACGGAGTACCGGGCGCCGTCCAGGCACCTCGATTAGTTCTCG
AGCTTTTGGAGTACGTCGTCTTTAGGTTGGGGGGAGGGGTTTTATGCGATGGAGTTTCCCCACACTGAGTGGGTGGAGACT
GAAGTTAGGCCAGCTTGGCACTTGATGTAATTCTCCTTGGAATTTGCCCTTTTTGAGTTTGGATCTTGGTTCATTCTCAAG
CCTCAGACAGTGGTTCAAAGTTTTTTTCTTCCATTTCAGGTGTCGTGA
[SEQ ID No: 8]

In a further embodiment, the promoter is EF1α without the large intron. One embodiment of the nucleotide sequence (230 bp) encoding the EF1α promoter is referred to herein as SEQ ID No: 9, as follows:

GCTCCGGTGCCCGTCAGTGGGCAGAGCGCACATCGCCCACAGTCCCCGAGAAGTTGGGGGGAGGGGTCGGCAATTGAACCG
GTGCCTAGAGAAGGTGGCGCGGGGTAAACTGGGAAAGTGATGTCGTGTACTGGCTCCGCCTTTTTCCCGAGGGTGGGGGAG
AACCGTATATAAGTGCAGTAGTCGCCGTGAACGTTCTTTTTCGCAACGGGTTTGCCGCCAGAACACAG
[SEQ ID No: 9]

Therefore, preferably the promoter comprises a nucleic acid sequence substantially as set out in SEQ ID No: 1, 2, 3, 4, 5, 6, 7, 8, or 9, or a fragment or variant thereof.

To ensure survival of damaged and vulnerable RPE cells in patients with GA, the inventors have incorporated a PEDF receptor agonist into the genetic construct of the invention. The inventors have carefully considered the sequences of the PEDF receptor agonist, and have produced several preferred embodiments of the protein that may be encoded by the first coding sequence in the genetic construct of the first aspect.

In one embodiment, the first coding sequence comprises a nucleotide sequence encoding a PEDF protein. Preferably, the PEDF protein is the human PEDF protein. Preferably, the human PEDF protein comprises an amino acid sequence (418 residues) referred to herein as SEQ ID No: 10, or a fragment or variant thereof, as follows:







[SEQ ID No: 10]

Preferably, in this embodiment, the first coding sequence comprises a nucleotide sequence (1254 bp) referred to herein as SEQ ID No: 11, as follows:

ATGCAGGCCCTGGTGCTACTCCTCTGCATTGGAGCCCTCCTCGGGCACAGCAGCTGCCAGAACCCTGCCAGCCCCCCG
GAGGAGGGCTCCCCAGACCCCGACAGCACAGGGGCGCTGGTGGAGGAGGAGGATCCTTTCTTCAAAGTCCCCGTGAAC
AAGCTGGCAGCGGCTGTCTCCAACTTCGGCTATGACCTGTACCGGGTGCGATCCAGCACGAGCCCCACGACCAACGTG
CTCCTGTCTCCTCTCAGTGTGGCCACGGCCCTCTCGGCCCTCTCGCTGGGAGCGGAGCAGCGAACAGAATCCATCATT
CACCGGGCTCTCTACTATGACTTGATCAGCAGCCCAGACATCCATGGTACCTATAAGGAGCTCCTTGACACGGTCACT
GCCCCCCAGAAGAACCTCAAGAGTGCCTCCCGGATCGTCTTTGAGAAGAAGCTGCGCATAAAATCCAGCTTTGTGGCA
CCTCTGGAAAAGTCATATGGGACCAGGCCCAGAGTCCTGACGGGCAACCCTCGCTTGGACCTGCAAGAGATCAACAAC
TGGGTGCAGGCGCAGATGAAAGGGAAGCTCGCCAGGTCCACAAAGGAAATTCCCGATGAGATCAGCATTCTCCTTCTC
GGTGTGGCGCACTTCAAGGGGCAGTGGGTAACAAAGTTTGACTCCAGAAAGACTTCCCTCGAGGATTTCTACTTGGAT
GAAGAGAGGACCGTGAGGGTCCCCATGATGTCGGACCCTAAGGCTGTTTTACGCTATGGCTTGGATTCAGATCTCAGC
TGCAAGATTGCCCAGCTGCCCTTGACCGGAAGCATGAGTATCATCTTCTTCCTGCCCCTGAAAGTGACCCAGAATTTG
ACCTTGATAGAGGAGAGCCTCACCTCCGAGTTCATTCATGACATAGACCGAGAACTGAAGACCGTGCAGGCGGTCCTC
ACTGTCCCCAAGCTGAAGCTGAGTTATGAAGGCGAAGTCACCAAGTCCCTGCAGGAGATGAAGCTGCAATCCTTGTTT
GATTCACCAGACTTTAGCAAGATCACAGGCAAACCCATCAAGCTGACTCAGGTGGAACACCGGGCTGGCTTTGAGTGG
AACGAGGATGGGGCGGGAACCACCCCCAGCCCAGGGCTGCAGCCTGCCCACCTCACCTTCCCGCTGGACTATCACCTT
AACCAGCCTTTCATCTTCGTACTGAGGGACACAGACACAGGGGCCCTTCTCTTCATTGGCAAGATTCTGGACCCCAGG
GGCCCC
[SEQ ID No: 11]

Codon analysis of the endogenous PEDF 1254 nucleotide sequence (SEQ ID No: 11) using the online rare codon analysis tools (www.jcat.de and https://www.genscript.com/tools/rare-codon-analysis) revealed that the sequence displayed a poor codon adaptation index (CAI) (JCAT CAI =0.40 and Genscript CAI = 0.78). A high expressing transgene should ideally display a CAI of between 0.80 and 1.00 for efficient gene expression. Therefore, rare codons contained within the endogenous PEDF transgene were identified and substituted with those which are more frequently used in the expression host (i.e. the human eye), thus generating a codon optimised 1254 sequence (JCAT CAI = 0.96; Genscript CAI = 1.00).

Accordingly, in another embodiment, the first coding sequence comprises a nucleotide sequence (1254 bp) referred to herein as herein as SEQ ID No: 12, or a fragment or variant thereof, as follows:

ATGCAGGCCCTGGTGCTGCTGCTGTGCATCGGCGCCCTGCTGGGCCACAGCAGCTGCCAGAACCCCGCCAGCCCCCCC
GAGGAGGGCAGCCCCGACCCCGACAGCACCGGCGCCCTGGTGGAGGAGGAGGACCCCTTCTTCAAGGTGCCCGTGAAC
AAGCTGGCCGCCGCCGTGAGCAACTTCGGCTACGACCTGTACCGCGTGCGCAGCAGCACCAGCCCCACCACCAACGTG
CTGCTGAGCCCCCTGAGCGTGGCCACCGCCCTGAGCGCCCTGAGCCTGGGCGCCGAGCAGCGCACCGAGAGCATCATC
CACCGCGCCCTGTACTACGACCTGATCAGCAGCCCCGACATCCACGGCACCTACAAGGAGCTGCTGGACACCGTGACC
GCCCCCCAGAAGAACCTGAAGAGCGCCAGCCGCATCGTGTTCGAGAAGAAGCTGCGCATCAAGAGCAGCTTCGTGGCC
CCCCTGGAGAAGAGCTACGGCACCCGCCCCCGCGTGCTGACCGGCAACCCCCGCCTGGACCTGCAGGAGATCAACAAC
TGGGTGCAGGCCCAGATGAAGGGCAAGCTGGCCCGCAGCACCAAGGAGATCCCCGACGAGATCAGCATCCTGCTGCTG
GGCGTGGCCCACTTCAAGGGCCAGTGGGTGACCAAGTTCGACAGCCGCAAGACCAGCCTGGAGGACTTCTACCTGGAC
GAGGAGCGCACCGTGCGCGTGCCCATGATGAGCGACCCCAAGGCCGTGCTGCGCTACGGCCTGGACAGCGACCTGAGC
TGCAAGATCGCCCAGCTGCCCCTGACCGGCAGCATGAGCATCATCTTCTTCCTGCCCCTGAAGGTGACCCAGAACCTG
ACCCTGATCGAGGAGAGCCTGACCAGCGAGTTCATCCACGACATCGACCGCGAGCTGAAGACCGTGCAGGCCGTGCTG
ACCGTGCCCAAGCTGAAGCTGAGCTACGAGGGCGAGGTGACCAAGAGCCTGCAGGAGATGAAGCTGCAGAGCCTGTTC
GACAGCCCCGACTTCAGCAAGATCACCGGCAAGCCCATCAAGCTGACCCAGGTGGAGCACCGCGCCGGCTTCGAGTGG
AACGAGGACGGCGCCGGCACCACCCCCAGCCCCGGCCTGCAGCCCGCCCACCTGACCTTCCCCCTGGACTACCACCTG
AACCAGCCCTTCATCTTCGTGCTGCGCGACACCGACACCGGCGCCCTGCTGTTCATCGGCAAGATCCTGGACCCCCGC
GGCCCC
[SEQ ID No: 12]

In yet another preferred embodiment, the first coding sequence comprises a nucleotide sequence (1254 bp) comprising a modified signal peptide sequence, referred to herein as herein as SEQ ID No: 13, or a fragment or variant thereof, as follows:

ATGCGCCGCCTGGTGCTGCTGCTGTGCATCGGCGCCCTGCTGGGCCACGTGTTCTGCCAGAACCCCGCCAGCCCCCCC
GAGGAGGGCAGCCCCGACCCCGACAGCACCGGCGCCCTGGTGGAGGAGGAGGACCCCTTCTTCAAGGTGCCCGTGAAC
AAGCTGGCCGCCGCCGTGAGCAACTTCGGCTACGACCTGTACCGCGTGCGCAGCAGCACCAGCCCCACCACCAACGTG
CTGCTGAGCCCCCTGAGCGTGGCCACCGCCCTGAGCGCCCTGAGCCTGGGCGCCGAGCAGCGCACCGAGAGCATCATC
CACCGCGCCCTGTACTACGACCTGATCAGCAGCCCCGACATCCACGGCACCTACAAGGAGCTGCTGGACACCGTGACC
GCCCCCCAGAAGAACCTGAAGAGCGCCAGCCGCATCGTGTTCGAGAAGAAGCTGCGCATCAAGAGCAGCTTCGTGGCC
CCCCTGGAGAAGAGCTACGGCACCCGCCCCCGCGTGCTGACCGGCAACCCCCGCCTGGACCTGCAGGAGATCAACAAC
TGGGTGCAGGCCCAGATGAAGGGCAAGCTGGCCCGCAGCACCAAGGAGATCCCCGACGAGATCAGCATCCTGCTGCTG

GGCGTGGCCCACTTCAAGGGCCAGTGGGTGACCAAGTTCGACAGCCGCAAGACCAGCCTGGAGGACTTCTACCTGGAC
GAGGAGCGCACCGTGCGCGTGCCCATGATGAGCGACCCCAAGGCCGTGCTGCGCTACGGCCTGGACAGCGACCTGAGC
TGCAAGATCGCCCAGCTGCCCCTGACCGGCAGCATGAGCATCATCTTCTTCCTGCCCCTGAAGGTGACCCAGAACCTG
ACCCTGATCGAGGAGAGCCTGACCAGCGAGTTCATCCACGACATCGACCGCGAGCTGAAGACCGTGCAGGCCGTGCTG
ACCGTGCCCAAGCTGAAGCTGAGCTACGAGGGCGAGGTGACCAAGAGCCTGCAGGAGATGAAGCTGCAGAGCCTGTTC
GACAGCCCCGACTTCAGCAAGATCACCGGCAAGCCCATCAAGCTGACCCAGGTGGAGCACCGCGCCGGCTTCGAGTGG
AACGAGGACGGCGCCGGCACCACCCCCAGCCCCGGCCTGCAGCCCGCCCACCTGACCTTCCCCCTGGACTACCACCTG
AACCAGCCCTTCATCTTCGTGCTGCGCGACACCGACACCGGCGCCCTGCTGTTCATCGGCAAGATCCTGGACCCCCGC
GGCCCC
[SEQ ID No: 13]

Therefore, in preferred embodiments, the first coding sequence comprises a nucleotide sequence substantially as set out in any one of SEQ ID No: 11, 12 or 13, or a fragment or variant thereof. Preferably, the PEDF receptor agonist comprises an amino acid sequence substantially as set out in SEQ ID No: 10, or a fragment or variant thereof.

It will be appreciated that the second coding sequence encodes an anti-complement protein. Preferably, the anti-complement protein is capable of neutralising or attenuating complement activation. Even more preferably, the anti-complement protein is capable of targeting the alternative pathway (AP) of the complement system. Preferably, the anti-complement protein minimally affects the classical pathway (CP) and/or the lectin pathway (LP) of the complement system. Preferably, the anti-complement protein does not target the classical pathway (CP) and/or the lectin pathway (LP) of the complement system.

Preferably, the anti-complement protein is capable of neutralising complement factors C3b, Bb and/or C5. Accordingly, in this embodiment, the anti-complement protein is an anti-C3b, anti-Bb and/or anti-C5 antibody, or antigen-binding fragment thereof.

The antigen-binding fragment thereof may comprise or consist of any of the fragments selected from a group consisting of VH, VL, Fd, Fv, Fab, Fab', scFv, F (ab')₂ and Fc fragment, which bind C3b and/or Bb. The antigen-binding fragment may include the complementarity Determining Regions (CDRs), which bind the C3b and/or Bb epitope.

Even more preferably, the anti-complement protein is a single chain variable fragment (SCVF). In other words, in this preferred embodiment, the anti-complement protein is an anti-C3b single chain variable fragment, an anti-Bb single chain variable fragment, or an anti-C5 single chain variable fragment.

Alternatively, in another preferred embodiment, the anti-complement protein is a soluble form of the normally membrane-bound CD55 (sCD55) (also sometimes known as decay accelerating factor; DAF). Preferably, the anti-complement protein is a non-membrane attached CD55 (sCD55). Soluble CD55 (DAF) destabilises the complement protein complexes, thereby reducing the activity of this biochemical pathway.

In another preferred embodiment, the anti-complement protein is complement factor H related protein-1 (CFHR1). Preferably, CFHR1 attenuates the complement system activity cascade.

In another preferred embodiment, the anti-complement protein is a soluble form of the normally membrane-bound CD46 (sCD46). Preferably, in this embodiment, the anti-complement protein is the soluble (non-membrane associated) human complement regulatory protein CD46 (sCD46).

In another preferred embodiment, the anti-complement protein is a Complement Factor H-Like protein 1 (CFHL1). CFHL1 is a splice variant of factor H that includes the regulatory domains and inhibits complement activation at the level of the central complement component C3 and beyond.

In one preferred embodiment, the amino acid sequence of anti-C3b single chain variable fragment is referred to herein as SEQ ID No: 14, or a fragment or variant thereof, as follows:

DIQMTQSPSSLSASVGDRVTITCRASQDVSTAVAWYQQKPGKAPKLLIYSASFLYSGVPSRFSGSGSGTDFTLTISSL
QPEDFATYYCQQSYATLPTFEQGTKVEIKRGGGGGSGGGGSGGGGSGGGGSEVQLVESGGGLVQPGGSLRLSCAASGF
SFTSSSVSPGKGLEWVGLIYPYNGFNYYADSVKGRFTISADTSLQMNSLRAEDTAVYYCARNALYGSGGYYAMDYWGQ
GTLVTVSS
[SEQ ID No: 14]

In a preferred embodiment, the nucleic acid sequence (726 bp) encoding the anti-C3b single chain variable fragment is referred to herein as SEQ ID No: 15, or a fragment or variant thereof, as follows:

GACATCCAGATGACCCAGAGCCCCAGCAGCCTGAGCGCCAGCGTGGGCGACCGCGTGACCATCACCTGCCGCGCCAGC
CAGGACGTAAGCACCGCCGTGGCCTGGTACCAGCAGAAGCCCGGCAAGGCCCCCAAGCTGCTGATCTACAGCGCCAGC
TTCCTGTACAGCGGCGTGCCCAGCCGCTTCAGCGGCAGCGGCAGCGGCACCGACTTCACCCTGACCATCAGCAGCCTG
CAGCCCGAGGACTTCGCCACCTACTACTGCCAGCAGAGCTACGCCACCCTGCCCACCTTCGAGCAGGGCACCAAGGTG
GAGATCAAGCGCGGCGGCGGCGGCAGCGGCGGCGGCGGCAGCGGCGGCGGCAGCGGCGGCGGCAGCGAG
GTGCAGCTGGTGGAGAGCGGCGGCGGCCTGGTGCAGCCCGGCGGCAGCCTGCGCCTGAGCTGCGCCGCCAGCGGCTTC
AGCTTCACCAGCAGCAGCGTGAGCCCCGGCAAGGGCCTGGAGTGGGTGGGCCTGATCTACCCCTACAACGGCTTCAAC
TACTACGCCGACAGCGTGAAGGGCCGCTTCACCATCAGCGCCGACACCAGCCTGCAGATGAACAGCCTGCGCGCCGAG
GACACCGCCGTGTACTACTGCGCCCGCAACGCCCTGTACGGCAGCGGCGGCTACTACGCCATGGACTACTGGGGCCAG
GGCACCCTGGTGACCGTGAGCAGC
[SEQ ID No: 15]

In another embodiment, the amino acid sequence of anti-Bb single chain variable fragment is referred to herein as SEQ ID No: 16, or a fragment or variant thereof, as follows:

DVQITQSPSYLAASPGETITINCRASKSISKYLAWYQDKPGKTNKLLIYSGSTLQSGIPSRFSGSGSGTDFTLTISSL
EPEDFAMYYCQQHDEYPWTFGGGTKLEIKRGGGGGSGGGGSGGGGSGGGGSQVQLQQSGAELAKPGASVRMSCKASGY
TFTNYWIHWVKQRPGQGLEWIGYINPNTGYNDYNQKFKDKATLTADKSSSTVYMQLSSLTSEDSAVYYCARGGQLGLR
RAMDYWGQGTSVTVSS
[SEQ ID No: 16]

In a preferred embodiment, the nucleic acid sequence (750 bp) encoding the anti-Bb single chain variable fragment is referred to herein as SEQ ID No: 17, or a fragment or variant thereof, as follows:

GACGTGCAGATCACCCAGAGCCCCAGCTACCTGGCCGCCAGCCCCGGCGAGACCATCACCATCAACTGCCGCGCCAGC
AAGAGCATCAGCAAGTACCTGGCCTGGTACCAGGACAAGCCCGGCAAGACCAACAAGCTGCTGATCTACAGCGGCAGC
ACCCTGCAGAGCGGCATCCCCAGCCGCTTCAGCGGCAGCGGCAGCGGCACCGACTTCACCCTGACCATCAGCAGCCTG
GAGCCCGAGGACTTCGCCATGTACTACTGCCAGCAGCACGACGAGTACCCCTGGACCTTCGGCGGCGGCACCAAGCTG
GAGATCAAGCGCGGCGGCGGCGGCAGCGGCGGCGGCGGCAGCGGCGGCGGCAGCGGCGGCGGCAGCCAG
GTGCAGCTGCAGCAGAGCGGCGCCGAGCTGGCCAAGCCCGGCGCCAGCGTGCGCATGAGCTGCAAGGCCAGCGGCTAC
ACCTTCACCAACTACTGGATCCACTGGGTGAAGCAGCGCCCCGGCCAGGGCCTGGAGTGGATCGGCTACATCAACCCC
AACACCGGCTACAACGACTACAACCAGAAGTTCAAGGACAAGGCCACCCTGACCGCCGACAAGAGCAGCAGCACCGTG
TACATGCAGCTGAGCAGCCTGACCAGCGAGGACAGCGCCGTGTACTACTGCGCCCGCGGCGGCCAGCTGGGCCTGCGC
CGCGCCATGGACTACTGGGGCCAGGGCACCAGCGTGACCGTGAGCAGC

[SEQ ID No: 17]

In another embodiment, the amino acid sequence of a soluble form of the normally plasma membrane bound CD55 (sCD55, also sometimes known as decay accelerating factor; DAF) is referred to herein as SEQ ID No: 18, or a fragment or variant thereof, as follows:

DCGLPPDVPNAQPALEGRTSFPEDTVITYKCEESFVKIPGEKDSVICLKGSQWSDIEEFCNRSCEVPTRLNSASLKQP
YITQNYFPVGTVVEYECRPGYRREPSLSPKLTCLQNLKWSTAVEFCKKKSCPNPGEIRNGQIDVPGGILFGATISFSC
NTGYKLFGSTSSFCLISGSSVQWSDPLPECREIYCPAPPQIDNGIIQGERDHYGYRQSVTYACNKGFTMIGEHSIYCT
VNNDEGEWSGPPPECRGKSLTSKVPPTVQKPTTVNVPTTEVSPTSQKTTTKTTTPNAQATRSTPVSRTTKHFHETTPN
KGSGTTSG
[SEQ ID No: 18]

In a preferred embodiment, the nucleic acid sequence (960 bp) encoding a soluble form of the normally plasma membrane-bound CD55 (sCD55, also sometimes known as decay accelerating factor; DAF) is referred to herein as SEQ ID No: 19, or a fragment or variant thereof, as follows:

GACTGCGGCCTGCCCCCCGACGTGCCCAACGCCCAGCCCGCCCTGGAGGGCCGCACCAGCTTCCCCGAGGACACCGTG
ATCACCTACAAGTGCGAGGAGAGCTTCGTGAAGATCCCCGGCGAGAAGGACAGCGTGATCTGCCTGAAGGGCAGCCAG
TGGAGCGACATCGAGGAGTTCTGCAACCGCAGCTGCGAGGTGCCCACCCGCCTGAACAGCGCCAGCCTGAAGCAGCCC
TACATCACCCAGAACTACTTCCCCGTGGGCACCGTGGTGGAGTACGAGTGCCGCCCCGGCTACCGCCGCGAGCCCAGC
CTGAGCCCCAAGCTGACCTGCCTGCAGAACCTGAAGTGGAGCACCGCCGTGGAGTTCTGCAAGAAGAAGAGCTGCCCC
AACCCCGGCGAGATCCGCAACGGCCAGATCGACGTGCCCGGCGGCATCCTGTTCGGCGCCACCATCAGCTTCAGCTGC
AACACCGGCTACAAGCTGTTCGGCAGCACCAGCAGCTTCTGCCTGATCAGCGGCAGCAGCGTGCAGTGGAGCGACCCC
CTGCCCGAGTGCCGCGAGATCTACTGCCCCGCCCCCCCCCAGATCGACAACGGCATCATCCAGGGCGAGCGCGACCAC
TACGGCTACCGCCAGAGCGTGACCTACGCCTGCAACAAGGGCTTCACCATGATCGGCGAGCACAGCATCTACTGCACC
GTGAACAACGACGAGGGCGAGTGGAGCGGCCCCCCCCCCGAGTGCCGAGGCAAGAGCCTGACCAGCAAGGTGCCCCCC
ACCGTGCAGAAGCCCACCACCGTGAACGTGCCCACCACCGAGGTGAGCCCCACCAGCCAGAAGACCACCACCAAGACC
ACCACCCCCAACGCCCAGGCCACCCGCAGCACCCCCGTGAGCCGCACCACCAAGCACTTCCACGAGACCACCCCCAAC
AAGGGCAGCGGCACCACCAGCGGC
[SEQ ID No: 19]

In a further embodiment, the amino acid sequence of human complement factor H related protein-1 (CFHR1) is referred to herein as SEQ ID No: 20, or a fragment or variant thereof, as follows:

EATFCDFPKINHGILYDEEKYKPFSQVPTGEVFYYSCEYNFVSPSKSFWTRITCTEEGWSPTPKCLRLCFFPFVENGH
SESSGQTHLEGDTVQIICNTGYRLQNNENNISCVERGWSTPPKCRSTDTSCVNPPTVQNAHILSRQMSKYPSGERVRY
ECRSPYEMFGDEEVMCLNGNWTEPPQCKDSTGKCGPPPPIDNGDITSFPLSVYAPASSVEYQCQNLYQLEGNKRITCR
NGQWSEPPKCLHPCVISREIMENYNIALRWTAKQKLYLRTGESAEFVCKRGYRLSSRSHTLRTTCWDGKLEYPTCAKR
[SEQ ID No: 20]

In a preferred embodiment, the nucleic acid sequence (936 bp) encoding human complement factor H related protein-1 (CFHR1) is referred to herein as SEQ ID No: 21, or a fragment or variant thereof, as follows:

GAAGCAACATTTTGTGATTTTCCAAAAATAAACCATGGAATTCTATATGATGAAGAAAAATATAAGCCATTTTCCCAG
GTTCCTACAGGGGAAGTTTTCTATTACTCCTGTGAATATAATTTTGTGTCTCCTTCAAAATCATTTTGGACTCGCATA
ACATGCACAGAAGAAGGATGGTCACCAACACCAAAGTGTCTCAGACTGTGTTTCTTTCCTTTTGTGGAAAATGGTCAT
TCTGAATCTTCAGGACAAACACATCTGGAAGGTGATACTGTGCAAATTATTTGCAACACAGGATACAGACTTCAAAAC
AATGAGAACAACATTTCATGTGTAGAACGGGGCTGGTCCACCCCTCCCAAATGCAGGTCCACTGACACTTCCTGTGTG
AATCCGCCCACAGTACAAAATGCTCATATACTGTCGAGACAGATGAGTAAATATCCATCTGGTGAGAGAGTACGTTAT
GAATGTAGGAGCCCTTATGAAATGTTTGGGGATGAAGAAGTGATGTGTTTAAATGGAAACTGGACAGAACCACCTCAA
TGCAAAGATTCTACGGGAAAATGTGGGCCCCCTCCACCTATTGACAATGGGGACATTACTTCATTCCCGTTGTCAGTA
TATGCTCCAGCTTCATCAGTTGAGTACCAATGCCAGAACTTGTATCAACTTGAGGGTAACAAGCGAATAACATGTAGA
AATGGACAATGGTCAGAACCACCAAAATGCTTACATCCGTGTGTAATATCCCGAGAAATTATGGAAAATTATAACATA
GCATTAAGGTGGACAGCCAAACAGAAGCTTTATTTGAGAACAGGTGAATCAGCTGAATTTGTGTGTAAACGGGGATAT
CGTCTTTCATCACGTTCTCACACATTGCGAACAACATGTTGGGATGGGAAACTGGAGTATCCAACTTGTGCAAAAAGA
[SEQ ID No: 21]

In another embodiment, the codon optimised nucleic acid sequence (936 bp) encoding human complement factor H related protein-1 (CFHR1) is referred to herein as SEQ ID No: 22, or a fragment or variant thereof, as follows:

GAGGCCACCTTCTGCGACTTCCCCAAGATCAACCACGGCATCCTGTACGACGAGGAGAAGTACAAGCCCTTCAGCCAG
GTGCCCACCGGCGAGGTGTTCTACTACAGCTGCGAGTACAACTTCGTGAGCCCCAGCAAGAGCTTCTGGACCCGCATC
ACCTGCACCGAGGAGGGCTGGAGCCCCACCCCCAAGTGCCTGCGCCTGTGCTTCTTCCCCTTCGTGGAGAACGGCCAC
AGCGAGAGCAGCGGCCAGACCCACCTGGAGGGCGACACCGTGCAGATCATCTGCAACACCGGCTACCGCCTGCAGAAC
AACGAGAACAACATCAGCTGCGTGGAGCGCGGCTGGAGCACCCCCCCCAAGTGCCGCAGCACCGACACCAGCTGCGTG
AACCCCCCCACCGTGCAGAACGCCCACATCCTGAGCCGCCAGATGAGCAAGTACCCCAGCGGCGAGCGCGTGCGCTAC
GAGTGCCGCAGCCCCTACGAGATGTTCGGCGACGAGGAGGTGATGTGCCTGAACGGCAACTGGACCGAGCCCCCCCAG
TGCAAGGACAGCACCGGCAAGTGCGGCCCCCCCCCCCCCATCGACAACGGCGACATCACCAGCTTCCCCCTGAGCGTG
TACGCCCCCGCCAGCAGCGTGGAGTACCAGTGCCAGAACCTGTACCAGCTGGAGGGCAACAAGCGCATCACCTGCCGC
AACGGCCAGTGGAGCGAGCCCCCCAAGTGCCTGCACCCCTGCGTGATCAGCCGCGAGATCATGGAGAACTACAACATC
GCCCTGCGCTGGACCGCCAAGCAGAAGCTGTACCTGCGCACCGGCGAGAGCGCCGAGTTCGTGTGCAAGCGCGGCTAC
CGCCTGAGCAGCCGCAGCCACACCCTGCGCACCACCTGCTGGGACGGCAAGCTGGAGTACCCCACCTGCGCCAAGCGC
[SEQ ID No: 22]

In a further embodiment, the amino acid sequence of a soluble form of the normally plasma membrane-bound CD46 (sCD46) is referred to herein as SEQ ID No: 23, or a fragment or variant thereof, as follows:

CEEPPTFEAMELIGKPKPYYEIGERVDYKCKKGYFYIPPLATHTICDRNHTWLPVSDDACYRETCPYIRDPLNGQAVP
ANGTYEFGYQMHFICNEGYYLIGEEILYCELKGSVAIWSGKPPICEKVLCTPPPKIKNGKHTFSEVEVFEYLDAVTYS
CDPAPGPDPFSLIGESTIYCGDNSVWSRAAPECKVVKCRFPVVENGKQISGFGKKFYYKATVMFECDKGFYLDGSDTI
VCDSNSTWDPPVPKCLKVLPPSSTKPPALSHSVSTSSTTKSPASSASGPRPTYKPPVSNYPGYPKPEEGILDSLDV
[SEQ ID No: 23]

In a preferred embodiment, the nucleic acid sequence (930 bp) encoding a soluble form of the normally plasma membrane-bound CD46 (sCD46) is referred to herein as SEQ ID No: 24, or a fragment or variant thereof, as follows:

TGCGAGGAGCCCCCCACCTTCGAGGCCATGGAGCTGATCGGCAAGCCCAAGCCCTACTACGAGATCGGCGAGCGCGTG
GACTACAAGTGCAAGAAGGGCTACTTCTACATCCCCCCCCTGGCCACCCACACCATCTGCGACCGCAACCACACCTGG
CTGCCCGTGAGCGACGACGCCTGCTACCGCGAGACCTGCCCCTACATCCGCGACCCCCTGAACGGCCAGGCCGTGCCC
GCCAACGGCACCTACGAGTTCGGCTACCAGATGCACTTCATCTGCAACGAGGGCTACTACCTGATCGGCGAGGAGATC
CTGTACTGCGAGCTGAAGGGCAGCGTGGCCATCTGGAGCGGCAAGCCCCCCATCTGCGAGAAGGTGCTGTGCACCCCC
CCCCCCAAGATCAAGAACGGCAAGCACACCTTCAGCGAGGTGGAGGTGTTCGAGTACCTGGACGCCGTGACCTACAGC
TGCGACCCCGCCCCCGGCCCCGACCCCTTCAGCCTGATCGGCGAGAGCACCATCTACTGCGGCGACAACAGCGTGTGG
AGCCGCGCCGCCCCCGAGTGCAAGGTGGTGAAGTGCCGCTTCCCCGTGGTGGAGAACGGCAAGCAGATCAGCGGCTTC
GGCAAGAAGTTCTACTACAAGGCCACCGTGATGTTCGAGTGCGACAAGGGCTTCTACCTGGACGGCAGCGACACCATC
GTGTGCGACAGCAACAGCACCTGGGACCCCCCCGTGCCCAAGTGCCTGAAGGTGCTGCCCCCCAGCAGCACCAAGCCC
CCCGCCCTGAGCCACAGCGTGAGCACCAGCAGCACCACCAAGAGCCCCGCCAGCAGCGCCAGCGGCCCCCGCCCCACC
TACAAGCCCCCCGTGAGCAACTACCCCGGCTACCCCAAGCCCGAGGAGGGCATCCTGGACAGCCTGGACGTG
[SEQ ID No: 24]

In a preferred embodiment, the amino acid sequence of CFHL1 is referred to herein as SEQ ID No: 80, or a fragment or variant thereof, as follows:

EDCNELPPRRNTEILTGSWSDQTYPEGTQAIYKCRPGYRSLGNVIMVCRKGEWVALNPLRKCQKRPCGHPGDTPFGTF
TLTGGNVFEYGVKAVYTCNEGYQLLGEINYRECDTDGWTNDIPICEVVKCLPVTAPENGKIVSSAMEPDREYHFGQAV
RFVCNSGYKIEGDEEMHCSDDGFWSKEKPKCVEISCKSPDVINGSPISQKIIYKENERFQYKCNMGYEYSERGDAVCT
ESGWRPLPSCEEKSCDNPYIPNGDYSPLRIKHRTGDEITYQCRNGFYPATRGNTAKCTSTGWIPAPRCTLKPCDYPDI
KHGGLYHENMRRPYFPVAVGKYYSYYCDEHFETPSGSYWDHIHCTQDGWSPAVPCLRKCYFPYLENGYNQNYGRKFVQ
GKSIDVACHPGYALPKAQTTVTCMENGWSPTPRCIR
[SEQ ID No: 80]

In a preferred embodiment, the nucleic acid sequence (1278 bp) encoding CFHL1 is referred to herein as SEQ ID No: 81, or a fragment or variant thereof, as follows:

GAGGACTGCAACGAGCTGCCCCCCCGCCGCAACACCGAGATCCTGACCGGCAGCTGGAGCGACCAGACCTACCCCGAG
GGCACCCAGGCCATCTACAAGTGCCGCCCCGGCTACCGCAGCCTGGGCAACGTGATCATGGTGTGCCGCAAGGGCGAG

TGGGTGGCCCTGAACCCCCTGCGCAAGTGCCAGAAGCGCCCCTGCGGCCACCCCGGCGACACCCCCTTCGGCACCTTC
ACCCTGACCGGCGGCAACGTGTTCGAGTACGGCGTGAAGGCCGTGTACACCTGCAACGAGGGCTACCAGCTGCTGGGC
GAGATCAACTACCGCGAGTGCGACACCGACGGCTGGACCAACGACATCCCCATCTGCGAGGTGGTGAAGTGCCTGCCC
GTGACCGCCCCCGAGAACGGCAAGATCGTGAGCAGCGCCATGGAGCCCGACCGCGAGTACCACTTCGGCCAGGCCGTG
CGCTTCGTGTGCAACAGCGGCTACAAGATCGAGGGCGACGAGGAGATGCACTGCAGCGACGACGGCTTCTGGAGCAAG
GAGAAGCCCAAGTGCGTGGAGATCAGCTGCAAGAGCCCCGACGTGATCAACGGCAGCCCCATCAGCCAGAAGATCATC
TACAAGGAGAACGAGCGCTTCCAGTACAAGTGCAACATGGGCTACGAGTACAGCGAGCGCGGCGACGCCGTGTGCACC
GAGAGCGGCTGGCGCCCCCTGCCCAGCTGCGAGGAGAAGAGCTGCGACAACCCCTACATCCCCAACGGCGACTACAGC
CCCCTGCGCATCAAGCACCGCACCGGCGACGAGATCACCTACCAGTGCCGCAACGGCTTCTACCCCGCCACCCGGGGC
AACACCGCCAAGTGCACCAGCACCGGCTGGATCCCCGCCCCCCGCTGCACCCTGAAGCCCTGCGACTACCCCGACATC
AAGCACGGCGGCCTGTACCACGAGAACATGCGCCGCCCCTACTTCCCCGTGGCCGTGGGCAAGTACTACAGCTACTAC
TGCGACGAGCACTTCGAGACCCCCAGCGGCAGCTACTGGGACCACATCCACTGCACCCAGGACGGCTGGAGCCCCGCC
GTGCCCTGCCTGCGCAAGTGCTACTTCCCCTACCTGGAGAACGGCTACAACCAGAACTACGGCCGCAAGTTCGTGCAG
GGCAAGAGCATCGACGTGGCCTGCCACCCCGGCTACGCCCTGCCCAAGGCCCAGACCACCGTGACCTGCATGGAGAAC
GGCTGGAGCCCCACCCCCCGCTGCATCCGC
[SEQ ID No: 81]

In a preferred embodiment, the amino acid sequence of the anti-C5 single chain variable fragment is referred to herein as SEQ ID No: 82, or a fragment or variant thereof, as follows:

DIQMTQSPSSLSASVGDRVTITCQASQSINNQLSWYQQKPGKAPKLLIYYASTLASGYPSRFSGSGSGTDFTLTISSL
QPEDFATYYCQGSYYSGGWDYGFGQGTKVEIKRGGGGGSGGGSGGGGSGGGGSEVQLVESGGGLVQPGGSLRLSCAAS
GFSFSGRYWIQWVRQAPGKGLEWVASVWPGITGTNYANWAKGRFTISRDDSKNTLYLQMNSLRAEDTAVYYCAREPVA
WGGGLDLWGQGTLVTVSS
[SEQ ID No: 82]

In a preferred embodiment, the nucleic acid sequence (759 bp) encoding anti-C5 single chain variable fragment is referred to herein as SEQ ID No: 83, or a fragment or variant thereof, as follows:

GACATCCAGATGACCCAGAGCCCCAGCAGCCTGAGCGCCAGCGTGGGCGACCGCGTGACCATCACCTGCCAGGCCAGC
CAGAGCATCAACAACCAGCTGAGCTGGTACCAGCAGAAGCCCGGCAAGGCCCCCAAGCTGCTGATCTACTACGCCAGC
ACCCTGGCCAGCGGCTACCCCAGCCGCTTCAGCGGCAGCGGCAGCGGCACCGACTTCACCCTGACCATCAGCAGCCTG
CAGCCCGAGGACTTCGCCACCTACTACTGCCAGGGCAGCTACTACAGCGGCGGCTGGGACTACGGCTTCGGCCAGGGC
ACCAAGGTGGAGATCAAGCGCGGCGGCGGCGGCAGCGGCGGCGGCAGCGGCGGCGGCAGCGGCGGCGGC
AGCGAGGTGCAGCTGGTGGAGAGCGGCGGCGGCCTGGTGCAGCCCGGCGGCAGCCTGCGCCTGAGCTGCGCCGCCAGC
GGCTTCAGCTTCAGCGGCCGCTACTGGATCCAGTGGGTGCGCCAGGCCCCCGGCAAGGGCCTGGAGTGGGTGGCCAGC
GTGTGGCCCGGCATCACCGGCACCAACTACGCCAACTGGGCCAAGGGCCGCTTCACCATCAGCCGCGACGACAGCAAG
AACACCCTGTACCTGCAGATGAACAGCCTGCGCGCCGAGGACACCGCCGTGTACTACTGCGCCCGCGAGCCCGTGGCC
TGGGGCGGCGGCCTGGACCTGTGGGGCCAGGGCACCCTGGTGACCGTGAGCAGCTGA

[SEQ ID No: 83]

Therefore, in preferred embodiments, the second coding sequence comprises a nucleotide sequence substantially as set out in any one of SEQ ID No: 15, 17, 19, 21, 22; 24, 81 or 83, or a fragment or variant thereof. Preferably, the anti-complement protein comprises an amino acid sequence substantially as set out in SEQ ID No: 14, 16, 18, 20, 23, 80 or 82, or a fragment or variant thereof.

Many gene constructs expressing two or more genes that are presented in the scientific literature have either (i) dual promoters to separately drive expression of two or more genes, or (ii) an internal ribosome entry site (IRES) to link the genes, such as that from the encephalomyocarditis virus (EMCV), to enable translation of the genes from a single transcript driven by a single promoter within recombinant viral vectors. However, the efficiency of IRES-dependent translation varies dramatically in different cells and tissues, and IRES-dependent translation can be significantly lower than cap-dependent translation, meaning that there is often lower expression of genes downstream of an IRES when compared to the gene in position one (i.e. the 5' end) of the expression cassette. Moreover, the limited coding capacity of rAAV vectors (generally <5kb) prevents the incorporation of large genes/ORFs, such as a coding sequence for the PEDF receptor agonist and the anti-complement protein using dual promoters and/or IRES linkers (for which the EMCV IRES is 553 nucleotides in length).

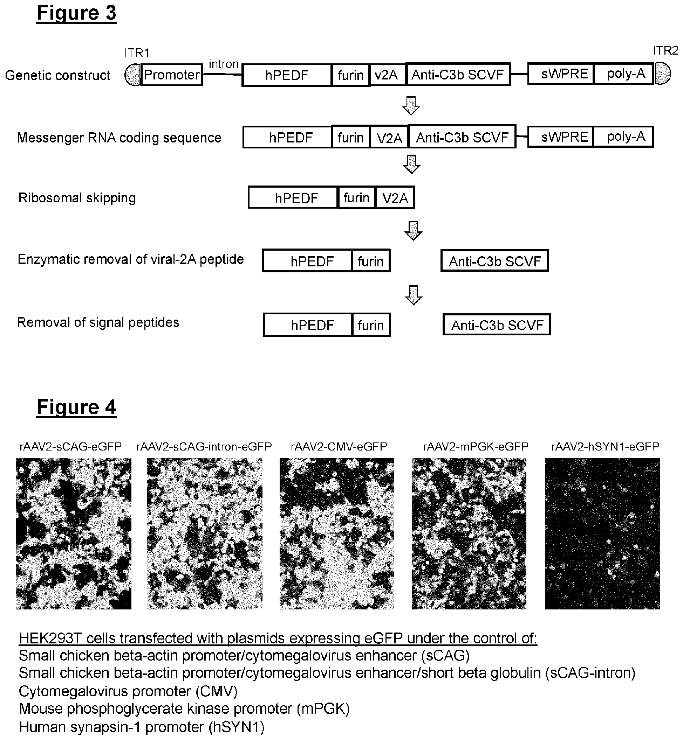

Accordingly, in a preferred embodiment, the genetic construct comprises a spacer sequence disposed between the first and second coding sequences. For example, see Figure 3, in which the spacer sequence (v2A) is disposed between the first and second coding sequences. This spacer sequence encodes a peptide spacer that is configured to produce the PEDF receptor agonist and the anti-complement protein as separate molecules. This is possible as the spacer is configured to skip the linear ribosomal sequence transcription to produce the separate molecules or peptides. It will be appreciated that the separate molecules are active.

Preferably, the spacer sequence comprises and encodes a viral peptide spacer sequence, most preferably a viral-2A peptide spacer sequence. In one embodiment, this viral-2A peptide spacer sequence comprises a F2A, E2A, T2A or P2A sequence.

Preferably, the viral-2A peptide sequence connects the first coding sequence to the second coding sequence. This enables the construct to overcome the size restrictions that occur with expression in various vectors and enables expression of all of the peptides encoded by the construct of the first aspect to occur under control of a single promoter, as a single mRNA transcript.

Thus, in one embodiment, following the transcription of the single mRNA transcript encoding the sequences of the PEDF receptor agonist, the viral-2A peptide, and the anti-complement protein, translational skipping may occur at the viral-2A peptide sequence between the terminal glycine-proline of the viral-2A peptide. This translational skipping will thereby generate two proteins, i.e. the PEDF receptor agonist and the anti-complement protein (see Figure 3).

The inventors have generated four embodiments of the spacer sequence. One important section of the peptide spacer sequence, which is common to all embodiments described herein, is the C-terminus.

In one embodiment, the peptide spacer sequence is P2A. Preferably, the P2A peptide spacer sequence encodes an amino acid sequence referred to herein as SEQ ID No: 25, or a fragment or variant thereof, as follows:
ATNFSLLKQAGDVEENPGP
[SEQ ID No: 25]

Preferably, the digestion or cut site of the peptide spacer sequence is disposed between the terminal glycine and end proline in SEQ ID No: 25.

In this first embodiment, the P2A peptide spacer sequence comprises a nucleotide sequence (57 bp) referred to herein as SEQ ID No: 26, or a fragment or variant thereof, as follows:
GCCACCAACTTCAGCCTGCTGAAGCAGGCCGGCGACGTGGAGGAGAACCCCGGCCCC
[SEQ ID No: 26]

In a second embodiment, the peptide spacer sequence is E2A. Preferably, the E2A peptide spacer sequence encodes an amino acid sequence referred to herein as SEQ ID No: 27, or a fragment or variant thereof, as follows:
QCTNYALLKLAGDVESNPGP
[SEQ ID No: 27]

Preferably, the digestion or cut site of the peptide spacer sequence is disposed between the terminal glycine and end proline in SEQ ID No: 27.

In this second embodiment, the E2A peptide spacer sequence comprises a nucleotide sequence (60 bp) referred to herein as SEQ ID No: 28, or a fragment or variant thereof, as follows:
CAGTGCACCAACTACGCCCTGCTGAAGCTGGCCGGCGACGTGGAGAGCAACCCCGGCCCC
[SEQ ID No: 28]

In a third embodiment, the peptide spacer sequence is T2A. Preferably, the T2A peptide spacer sequence encodes an amino acid sequence referred to herein as SEQ ID No: 29, or a fragment or variant thereof, as follows:
EGRGSLLTCGDVEENPGP
[SEQ ID No: 29]

Preferably, the digestion or cut site of the peptide spacer sequence is disposed between the terminal glycine and end proline in SEQ ID No: 29.

In this third embodiment, the T2A peptide spacer sequence comprises a nucleotide sequence (54 bp) referred to herein as SEQ ID No: 30, or a fragment or variant thereof, as follows:
GAGGGCCGCGGCAGCCTGCTGACCTGCGGCGACGTGGAGGAGAACCCCGGCCCC
[SEQ ID No: 30]

In a fourth preferred embodiment, the peptide spacer sequence is F2A. Preferably, the F2A peptide spacer sequence encodes an amino acid sequence referred to herein as SEQ ID No: 31 or a fragment or variant thereof, as follows:
VKQTLNFDLLKLAGDVESNPGP
[SEQ ID No: 31]

Preferably, the digestion or cut site of the peptide spacer sequence is disposed between the terminal glycine and end proline in SEQ ID No: 31.

In this fourth embodiment, the F2A peptide spacer sequence comprises a nucleotide sequence (66 bp) referred to herein as SEQ ID No: 32, or a fragment or variant thereof, as follows:
GTGAAGCAGACCCTGAACTTCGACCTGCTGAAGCTGGCCGGCGACGTGGAGAGCAACCCCGGCCCC
[SEQ ID No: 32]

Therefore, in one preferred embodiment, the peptide spacer sequence comprises a nucleotide sequence substantially as set out in any one of SEQ ID No: 26, 28, 30 or 32, or a fragment or variant thereof. Preferably, the peptide spacer sequence encodes an amino acid sequence substantially as set out in SEQ ID No: 25, 27, 29 or 31 or a fragment or variant thereof.

After translational skipping, the viral-2A peptide sequence remains fused to the C-terminus of the upstream protein (such as the PEDF receptor agonist), whilst the proline remains fused to the N-terminus of the downstream protein (such as the anti-complement protein). This poses an immunogenicity risk and may potentially interfere with the intracellular signalling capability at the PEDF receptor. Therefore, the inventors have introduced an enzyme cleavage coding sequence directly upstream of the viral-2A peptide sequence, such that the remaining viral-2A peptide sequence from both the encoded proteins (i.e. the PEDF receptor agonist and anti-complement protein) is removed. The introduction of the enzyme cleavage site has the effect of removing the viral-2A peptide either intracellularly prior to release of the secreted proteins, in the case of the enzyme furin, or shortly after the proteins have been secreted from the target (retinal) cells, in the case of the enzyme recognition sites for matrix metalloprotein-2 (MMP-2) or renin.

Accordingly, in one embodiment, the construct further comprises a viral-2A removal sequence. Preferably, the viral-2A removal sequence is disposed 5' of the viral-2A sequence. Preferably, the viral-2A removal sequence is separated from the viral-2A sequence by a linker sequence comprising a tripeptide glycine-serine-glycine sequence (G-S-G).

The inventors have introduced a furin recognition sequence to enzymatically remove the viral-2A peptide sequence from the C-terminal of the proteins. Accordingly, in one embodiment, the viral-2A removal sequence is a furin recognition sequence.

Currently, the furin recognition sequence is generally recognised as comprising three or four basic amino acids (arginine or lysine) with an optional non-basic amino acid at position 2, and is cleaved by the enzyme furin after the last basic amino acid. However, using various plasmid constructs, the inventors determined that this basic furin recognition sequence does not always result in enzymatic activity and separation of the viral-2A sequence. As such, the inventors have generated a preferred furin recognition sequence for use in the genetic construct of the invention.

Accordingly, in a preferred embodiment, the genetic construct comprises a viral-2A removal sequence encoding an amino acid sequence referred to herein as SEQ ID No: 33, or a fragment or variant thereof, in which: B = basic amino acid, X = hydrophilic amino acid, and S = serine, as follows:
BB (X) BBS
[SEQ ID No: 33]

Preferably, the hydrophilic amino acid (X) is either serine (S) or threonine (T). Accordingly, in one embodiment, the viral-2A removal sequence encodes an amino acid sequence substantially as set out in SEQ ID No: 33, or a fragment or variant thereof.

In one embodiment, the viral-2A removal sequence encodes an amino acid sequence referred to herein as SEQ ID No: 34 or a fragment or variant thereof, as follows:
RRSKRSGSG
[SEQ ID No: 34]

In this first embodiment, the viral-2A removal sequence comprises a nucleotide sequence referred to herein as SEQ ID No: 35, or a fragment or variant thereof, as follows:
CGCCGCAGCAAGCGCAGCGGCAGCGGC
[SEQ ID No: 35]

In a second embodiment, the viral-2A removal sequence encodes an amino acid sequence referred to herein as SEQ ID No: 36, or a fragment or variant thereof, as follows:
RRTKRSGSG
[SEQ ID No: 36]

In this second embodiment, the viral-2A removal sequence comprises a nucleotide sequence referred to herein as SEQ ID No: 37, or a fragment or variant thereof, as follows:
CGCCGCACCAAGCGCAGCGGCAGCGGC
[SEQ ID No: 37]

Therefore, in one embodiment, the viral-2A removal sequence comprises a nucleotide sequence substantially as set out in either SEQ ID No: 35 or 37, or a fragment or variant thereof. Preferably, the viral-2A removal sequence encodes an amino acid sequence substantially as set out in SEQ ID No: 34 or 36, or a fragment or variant thereof.

Alternatively, in another embodiment, the viral-2A removal sequence is a gelatinase MMP-2 recognition sequence. Preferably, in this embodiment, the viral-2A removal sequence encodes the amino acid sequence GPQGIAGQ [SEQ ID No: 74], GPLGIAGA [SEQ ID No: 75] or GPQGLLGQ [SEQ ID No: 76], or a fragment or variant thereof. Cleavage preferably occurs after the second glycine residue.

The inventors have generated a preferred amino acid sequence referred to herein as SEQ ID No: 38, which comprises a gelatinase MMP-2 recognition sequence and the tripeptide GSG linker sequence.

Accordingly, in one embodiment, the viral-2A removal sequence encodes an amino acid sequence referred to herein as SEQ ID No: 38, or a fragment or variant thereof, as follows:
GPQGIAGQGSG
[SEQ ID No: 38]

In this embodiment, the viral-2A removal sequence comprises a nucleotide sequence referred to herein as SEQ ID No: 39, or a fragment or variant thereof, as follows:
GGCCCCCTGGGCATCGCCGGCCAGGGCAGCGGC
[SEQ ID No: 39]

Therefore, in one embodiment, the viral-2A removal sequence comprises a nucleotide sequence substantially as set out in SEQ ID No: 39, or a fragment or variant thereof. Preferably, the viral-2A removal sequence encodes an amino acid sequence substantially as set out in SEQ ID No: 38, or a fragment or variant thereof.

Alternatively, in another embodiment, the viral-2A removal sequence is a renin recognition sequence. Preferably, in this embodiment, the viral-2A removal sequence encodes the amino acid sequence HPFHLVYS [SEQ ID No: 77] or HPFHLLVYS [SEQ ID No: 78], or a fragment or variant thereof. Cleavage preferably occurs after the leucine residue(s).

The inventors have generated a preferred amino acid sequence referred to herein as SEQ ID No: 40, which comprises a renin recognition sequence and the tripeptide GSG linker sequence.

Accordingly, in one embodiment, the viral-2A removal sequence encodes an amino acid sequence referred to herein as SEQ ID No: 40, or a fragment or variant thereof, as follows:
HPFHLLVYSGSG
[SEQ ID No: 40]

In this embodiment, the viral-2A removal sequence comprises a nucleotide sequence referred to herein as SEQ ID No: 41, or a fragment or variant thereof, as follows:
CGCCCCTTCCACCTGCTGGTCATCCACGGCAGCGGC
[SEQ ID No: 41]

Therefore, in one embodiment, the viral-2A removal sequence comprises a nucleotide sequence substantially as set out in SEQ ID No: 41, or a fragment or variant thereof. Preferably, the viral-2A removal sequence encodes an amino acid sequence substantially as set out in SEQ ID No: 40, or a fragment or variant thereof.

As illustrated in Figure 2, the expression cassette further comprises a sequence encoding Hepatitis Virus Post-transcriptional Regulatory Element (WPRE), a sequence encoding a poly-A tail, and left and right-hand Inverted Terminal Repeat sequences (ITRs). Preferably, the genetic construct comprises a nucleotide sequence encoding Woodchuck Hepatitis Virus (WHP) Post-transcriptional Regulatory Element (WPRE), which enhances the expression of the transgenes, i.e. the PEDF receptor agonist and the anti-complement protein. Preferably, the WPRE coding sequence is disposed 3' of the transgene coding sequence.

One embodiment of the Woodchuck Hepatitis Virus Post-transcriptional Regulatory Element (WPRE) is 592 nucleotides long, including gamma-alpha-beta elements, and is referred to herein as SEQ ID No: 42, as follows:

AATCAACCTCTGGATTACAAAATTTGTGAAAGATTGACTGGTATTCTTAACTATGTTGCTCCTTTTACGCTATGTGGA
TACGCTGCTTTAATGCCTTTGTATCATGCTATTGCTTCCCGTATGGCTTTCATTTTCTCCTCCTTGTATAAATCCTGG
TTGCTGTCTCTTTATGAGGAGTTGTGGCCCGTTGTCAGGCAACGTGGCGTGGTGTGCACTGTGTTTGCTGACGCAACC
CCCACTGGTTGGGGCATTGCCACCACCTGTCAGCTCCTTTCCGGGACTTTCGCTTTCCCCCTCCCTATTGCCACGGCG
GAACTCATCGCCGCCTGCCTTGCCCGCTGCTGGACAGGGGCTCGGCTGTTGGGCACTGACAATTCCGTGGTGTTGTCG
GGGAAGCTGACGTCCTTTCCATGGCTGCTCGCCTGTGTTGCCACCTGGATTCTGCGCGGGACGTCCTTCTGCTACGTC
CCTTCGGCCCTCAATCCAGCGGACCTTCCTTCCCGCGGCCTGCTGCCGGCTCTGCGGCCTCTTCCGCGTCTTCGCCTT
CGCCCTCAGACGAGTCGGATCTCCCTTTGGGCCGCCTCCCCGCCTG

[SEQ ID No: 42]

Preferably, the WPRE comprises a nucleic acid sequence substantially as set out in SEQ ID No: 42, or a fragment or variant thereof.

However, in a preferred embodiment, a truncated WPRE is used, which is 247 nucleotides long due to deletion of the β-element, and which is referred to herein as SEQ ID No: 43, as follows:

AATCAACCTCTGGATTACAAAATTTGTGAAAGATTGACTGGTATTCTTAACTATGTTGCTCCTTTTACGCTATGTGGA
TACGCTGCTTTAATGCCTTTGTATCATGCTATTGCTTCCCGTATGGCTTTCATTTTCTCCTCCTTGTATAAATCCTGG
TTAGTTCTTGCCACGGCGGAACTCATCGCCGCCTGCCTTGCCCGCTGCTGGACAGGGGCTCGGCTGTTGGGCACTGAC
AATTCCGTGGTGT
[SEQ ID No: 43]

Preferably, therefore, the truncated WPRE comprises a nucleic acid sequence substantially as set out in SEQ **ID** No: 43, or a fragment or variant thereof.

Advantageously, the truncated WPRE sequence used in the construct saves about 300 nucleotides in total without negatively impacting on transgene expression. Preferably, therefore, the WPRE comprises a nucleic acid sequence substantially as set out in SEQ **ID** No: 43, or a fragment or variant thereof.

Preferably, the genetic construct comprises a nucleotide sequence encoding a poly-A tail. Preferably, the poly-A tail coding sequence is disposed 3' of the transgene coding sequence, and preferably 3' of the WPRE coding sequence. The polyA tail is important for the nuclear export, translation, and stability of mRNA. The tail is shortened over time, and, when it is short enough, the mRNA is enzymatically degraded.

Preferably, the poly-A tail comprises the simian virus-40 poly-A 224 nucleotide sequence. One embodiment of the poly-A tail is referred to herein as SEQ ID No: **44, as** follows:

AGCAGACATGATAAGATACATTGATGAGTTTGGACAAACCACAACTAGAATGCAGTGAAAAAAATGCTTTATTTGTGA
AATTTGTGATGCTATTGCTTTATTTGTAACCATTATAAGCTGCAATAAACAAGTTAACAACAACAATTGCATTCATTT
TATGTTTCAGGTTCAGGGGGAGGTGTGGGAGGTTTTTTAAAGCAAGTAAAACCTCTACAAATGTGGTA
[SEQ ID No: 44]

In another embodiment, the poly-A tail comprises a 169 nucleotide sequence polyA component, which is referred to herein as SEQ ID No: 45, as follows:

TTCGAGCAACTTGTTTATTGCAGCTTATAATGGTTACAAATAAAGCAATAGCATCACAAATTTCACAAATAAAGCATT
TTTTTCACTGCATTCTAGTTGTGGTTTGTCCAAACTCATCAATGTATCTTATCATGTCTGGATCGTCTAGCATCGAAG
ATCCCCCGATCTG
[SEQ ID No: 45]

In a further embodiment, the poly-A tail comprises the bovine growth hormone 225 nucleotide sequence. which is referred to herein as SEQ ID No: 84, as follows:

CTGTGCCTTCTAGTTGCCAGCCATCTGTTGTTTGCCCCTCCCCCGTGCCTTCCTTGACCCTGGAAGGTGCCACTCCCA
CTGTCCTTTCCTAATAAAATGAGGAAATTGCATCGCATTGTCTGAGTAGGTGTCATTCTATTCTGGGGGGTGGGGTGG
GGCAGGACAGCAAGGGGGAGGATTGGGAAGACAATAGCAGGCATGCTGGGGATGCGGTGGGCTCTATGG
[SEQ ID No: 84]

Preferably, therefore, the poly-A tail comprises a nucleic acid sequence substantially as set out in SEQ ID No: 44, 45 or 84, or a fragment or variant thereof.

Preferably, the genetic construct comprises left and/or right Inverted Terminal Repeat sequences (ITRs). Preferably, each ITR is disposed at the 5' and/or 3' end of the construct. An ITR can be specific to a virus (e.g. AAV or lentivirus) serotype, and can be any sequence, so long as it forms a hairpin loop in its secondary structure.

The DNA sequence of one embodiment (left ITR sequence taken from a commercially available recombinant AAV genome plasmid) of the ITR is represented herein as SEQ ID No: 46, as follows:

CGCGCTCGCTCGCTCACTGAGGCCGCCCGGGCAAAGCCCGGGCGTCGGGCGACCTTTGGTCGCCCGGCCTCAGTGAGC
GAGCGAGCGCGCAGAGAGGGAGTGGCCAACTCCATCACTAGGGGTTCCTTG
[SEQ ID No: 46]

The DNA sequence of another embodiment (right ITR sequence taken from a commercially available recombinant AAV genome plasmid) of the ITR is represented herein as SEQ ID No: 47, as follows:

AGGAACCCCTAGTGATGGAGTTGGCCACTCCCTCTCTGCGCGCTCGCTCGCTCACTGAGGCCGGGCGACCAAAGGTCG
CCCGACGCCCGGGCTTTGCCCGGGCGGCCTCAGTGAGCGAGCGAGCGCGC
[SEQ ID No: 47]

Preferably, the left and/or right Inverted Terminal Repeats comprise a nucleic acid sequence substantially as set out in SEQ ID No: 46 or 47, or a fragment or variant thereof.

Recently, it has been discovered that non-coding introns located between the promoter and the gene (next to the 3' end of a promoter and the 5' end of the gene) can facilitate gene expression in certain genomic sequences through mRNA accumulation [59,60]. Therefore, inclusion of an intron to the genetic construct of a viral vector may facilitate greater transgene expression and subsequent generation of mature proteins when used in combination with a constitutive or regulated promoter.

Hence, in one embodiment, the genetic construct comprises a non-coding intron. Preferably, the non-coding intron is located between the promoter and the first coding sequence. In other words, the non-coding intron is disposed 3' of the promoter and 5' of the first coding sequence.

In one embodiment, the non-coding intron is a minute virus of mice (MVM) small (121 bp) intron [61], referred to herein as SEQ ID No: 48, as follows:

AGGTACGATGGCGCCTCCAGCTAAAAGAGCTAAAAGAGGTAAGGGTTTAAGGGATGGTTGGTTGGTGGGGTATTAATG
TTTAATTACCTGTTTTACAGGCCTGAAATCACTTGGTTTTAGG
[SEQ ID No: 48]

In another embodiment, the non-coding intron is a sequence (133 bp) from the 5'-donor sites of the first intron of the human β-globin gene and the branch and 3'-acceptor sites from the intron of an immunoglobulin gene heavy chain variable region, referred to herein as SEQ ID No: 49, as follows:

GTAAGTATCAAGGTTACAAGACAGGTTTAAGGAGACCAATAGAAACTGGGCTTGTCGAGACAGAGAAGACTCTTGCGT
TTCTGATAGGCACCTATTGGTCTTACTGACATCCACTTTGCCTTTCTCTCCACAG
[SEQ ID No: 49]

In another embodiment, the non-coding intron is a fusion (210 bp) of 5' and 3' nucleotide components of the splice acceptor of the rabbit β-globulin gene 1, referred to herein as SEQ ID No: 50, as follows:

GTGAGCGGGCGGGACGGCCCTTCTCCTCCGGGCTGTAATTAGCGCTTGGTTTAATGACGGCTTGTTTCTTTTCTGTGG
CTGCGTGAAAGCCTTGAGGGGCTCCGGGAGGCTAGAGCCTCTGCTAACCATGTTCATGCCTTCTTCTTTTTCCTACAG
CTCCTGGGCAACGTGCTGGTTATTGTGCTGTCTCATCATTTTGGCAAAGAATTC
[SEQ ID NO. 50]

Therefore, preferably the non-coding intron comprises a nucleic acid sequence substantially as set out in SEQ ID No: 48, 49 or 50, or a fragment or variant thereof. In order to allow the correct folding of the polypeptides encoded by the genetic construct, intracellular trafficking and secretion of the PEDF receptor agonist and anti-complement protein from the target cells, the coding sequence for these proteins is preceded by a novel N-terminal minimal signal peptide coding sequence derived from the sequences of known secreted human proteins. The secretory signal peptides are comprised of a methionine initiator amino acid, a series of 2 or more basic amino acids (arginine or lysine), followed by a series of hydrophobic amino acids (leucine, isoleucine, valine or phenylalanine) and finally a cutting sequence to allow cleavage of the signal peptide from the final mature secreted protein.

Accordingly, in one embodiment, the genetic construct comprises a signal peptide coding sequence. Advantageously, this novel signal peptide coding sequence generated by the inventors, optimises intracellular cleavage and trafficking of the secreted proteins within the cell. Preferably, the genetic construct comprises a first signal peptide coding sequence disposed before the first coding sequence, and a second signal peptide coding sequence disposed before the second coding sequence. Preferably, the first and second signal peptide coding sequences are disposed 5' of the first and second coding sequences, respectively.

In one embodiment, the inventors optimised the signal peptide using the online programme Signal-P 5.0 (http://www.cbs.dtu.dk/services/SignalP/data.php) to increase the level of enzymatic cleavage by the signal peptidase and thereby enhance cell secretion of the mature PEDF. The N-terminal glutamine [Q] and alanine [A] amino acids of the endogenous signal peptide sequence MQALVLLLCIGALLGHSSC [SEQ ID No: 79] have been substituted to basic amino acids, such as arginine [R] or lysine [K] residues and the terminal three amino acids [SSC] were substituted to [VFC].

Accordingly, in one embodiment, the signal peptide coding sequence encodes an amino acid sequence referred herein as SEQ ID No. 51, or a fragment or variant thereof, as follows:
MRRLVLLLCIGALLGHVFC
[SEQ ID No: 51]

Preferably, in this embodiment, the signal peptide coding sequence comprises a nucleotide sequence referred to herein as SEQ ID No: 52, or a fragment or variant thereof, as follows:
ATGCGCCGCCTGGTGCTGCTGCTGTGCATCGGCGCCCTGCTGGGCCACGTGTTCTGC
[SEQ ID No: 52]

In order for the anti-complement protein (the anti-C3b, anti-Bb or anti-C5 single-chain variable fragment, CD55, sCD46, CFHL1 or CFHR1) to be released from the producer cell, the coding sequence is proceeded by a modified form of the signal peptide from human brain-derived neurotrophic factor (BDNF) in which the N-terminal amino acids have been substituted with arginine [R] or lysine [K].

Accordingly, in one embodiment, the signal peptide coding sequence encodes an amino acid sequence referred to herein as SEQ ID No: 53 or a fragment or variant thereof, as follows:
MRRFLTVISFLLYFGCAFA
[SEQ ID No: 53]

Preferably, in this embodiment, the signal peptide coding sequence comprises a nucleotide sequence referred to herein as SEQ ID No: 54, or a fragment or variant thereof, as follows:
ATGCGCCGCTTCCTGACCGTGATCAGCTTCCTGCTGTACTTCGGCTGCGCCTTCGCC
[SEQ ID No: 54]

Therefore, preferably the signal peptide coding sequence comprises a nucleotide sequence substantially as set out in SEQ ID No: 52 or 54, or a fragment or variant thereof. Preferably, the signal peptide coding sequence encodes an amino acid sequence substantially as set out in SEQ ID No: 51 or 53, or a fragment or variant thereof.

In a preferred embodiment, the genetic construct may comprise, in this specified order, a 5' promoter; a first coding sequence encoding a PEDF receptor agonist; and a 3' second coding sequence encoding an anti-complement protein. The use of 5' and 3' indicates that the features are either upstream or downstream, and is not intended to indicate that the features are necessarily terminal features. Furthermore, the skilled person would understand that the first and second coding sequences encoding a PEDF receptor agonist and an anti-complement protein may be disposed in any 5' to 3' order.

In a particular embodiment, the genetic construct may comprise in this specified order, a 5' promoter; a first coding sequence encoding a PEDF receptor agonist; a spacer sequence; and a 3' second coding sequence encoding an anti-complement protein.

In a particular embodiment, the genetic construct may comprise in this specified order, a 5' promoter; a first coding sequence encoding a PEDF receptor agonist; a viral-2A removal sequence; a spacer sequence; and a 3' second coding sequence encoding an anti-complement protein.

In a particular embodiment, the genetic construct may comprise in this specified order, a 5' ITR; a promoter; a first coding sequence encoding a PEDF receptor agonist; a viral-2A removal sequence; a spacer sequence; a second coding sequence encoding an anti-complement protein; a sequence encoding WPRE; a sequence encoding a poly A tail; and a 3' ITR.

In a particular embodiment, the genetic construct may comprise in this specified order, a 5' ITR; a promoter; a non-coding intron; a first coding sequence encoding a PEDF receptor agonist; a viral-2A removal sequence; a spacer sequence; a second coding sequence encoding an anti-complement protein; a sequence encoding WPRE; a sequence encoding a poly A tail; and a 3' ITR.

In a particular embodiment, the genetic construct may comprise in this specified order, a 5' ITR; a promoter; a non-coding intron; a first signal peptide coding sequence; a first coding sequence encoding a PEDF receptor agonist; a viral-2A removal sequence; a spacer sequence; a second signal peptide coding sequence; a second coding sequence encoding an anti-complement protein; a sequence encoding WPRE; a sequence encoding a poly A tail; and a 3' ITR.

From the foregoing, the skilled person will appreciate the nucleotide sequence of an embodiment of the construct of the first aspect, as well as the amino acid sequence of the encoded transgene. However, for the avoidance of doubt, in one embodiment, the amino acid sequence of PEDF-furin-P2A-anti-C3b SCVF, is referred to herein as SEQ ID No: 55, as follows:

MRRLVLLLCIGALLGHVFCQNPASPPEEGSPDPDSTGALVEEEDPFFKVPVNKLAAAVSNFGYDLYRVRSSTSPTTNVLLS
PLSVATALSALSLGAEQRTESIIHRALYYDLISSPDIHGTYKELLDTVTAPQKNLKSASRIVFEKKLRIKSSFVAPLEKSY
GTRPRVLTGNPRLDLQEINNWVQAQMKGKLARSTKEIPDEISILLLGVAHFKGQWVTKFDSRKTSLEDFYLDEERTVRVPM
MSDPKAVLRYGLDSDLSCKIAQLPLTGSMSIIFFLPLKVTQNLTLIEESLTSEFIHDIDRELKTVQAVLTVPKLKLSYEGE
VTKSLQEMKLQSLFDSPDFSKITGKPIKLTQVEHRAGFEWNEDGAGTTPSPGLQPAHLTFPLDYHLNQPFIFVLRDTDTGA
LLFIGKILDPRGPRRSKRSGSGATNFSLLKQAGDVEENPGPMRRLLTFISILALVGAFADIQMTQSPSSLSASVGDRVTIT
CRASQDVSTAVAWYQQKPGKAPKLLIYSASFLYSGVPSRFSGSGSGTDFTLTISSLQPEDFATYYCQQSYATLPTFEQGTK
VEIKRGGGGSGGGGSGGGGSGGGGSEVQLVESGGGLVQPGGSLRLSCAASGFSFTSSSVSPGKGLEWVGLIYPYNGFNYY
ADSVKGRFTISADTSLQMNSLRAEDTAVYYCARNALYGSGGYYAMDYWGQGTLVTVSS
[SEQ ID No: 55]

Preferably, in this embodiment, the construct comprises a 2121 nucleotide sequence (contained within the plasmid IKC157P), which is referred to herein as SEQ ID No: 56, or a fragment or variant thereof, as follows:

ATGCGCCGCCTGGTGCTGCTGCTGTGCATCGGCGCCCTGCTGGGCCACGTGTTCTGCCAGAACCCCGCCAGCCCCCCCGAG
GAGGGCAGCCCCGACCCCGACAGCACCGGCGCCCTGGTGGAGGAGGAGGACCCCTTCTTCAAGGTGCCCGTGAACAAGCTG
GCCGCCGCCGTGAGCAACTTCGGCTACGACCTGTACCGCGTGCGCAGCAGCACCAGCCCCACCACCAACGTGCTGCTGAGC
CCCCTGAGCGTGGCCACCGCCCTGAGCGCCCTGAGCCTGGGCGCCGAGCAGCGCACCGAGAGCATCATCCACCGCGCCCTG
TACTACGACCTGATCAGCAGCCCCGACATCCACGGCACCTACAAGGAGCTGCTGGACACCGTGACCGCCCCCCAGAAGAAC
CTGAAGAGCGCCAGCCGCATCGTGTTCGAGAAGAAGCTGCGCATCAAGAGCAGCTTCGTGGCCCCCCTGGAGAAGAGCTAC
GGCACCCGCCCCCGCGTGCTGACCGGCAACCCCCGCCTGGACCTGCAGGAGATCAACAACTGGGTGCAGGCCCAGATGAAG
GGCAAGCTGGCCCGCAGCACCAAGGAGATCCCCGACGAGATCAGCATCCTGCTGCTGGGCGTGGCCCACTTCAAGGGCCAG
TGGGTGACCAAGTTCGACAGCCGCAAGACCAGCCTGGAGGACTTCTACCTGGACGAGGAGCGCACCGTGCGCGTGCCCATG
ATGAGCGACCCCAAGGCCGTGCTGCGCTACGGCCTGGACAGCGACCTGAGCTGCAAGATCGCCCAGCTGCCCCTGACCGGC
AGCATGAGCATCATCTTCTTCCTGCCCCTGAAGGTGACCCAGAACCTGACCCTGATCGAGGAGAGCCTGACCAGCGAGTTC
ATCCACGACATCGACCGCGAGCTGAAGACCGTGCAGGCCGTGCTGACCGTGCCCAAGCTGAAGCTGAGCTACGAGGGCGAG
GTGACCAAGAGCCTGCAGGAGATGAAGCTGCAGAGCCTGTTCGACAGCCCCGACTTCAGCAAGATCACCGGCAAGCCCATC
AAGCTGACCCAGGTGGAGCACCGCGCCGGCTTCGAGTGGAACGAGGACGGCGCCGGCACCACCCCCAGCCCCGGCCTGCAG
CCCGCCCACCTGACCTTCCCCCTGGACTACCACCTGAACCAGCCCTTCATCTTCGTGCTGCGCGACACCGACACCGGCGCC
CTGCTGTTCATCGGCAAGATCCTGGACCCCCGCGGCCCCCGCCGCAGCAAGCGCAGCGGCAGCGGCGCCACCAACTTCAGC
CTGCTGAAGCAGGCCGGCGACGTGGAGGAGAACCCCGGCCCCATGCGCCGCCTGCTGACCTTCATCAGCATCCTGGCCCTG
GTGGGCGCCTTCGCCGACATCCAGATGACCCAGAGCCCCAGCAGCCTGAGCGCCAGCGTGGGCGACCGCGTGACCATCACC
TGCCGCGCCAGCCAGGACGTAAGCACCGCCGTGGCCTGGTACCAGCAGAAGCCCGGCAAGGCCCCCAAGCTGCTGATCTAC
AGCGCCAGCTTCCTGTACAGCGGCGTGCCCAGCCGCTTCAGCGGCAGCGGCAGCGGCACCGACTTCACCCTGACCATCAGC
AGCCTGCAGCCCGAGGACTTCGCCACCTACTACTGCCAGCAGAGCTACGCCACCCTGCCCACCTTCGAGCAGGGCACCAAG
GTGGAGATCAAGCGCGGCGGCGGCGGCAGCGGCGGCGGCAGCGGCGGCGGCAGCGGCGGCGGCAGCGAG
GTGCAGCTGGTGGAGAGCGGCGGCGGCCTGGTGCAGCCCGGCGGCAGCCTGCGCCTGAGCTGCGCCGCCAGCGGCTTCAGC
TTCACCAGCAGCAGCGTGAGCCCCGGCAAGGGCCTGGAGTGGGTGGGCCTGATCTACCCCTACAACGGCTTCAACTACTAC
GCCGACAGCGTGAAGGGCCGCTTCACCATCAGCGCCGACACCAGCCTGCAGATGAACAGCCTGCGCGCCGAGGACACCGCC
GTGTACTACTGCGCCCGCAACGCCCTGTACGGCAGCGGCGGCTACTACGCCATGGACTACTGGGGCCAGGGCACCCTGGTG
ACCGTGAGCAGCTGA

[SEQ ID No: 56]

In another embodiment, the amino acid sequence of PEDF-furin-P2A-sCD55, is referred to herein as SEQ ID No: 57, or a fragment or variant thereof, as follows:

MRRLVLLLCIGALLGHVFCQNPASPPEEGSPDPDSTGALVEEEDPFFKVPVNKLAAAVSNFGYDLYRVRSSTSPTTNV
LLSPLSVATALSALSLGAEQRTESIIHRALYYDLISSPDIHGTYKELLDTVTAPQKNLKSASRIVFEKKLRIKSSFVA
PLEKSYGTRPRVLTGNPRLDLQEINNWVQAQMKGKLARSTKEIPDEISILLLGVAHFKGQWVTKFDSRKTSLEDFYLD
EERTVRVPMMSDPKAVLRYGLDSDLSCKIAQLPLTGSMSIIFFLPLKVTQNLTLIEESLTSEFIHDIDRELKTVQAVL
TVPKLKLSYEGEVTKSLQEMKLQSLFDSPDFSKITGKPIKLTQVEHRAGFEWNEDGAGTTPSPGLQPAHLTFPLDYHL
NQPFIFVLRDTDTGALLFIGKILDPRGPRRSKRSGSGATNFSLLKQAGDVEENPGPMRRFLTVISFLLYFGCAFADCG
LPPDVPNAQPALEGRTSFPEDTVITYKCEESFVKIPGEKDSVICLKGSQWSDIEEFCNRSCEVPTRLNSASLKQPYIT
QNYFPVGTVVEYECRPGYRREPSLSPKLTCLQNLKWSTAVEFCKKKSCPNPGEIRNGQIDVPGGILFGATISFSCNTG
YKLFGSTSSFCLISGSSVQWSDPLPECREIYCPAPPQIDNGIIQGERDHYGYRQSVTYACNKGFTMIGEHSIYCTVNN
DEGEWSGPPPECRGKSLTSKVPPTVQKPTTVNVPTTEVSPTSQKTTTKTTTPNAQATRSTPVSRTTKHFHETTPNKGS
GTTSG
[SEQ ID No: 57]

Preferably, in this embodiment, the construct comprises a 2358 nucleotide sequence (as contained within the plasmid IKC158P), which is referred to herein as SEQ ID No: 58, or a fragment or variant thereof, as follows:

ATGCGCCGCCTGGTGCTGCTGCTGTGCATCGGCGCCCTGCTGGGCCACGTGTTCTGCCAGAACCCCGCCAGCCCCCCC
GAGGAGGGCAGCCCCGACCCCGACAGCACCGGCGCCCTGGTGGAGGAGGAGGACCCCTTCTTCAAGGTGCCCGTGAAC
AAGCTGGCCGCCGCCGTGAGCAACTTCGGCTACGACCTGTACCGCGTGCGCAGCAGCACCAGCCCCACCACCAACGTG
CTGCTGAGCCCCCTGAGCGTGGCCACCGCCCTGAGCGCCCTGAGCCTGGGCGCCGAGCAGCGCACCGAGAGCATCATC
CACCGCGCCCTGTACTACGACCTGATCAGCAGCCCCGACATCCACGGCACCTACAAGGAGCTGCTGGACACCGTGACC
GCCCCCCAGAAGAACCTGAAGAGCGCCAGCCGCATCGTGTTCGAGAAGAAGCTGCGCATCAAGAGCAGCTTCGTGGCC
CCCCTGGAGAAGAGCTACGGCACCCGCCCCCGCGTGCTGACCGGCAACCCCCGCCTGGACCTGCAGGAGATCAACAAC
TGGGTGCAGGCCCAGATGAAGGGCAAGCTGGCCCGCAGCACCAAGGAGATCCCCGACGAGATCAGCATCCTGCTGCTG
GGCGTGGCCCACTTCAAGGGCCAGTGGGTGACCAAGTTCGACAGCCGCAAGACCAGCCTGGAGGACTTCTACCTGGAC
GAGGAGCGCACCGTGCGCGTGCCCATGATGAGCGACCCCAAGGCCGTGCTGCGCTACGGCCTGGACAGCGACCTGAGC
TGCAAGATCGCCCAGCTGCCCCTGACCGGCAGCATGAGCATCATCTTCTTCCTGCCCCTGAAGGTGACCCAGAACCTG
ACCCTGATCGAGGAGAGCCTGACCAGCGAGTTCATCCACGACATCGACCGCGAGCTGAAGACCGTGCAGGCCGTGCTG
ACCGTGCCCAAGCTGAAGCTGAGCTACGAGGGCGAGGTGACCAAGAGCCTGCAGGAGATGAAGCTGCAGAGCCTGTTC
GACAGCCCCGACTTCAGCAAGATCACCGGCAAGCCCATCAAGCTGACCCAGGTGGAGCACCGCGCCGGCTTCGAGTGG
AACGAGGACGGCGCCGGCACCACCCCCAGCCCCGGCCTGCAGCCCGCCCACCTGACCTTCCCCCTGGACTACCACCTG
AACCAGCCCTTCATCTTCGTGCTGCGCGACACCGACACCGGCGCCCTGCTGTTCATCGGCAAGATCCTGGACCCCCGC
GGCCCCCGCCGCAGCAAGCGCAGCGGCAGCGGCGCCACCAACTTCAGCCTGCTGAAGCAGGCCGGCGACGTGGAGGAG
AACCCCGGCCCCATGCGCCGCTTCCTGACCGTGATCAGCTTCCTGCTGTACTTCGGCTGCGCCTTCGCCGACTGCGGC
CTGCCCCCCGACGTGCCCAACGCCCAGCCCGCCCTGGAGGGCCGCACCAGCTTCCCCGAGGACACCGTGATCACCTAC

AAGTGCGAGGAGAGCTTCGTGAAGATCCCCGGCGAGAAGGACAGCGTGATCTGCCTGAAGGGCAGCCAGTGGAGCGAC
ATCGAGGAGTTCTGCAACCGCAGCTGCGAGGTGCCCACCCGCCTGAACAGCGCCAGCCTGAAGCAGCCCTACATCACC
CAGAACTACTTCCCCGTGGGCACCGTGGTGGAGTACGAGTGCCGCCCCGGCTACCGCCGCGAGCCCAGCCTGAGCCCC
AAGCTGACCTGCCTGCAGAACCTGAAGTGGAGCACCGCCGTGGAGTTCTGCAAGAAGAAGAGCTGCCCCAACCCCGGC
GAGATCCGCAACGGCCAGATCGACGTGCCCGGCGGCATCCTGTTCGGCGCCACCATCAGCTTCAGCTGCAACACCGGC
TACAAGCTGTTCGGCAGCACCAGCAGCTTCTGCCTGATCAGCGGCAGCAGCGTGCAGTGGAGCGACCCCCTGCCCGAG
TGCCGCGAGATCTACTGCCCCGCCCCCCCCCAGATCGACAACGGCATCATCCAGGGCGAGCGCGACCACTACGGCTAC
CGCCAGAGCGTGACCTACGCCTGCAACAAGGGCTTCACCATGATCGGCGAGCACAGCATCTACTGCACCGTGAACAAC
GACGAGGGCGAGTGGAGCGGCCCCCCCCCCGAGTGCCGAGGCAAGAGCCTGACCAGCAAGGTGCCCCCCACCGTGCAG
AAGCCCACCACCGTGAACGTGCCCACCACCGAGGTGAGCCCCACCAGCCAGAAGACCACCACCAAGACCACCACCCCC
AACGCCCAGGCCACCCGCAGCACCCCCGTGAGCCGCACCACCAAGCACTTCCACGAGACCACCCCCAACAAGGGCAGC
GGCACCACCAGCGGCTAA

[SEQ ID No: 58]

In another embodiment, the amino acid sequence of sCD55-furin-P2A-PEDF is referred to herein as SEQ ID No: 59, or a fragment or variant thereof, as follows:

MRRFLTVISFLLYFGCAFADCGLPPDVPNAQPALEGRTSFPEDTVITYKCEESFVKIPGEKDSVICLKGSQWSDIEEF
CNRSCEVPTRLNSASLKQPYITQNYFPVGTVVEYECRPGYRREPSLSPKLTCLQNLKWSTAVEFCKKKSCPNPGEIRN
GQIDVPGGILFGATISFSCNTGYKLFGSTSSFCLISGSSVQWSDPLPECREIYCPAPPQIDNGIIQGERDHYGYRQSV
TYACNKGFTMIGEHSIYCTVNNDEGEWSGPPPECRGKSLTSKVPPTVQKPTTVNVPTTEVSPTSQKTTTKTTTPNAQA
TRSTPVSRTTKHFHETTPNKGSGTTSGRRSKRSGSGATNFSLLKQAGDVEENPGPMRRLVLLLCIGALLGHVFCQNPA
SPPEEGSPDPDSTGALVEEEDPFFKVPVNKLAAAVSNFGYDLYRVRSSTSPTTNVLLSPLSVATALSALSLGAEQRTE
SIIHRALYYDLISSPDIHGTYKELLDTVTAPQKNLKSASRIVFEKKLRIKSSFVAPLEKSYGTRPRVLTGNPRLDLQE
INNWVQAQMKGKLARSTKEIPDEISILLLGVAHFKGQWVTKFDSRKTSLEDFYLDEERTVRVPMMSDPKAVLRYGLDS
DLSCKIAQLPLTGSMSIIFFLPLKVTQNLTLIEESLTSEFIHDIDRELKTVQAVLTVPKLKLSYEGEVTKSLQEMKLQ
SLFDSPDFSKITGKPIKLTQVEHRAGFEWNEDGAGTTPSPGLQPAHLTFPLDYHLNQPFIFVLRDTDTGALLFIGKIL
DPRGP
[SEQ ID No: 59]

Preferably, in this embodiment, the construct comprises a 2358 nucleotide sequence (as contained within the plasmid IKC126P), which is referred to herein as SEQ ID No: 60, or a fragment or variant thereof, as follows:

ATGCGCCGCTTCCTGACCGTGATCAGCTTCCTGCTGTACTTCGGCTGCGCCTTCGCCGACTGCGGCCTGCCCCCCGAC
GTGCCCAACGCCCAGCCCGCCCTGGAGGGCCGCACCAGCTTCCCCGAGGACACCGTGATCACCTACAAGTGCGAGGAG
AGCTTCGTGAAGATCCCCGGCGAGAAGGACAGCGTGATCTGCCTGAAGGGCAGCCAGTGGAGCGACATCGAGGAGTTC
TGCAACCGCAGCTGCGAGGTGCCCACCCGCCTGAACAGCGCCAGCCTGAAGCAGCCCTACATCACCCAGAACTACTTC
CCCGTGGGCACCGTGGTGGAGTACGAGTGCCGCCCCGGCTACCGCCGCGAGCCCAGCCTGAGCCCCAAGCTGACCTGC
CTGCAGAACCTGAAGTGGAGCACCGCCGTGGAGTTCTGCAAGAAGAAGAGCTGCCCCAACCCCGGCGAGATCCGCAAC
GGCCAGATCGACGTGCCCGGCGGCATCCTGTTCGGCGCCACCATCAGCTTCAGCTGCAACACCGGCTACAAGCTGTTC

GGCAGCACCAGCAGCTTCTGCCTGATCAGCGGCAGCAGCGTGCAGTGGAGCGACCCCCTGCCCGAGTGCCGCGAGATC
TACTGCCCCGCCCCCCCCCAGATCGACAACGGCATCATCCAGGGCGAGCGCGACCACTACGGCTACCGCCAGAGCGTG
ACCTACGCCTGCAACAAGGGCTTCACCATGATCGGCGAGCACAGCATCTACTGCACCGTGAACAACGACGAGGGCGAG
TGGAGCGGCCCCCCCCCCGAGTGCCGAGGCAAGAGCCTGACCAGCAAGGTGCCCCCCACCGTGCAGAAGCCCACCACC
GTGAACGTGCCCACCACCGAGGTGAGCCCCACCAGCCAGAAGACCACCACCAAGACCACCACCCCCAACGCCCAGGCC
ACCCGCAGCACCCCCGTGAGCCGCACCACCAAGCACTTCCACGAGACCACCCCCAACAAGGGCAGCGGCACCACCAGC
GGCCGCCGCAGCAAGCGCAGCGGCAGCGGCGCCACCAACTTCAGCCTGCTGAAGCAGGCCGGCGACGTGGAGGAGAAC
CCCGGCCCCATGCGCCGCCTGGTGCTGCTGCTGTGCATCGGCGCCCTGCTGGGCCACGTGTTCTGCCAGAACCCCGCC
AGCCCCCCCGAGGAGGGCAGCCCCGACCCCGACAGCACCGGCGCCCTGGTGGAGGAGGAGGACCCCTTCTTCAAGGTG
CCCGTGAACAAGCTGGCCGCCGCCGTGAGCAACTTCGGCTACGACCTGTACCGCGTGCGCAGCAGCACCAGCCCCACC
ACCAACGTGCTGCTGAGCCCCCTGAGCGTGGCCACCGCCCTGAGCGCCCTGAGCCTGGGCGCCGAGCAGCGCACCGAG
AGCATCATCCACCGCGCCCTGTACTACGACCTGATCAGCAGCCCCGACATCCACGGCACCTACAAGGAGCTGCTGGAC
ACCGTGACCGCCCCCCAGAAGAACCTGAAGAGCGCCAGCCGCATCGTGTTCGAGAAGAAGCTGCGCATCAAGAGCAGC
TTCGTGGCCCCCCTGGAGAAGAGCTACGGCACCCGCCCCCGCGTGCTGACCGGCAACCCCCGCCTGGACCTGCAGGAG
ATCAACAACTGGGTGCAGGCCCAGATGAAGGGCAAGCTGGCCCGCAGCACCAAGGAGATCCCCGACGAGATCAGCATC
CTGCTGCTGGGCGTGGCCCACTTCAAGGGCCAGTGGGTGACCAAGTTCGACAGCCGCAAGACCAGCCTGGAGGACTTC
TACCTGGACGAGGAGCGCACCGTGCGCGTGCCCATGATGAGCGACCCCAAGGCCGTGCTGCGCTACGGCCTGGACAGC
GACCTGAGCTGCAAGATCGCCCAGCTGCCCCTGACCGGCAGCATGAGCATCATCTTCTTCCTGCCCCTGAAGGTGACC
CAGAACCTGACCCTGATCGAGGAGAGCCTGACCAGCGAGTTCATCCACGACATCGACCGCGAGCTGAAGACCGTGCAG
GCCGTGCTGACCGTGCCCAAGCTGAAGCTGAGCTACGAGGGCGAGGTGACCAAGAGCCTGCAGGAGATGAAGCTGCAG
AGCCTGTTCGACAGCCCCGACTTCAGCAAGATCACCGGCAAGCCCATCAAGCTGACCCAGGTGGAGCACCGCGCCGGC
TTCGAGTGGAACGAGGACGGCGCCGGCACCACCCCCAGCCCCGGCCTGCAGCCCGCCCACCTGACCTTCCCCCTGGAC
TACCACCTGAACCAGCCCTTCATCTTCGTGCTGCGCGACACCGACACCGGCGCCCTGCTGTTCATCGGCAAGATCCTG
GACCCCCGCGGCCCCTAA
[SEQ ID No: 60]

In another embodiment, the amino acid sequence of PEDF-furin-P2A-CFHR1 is referred to herein as SEQ ID No: 61, or a fragment or variant thereof, as follows:

MRRLVLLLCIGALLGHVFCQNPASPPEEGSPDPDSTGALVEEEDPFFKVPVNKLAAAVSNFGYDLYRVRSSTSPTTNV
LLSPLSVATALSALSLGAEQRTESIIHRALYYDLISSPDIHGTYKELLDTVTAPQKNLKSASRIVFEKKLRIKSSFVA
PLEKSYGTRPRVLTGNPRLDLQEINNWVQAQMKGKLARSTKEIPDEISILLLGVAHFKGQWVTKFDSRKTSLEDFYLD
EERTVRVPMMSDPKAVLRYGLDSDLSCKIAQLPLTGSMSIIFFLPLKVTQNLTLIEESLTSEFIHDIDRELKTVQAVL
TVPKLKLSYEGEVTKSLQEMKLQSLFDSPDFSKITGKPIKLTQVEHRAGFEWNEDGAGTTPSPGLQPAHLTFPLDYHL
NQPFIFVLRDTDTGALLFIGKILDPRGPRRSKRSGSGATNFSLLKQAGDVEENPGPMRRFLTVISFLLYFGCAFAEAT
FCDFPKINHGILYDEEKYKPFSQVPTGEVFYYSCEYNFVSPSKSFWTRITCTEEGWSPTPKCLRLCFFPFVENGHSES
SGQTHLEGDTVQIICNTGYRLQNNENNISCVERGWSTPPKCRSTDTSCVNPPTVQNAHILSRQMSKYPSGERVRYECR
SPYEMFGDEEVMCLNGNWTEPPQCKDSTGKCGPPPPIDNGDITSFPLSVYAPASSVEYQCQNLYQLEGNKRITCRNGQ
WSEPPKCLHPCVISREIMENYNIALRWTAKQKLYLRTGESAEFVCKRGYRLSSRSHTLRTTCWDGKLEYPTCAKR
[SEQ ID No: 61]

Preferably, in this embodiment, the construct comprises a 2334 nucleotide sequence (as contained within the plasmid IKC127P), which is referred to herein as SEQ ID No: 62, or a fragment or variant thereof, as follows:

ATGCGCCGCCTGGTGCTGCTGCTGTGCATCGGCGCCCTGCTGGGCCACGTGTTCTGCCAGAACCCCGCCAGCCCCCCCGAG
GAGGGCAGCCCCGACCCCGACAGCACCGGCGCCCTGGTGGAGGAGGAGGACCCCTTCTTCAAGGTGCCCGTGAACAAGCTG
GCCGCCGCCGTGAGCAACTTCGGCTACGACCTGTACCGCGTGCGCAGCAGCACCAGCCCCACCACCAACGTGCTGCTGAGC
CCCCTGAGCGTGGCCACCGCCCTGAGCGCCCTGAGCCTGGGCGCCGAGCAGCGCACCGAGAGCATCATCCACCGCGCCCTG
TACTACGACCTGATCAGCAGCCCCGACATCCACGGCACCTACAAGGAGCTGCTGGACACCGTGACCGCCCCCCAGAAGAAC
CTGAAGAGCGCCAGCCGCATCGTGTTCGAGAAGAAGCTGCGCATCAAGAGCAGCTTCGTGGCCCCCCTGGAGAAGAGCTAC
GGCACCCGCCCCCGCGTGCTGACCGGCAACCCCCGCCTGGACCTGCAGGAGATCAACAACTGGGTGCAGGCCCAGATGAAG
GGCAAGCTGGCCCGCAGCACCAAGGAGATCCCCGACGAGATCAGCATCCTGCTGCTGGGCGTGGCCCACTTCAAGGGCCAG
TGGGTGACCAAGTTCGACAGCCGCAAGACCAGCCTGGAGGACTTCTACCTGGACGAGGAGCGCACCGTGCGCGTGCCCATG
ATGAGCGACCCCAAGGCCGTGCTGCGCTACGGCCTGGACAGCGACCTGAGCTGCAAGATCGCCCAGCTGCCCCTGACCGGC
AGCATGAGCATCATCTTCTTCCTGCCCCTGAAGGTGACCCAGAACCTGACCCTGATCGAGGAGAGCCTGACCAGCGAGTTC
ATCCACGACATCGACCGCGAGCTGAAGACCGTGCAGGCCGTGCTGACCGTGCCCAAGCTGAAGCTGAGCTACGAGGGCGAG
GTGACCAAGAGCCTGCAGGAGATGAAGCTGCAGAGCCTGTTCGACAGCCCCGACTTCAGCAAGATCACCGGCAAGCCCATC
AAGCTGACCCAGGTGGAGCACCGCGCCGGCTTCGAGTGGAACGAGGACGGCGCCGGCACCACCCCCAGCCCCGGCCTGCAG
CCCGCCCACCTGACCTTCCCCCTGGACTACCACCTGAACCAGCCCTTCATCTTCGTGCTGCGCGACACCGACACCGGCGCC
CTGCTGTTCATCGGCAAGATCCTGGACCCCCGCGGCCCCCGCCGCAGCAAGCGCAGCGGCAGCGGCGCCACCAACTTCAGC
CTGCTGAAGCAGGCCGGCGACGTGGAGGAGAACCCCGGCCCCATGCGCCGCTTCCTGACCGTGATCAGCTTCCTGCTGTAC
TTCGGCTGCGCCTTCGCCGAGGCCACCTTCTGCGACTTCCCCAAGATCAACCACGGCATCCTGTACGACGAGGAGAAGTAC
AAGCCCTTCAGCCAGGTGCCCACCGGCGAGGTGTTCTACTACAGCTGCGAGTACAACTTCGTGAGCCCCAGCAAGAGCTTC
TGGACCCGCATCACCTGCACCGAGGAGGGCTGGAGCCCCACCCCCAAGTGCCTGCGCCTGTGCTTCTTCCCCTTCGTGGAG
AACGGCCACAGCGAGAGCAGCGGCCAGACCCACCTGGAGGGCGACACCGTGCAGATCATCTGCAACACCGGCTACCGCCTG
CAGAACAACGAGAACAACATCAGCTGCGTGGAGCGCGGCTGGAGCACCCCCCCCAAGTGCCGCAGCACCGACACCAGCTGC
GTGAACCCCCCCACCGTGCAGAACGCCCACATCCTGAGCCGCCAGATGAGCAAGTACCCCAGCGGCGAGCGCGTGCGCTAC
GAGTGCCGCAGCCCCTACGAGATGTTCGGCGACGAGGAGGTGATGTGCCTGAACGGCAACTGGACCGAGCCCCCCCAGTGC
AAGGACAGCACCGGCAAGTGCGGCCCCCCCCCCCCCATCGACAACGGCGACATCACCAGCTTCCCCCTGAGCGTGTACGCC
CCCGCCAGCAGCGTGGAGTACCAGTGCCAGAACCTGTACCAGCTGGAGGGCAACAAGCGCATCACCTGCCGCAACGGCCAG
TGGAGCGAGCCCCCCAAGTGCCTGCACCCCTGCGTGATCAGCCGCGAGATCATGGAGAACTACAACATCGCCCTGCGCTGG
ACCGCCAAGCAGAAGCTGTACCTGCGCACCGGCGAGAGCGCCGAGTTCGTGTGCAAGCGCGGCTACCGCCTGAGCAGCCGC
AGCCACACCCTGCGCACCACCTGCTGGGACGGCAAGCTGGAGTACCCCACCTGCGCCAAGCGCTAA
[SEQ ID No: 62]

In another embodiment, the amino acid sequence of CFHR1-furin-P2A- PEDF, is referred to herein as SEQ ID No: 63 or a fragment or variant thereof, as follows:

MRRFLTVISFLLYFGCAFAEATFCDFPKINHGILYDEEKYKPFSQVPTGEVFYYSCEYNFVSPSKSFWTRITCTEEGW
SPTPKCLRLCFFPFVENGHSESSGQTHLEGDTVQIICNTGYRLQNNENNISCVERGWSTPPKCRSTDTSCVNPPTVQN
AHILSRQMSKYPSGERVRYECRSPYEMFGDEEVMCLNGNWTEPPQCKDSTGKCGPPPPIDNGDITSFPLSVYAPASSV
EYQCQNLYQLEGNKRITCRNGQWSEPPKCLHPCVISREIMENYNIALRWTAKQKLYLRTGESAEFVCKRGYRLSSRSH

TLRTTCWDGKLEYPTCAKRSKRSGSATNFSLLKQAGDVEENPGPMRRLVLLLCIGALLGHVFCQNPASPPEEGSPDPD
STGALVEEEDPFFKVPVNKLAAAVSNFGYDLYRVRSSTSPTTNVLLSPLSVATALSALSLGAEQRTESIIHRALYYDL
ISSPDIHGTYKELLDTVTAPQKNLKSASRIVFEKKLRIKSSFVAPLEKSYGTRPRVLTGNPRLDLQEINNWVQAQMKG
KLARSTKEIPDEISILLLGVAHFKGQWVTKFDSRKTSLEDFYLDEERTVRVPMMSDPKAVLRYGLDSDLSCKIAQLPL
TGSMSIIFFLPLKVTQNLTLIEESLTSEFIHDIDRELKTVQAVLTVPKLKLSYEGEVTKSLQEMKLQSLFDSPDFSKI
TGKPIKLTQVEHRAGFEWNEDGAGTTPSPGLQPAHLTFPLDYHLNQPFIFVLRDTDTGALLFIGKILDPRGP
[SEQ ID No: 63]

Preferably, in this embodiment, the construct comprises a 2325 nucleotide sequence (as contained within the plasmid IKC128P), which is referred to herein as SEQ ID No: 64, or a fragment or variant thereof, as follows:

ATGCGCCGCCTGCTGACCTTCATCAGCATCCTGGCCCTGGTGGGCGCCTTCGCCGAGGCCACCTTCTGCGACTTCCCC
AAGATCAACCACGGCATCCTGTACGACGAGGAGAAGTACAAGCCCTTCAGCCAGGTGCCCACCGGCGAGGTGTTCTAC
TACAGCTGCGAGTACAACTTCGTGAGCCCCAGCAAGAGCTTCTGGACCCGCATCACCTGCACCGAGGAGGGCTGGAGC
CCCACCCCCAAGTGCCTGCGCCTGTGCTTCTTCCCCTTCGTGGAGAACGGCCACAGCGAGAGCAGCGGCCAGACCCAC
CTGGAGGGCGACACCGTGCAGATCATCTGCAACACCGGCTACCGCCTGCAGAACAACGAGAACAACATCAGCTGCGTG
GAGCGCGGCTGGAGCACCCCCCCCAAGTGCCGCAGCACCGACACCAGCTGCGTGAACCCCCCCACCGTGCAGAACGCC
CACATCCTGAGCCGCCAGATGAGCAAGTACCCCAGCGGCGAGCGCGTGCGCTACGAGTGCCGCAGCCCCTACGAGATG
TTCGGCGACGAGGAGGTGATGTGCCTGAACGGCAACTGGACCGAGCCCCCCCAGTGCAAGGACAGCACCGGCAAGTGC
GGCCCCCCCCCCCCCATCGACAACGGCGACATCACCAGCTTCCCCCTGAGCGTGTACGCCCCCGCCAGCAGCGTGGAG
TACCAGTGCCAGAACCTGTACCAGCTGGAGGGCAACAAGCGCATCACCTGCCGCAACGGCCAGTGGAGCGAGCCCCCC
AAGTGCCTGCACCCCTGCGTGATCAGCCGCGAGATCATGGAGAACTACAACATCGCCCTGCGCTGGACCGCCAAGCAG
AAGCTGTACCTGCGCACCGGCGAGAGCGCCGAGTTCGTGTGCAAGCGCGGCTACCGCCTGAGCAGCCGCAGCCACACC
CTGCGCACCACCTGCTGGGACGGCAAGCTGGAGTACCCCACCTGCGCCAAGCGCAGCAAGCGCAGCGGCAGCGGCGCC
ACCAACTTCAGCCTGCTGAAGCAGGCCGGCGACGTGGAGGAGAACCCCGGCCCCATGCGCCGCCTGGTGCTGCTGCTG
TGCATCGGCGCCCTGCTGGGCCACGTGTTCTGCCAGAACCCCGCCAGCCCCCCCGAGGAGGGCAGCCCCGACCCCGAC
AGCACCGGCGCCCTGGTGGAGGAGGAGGACCCCTTCTTCAAGGTGCCCGTGAACAAGCTGGCCGCCGCCGTGAGCAAC
TTCGGCTACGACCTGTACCGCGTGCGCAGCAGCACCAGCCCCACCACCAACGTGCTGCTGAGCCCCCTGAGCGTGGCC
ACCGCCCTGAGCGCCCTGAGCCTGGGCGCCGAGCAGCGCACCGAGAGCATCATCCACCGCGCCCTGTACTACGACCTG
ATCAGCAGCCCCGACATCCACGGCACCTACAAGGAGCTGCTGGACACCGTGACCGCCCCCCAGAAGAACCTGAAGAGC
GCCAGCCGCATCGTGTTCGAGAAGAAGCTGCGCATCAAGAGCAGCTTCGTGGCCCCCCTGGAGAAGAGCTACGGCACC
CGCCCCCGCGTGCTGACCGGCAACCCCCGCCTGGACCTGCAGGAGATCAACAACTGGGTGCAGGCCCAGATGAAGGGC
AAGCTGGCCCGCAGCACCAAGGAGATCCCCGACGAGATCAGCATCCTGCTGCTGGGCGTGGCCCACTTCAAGGGCCAG
TGGGTGACCAAGTTCGACAGCCGCAAGACCAGCCTGGAGGACTTCTACCTGGACGAGGAGCGCACCGTGCGCGTGCCC
ATGATGAGCGACCCCAAGGCCGTGCTGCGCTACGGCCTGGACAGCGACCTGAGCTGCAAGATCGCCCAGCTGCCCCTG
ACCGGCAGCATGAGCATCATCTTCTTCCTGCCCCTGAAGGTGACCCAGAACCTGACCCTGATCGAGGAGAGCCTGACC
AGCGAGTTCATCCACGACATCGACCGCGAGCTGAAGACCGTGCAGGCCGTGCTGACCGTGCCCAAGCTGAAGCTGAGC
TACGAGGGCGAGGTGACCAAGAGCCTGCAGGAGATGAAGCTGCAGAGCCTGTTCGACAGCCCCGACTTCAGCAAGATC
ACCGGCAAGCCCATCAAGCTGACCCAGGTGGAGCACCGCGCCGGCTTCGAGTGGAACGAGGACGGCGCCGGCACCACC
CCCAGCCCCGGCCTGCAGCCCGCCCACCTGACCTTCCCCCTGGACTACCACCTGAACCAGCCCTTCATCTTCGTGCTG
CGCGACACCGACACCGGCGCCCTGCTGTTCATCGGCAAGATCCTGGACCCCCGCGGCCCCTAA

[SEQ ID No: 64]

In another embodiment, the amino acid sequence of PEDF-furin-P2A-anti-C5 SCVF, is referred to herein as SEQ ID No: 85, or a fragment or variant thereof, as follows:

MRRLVLLLCIGALLGHSSCQNPASPPEEGSPDPDSTGALVEEEDPFFKVPVNKLAAAVSNFGYDLYRVRSSTSPTTNV
LLSPLSVATALSALSLGAEQRTESIIHRALYYDLISSPDIHGTYKELLDTVTAPQKNLKSASRIVFEKKLRIKSSFVA
PLEKSYGTRPRVLTGNPRLDLQEINNWVQAQMKGKLARSTKEIPDEISILLLGVAHFKGQWVTKFDSRKTSLEDFYLD
EERTVRVPMMSDPKAVLRYGLDSDLSCKIAQLPLTGSMSIIFFLPLKVTQNLTLIEESLTSEFIHDIDRELKTVQAVL
TVPKLKLSYEGEVTKSLQEMKLQSLFDSPDFSKITGKPIKLTQVEHRAGFEWNEDGAGTTPSPGLQPAHLTFPLDYHL
NQPFIFVLRDTDTGALLFIGKILDPRGPRVRRGSGATNFSLLKQAGDVEENPGPMRRLLILALVGAAVADIQMTQSPS
SLSASVGDRVTITCQASQSINNQLSWYQQKPGKAPKLLIYYASTLASGYPSRFSGSGSGTDFTLTISSLQPEDFATYY
CQGSYYSGGWDYGFGQGTKVEIKRGGGGSGGGSGGGGSGGGGSEVQLVESGGGLVQPGGSLRLSCAASGFSFSGRYW
IQWVRQAPGKGLEWVASVWPGITGTNYANWAKGRFTISRDDSKNTLYLQMNSLRAEDTAVYYCAREPVAWGGGLDLWG
QGTLVTVSS
[SEQ ID No: 85]

Preferably, in this embodiment, the construct comprises a 2136 nucleotide sequence (as contained within the plasmid IKC094P), which is referred to herein as SEQ ID No: 86, or a fragment or variant thereof, as follows:

ATGCGCCGCCTGGTGCTGCTGCTGTGCATCGGCGCCCTGCTGGGCCACAGCAGCTGCCAGAACCCCGCCAGCCCCCCC
GAGGAGGGCAGCCCCGACCCCGACAGCACCGGCGCCCTGGTGGAGGAGGAGGACCCCTTCTTCAAGGTGCCCGTGAAC
AAGCTGGCCGCCGCCGTGAGCAACTTCGGCTACGACCTGTACCGCGTGCGCAGCAGCACCAGCCCCACCACCAACGTG
CTGCTGAGCCCCCTGAGCGTGGCCACCGCCCTGAGCGCCCTGAGCCTGGGCGCCGAGCAGCGCACCGAGAGCATCATC
CACCGCGCCCTGTACTACGACCTGATCAGCAGCCCCGACATCCACGGCACCTACAAGGAGCTGCTGGACACCGTGACC
GCCCCCCAGAAGAACCTGAAGAGCGCCAGCCGCATCGTGTTCGAGAAGAAGCTGCGCATCAAGAGCAGCTTCGTGGCC
CCCCTGGAGAAGAGCTACGGCACCCGCCCCCGCGTGCTGACCGGCAACCCCCGCCTGGACCTGCAGGAGATCAACAAC
TGGGTGCAGGCCCAGATGAAGGGCAAGCTGGCCCGCAGCACCAAGGAGATCCCCGACGAGATCAGCATCCTGCTGCTG
GGCGTGGCCCACTTCAAGGGCCAGTGGGTGACCAAGTTCGACAGCCGCAAGACCAGCCTGGAGGACTTCTACCTGGAC
GAGGAGCGCACCGTGCGCGTGCCCATGATGAGCGACCCCAAGGCCGTGCTGCGCTACGGCCTGGACAGCGACCTGAGC
TGCAAGATCGCCCAGCTGCCCCTGACCGGCAGCATGAGCATCATCTTCTTCCTGCCCCTGAAGGTGACCCAGAACCTG
ACCCTGATCGAGGAGAGCCTGACCAGCGAGTTCATCCACGACATCGACCGCGAGCTGAAGACCGTGCAGGCCGTGCTG
ACCGTGCCCAAGCTGAAGCTGAGCTACGAGGGCGAGGTGACCAAGAGCCTGCAGGAGATGAAGCTGCAGAGCCTGTTC
GACAGCCCCGACTTCAGCAAGATCACCGGCAAGCCCATCAAGCTGACCCAGGTGGAGCACCGCGCCGGCTTCGAGTGG
AACGAGGACGGCGCCGGCACCACCCCCAGCCCCGGCCTGCAGCCCGCCCACCTGACCTTCCCCCTGGACTACCACCTG
AACCAGCCCTTCATCTTCGTGCTGCGCGACACCGACACCGGCGCCCTGCTGTTCATCGGCAAGATCCTGGACCCCCGC
GGCCCCCGCGTGCGCCGCGGCAGCGGCGCCACCAACTTCAGCCTGCTGAAGCAGGCCGGCGACGTGGAGGAGAACCCC
GGCCCCATGCGCCGCCTGCTGATCCTGGCCCTGGTGGGCGCCGCCGTGGCCGACATCCAGATGACCCAGAGCCCCAGC
AGCCTGAGCGCCAGCGTGGGCGACCGCGTGACCATCACCTGCCAGGCCAGCCAGAGCATCAACAACCAGCTGAGCTGG
TACCAGCAGAAGCCCGGCAAGGCCCCCAAGCTGCTGATCTACTACGCCAGCACCCTGGCCAGCGGCTACCCCAGCCGC

TTCAGCGGCAGCGGCAGCGGCACCGACTTCACCCTGACCATCAGCAGCCTGCAGCCCGAGGACTTCGCCACCTACTAC
TGCCAGGGCAGCTACTACAGCGGCGGCTGGGACTACGGCTTCGGCCAGGGCACCAAGGTGGAGATCAAGCGCGGCGGC
GGCGGCGGCAGCGGCGGCGGCAGCGGCGGCGGCAGCGGCGGCGGCAGCGAGGTGCAGCTGGTGGAGAGCGGC
GGCGGCCTGGTGCAGCCCGGCGGCAGCCTGCGCCTGAGCTGCGCCGCCAGCGGCTTCAGCTTCAGCGGCCGCTACTGG
ATCCAGTGGGTGCGCCAGGCCCCCGGCAAGGGCCTGGAGTGGGTGGCCAGCGTGTGGCCCGGCATCACCGGCACCAAC
TACGCCAACTGGGCCAAGGGCCGCTTCACCATCAGCCGCGACGACAGCAAGAACACCCTGTACCTGCAGATGAACAGC
CTGCGCGCCGAGGACACCGCCGTGTACTACTGCGCCCGCGAGCCCGTGGCCTGGGGCGGCGGCCTGGACCTGTGGGGC
CAGGGCACCCTGGTGACCGTGAGCAGCTGA
[SEQ ID No: 86]

In another embodiment, the amino acid sequence of PEDF-furin-P2A-anti-Bb SCVF, is referred to herein as SEQ ID No: 65, or a fragment or variant thereof, as follows:

MRRLVLLLCIGALLGHSSCQNPASPPEEGSPDPDSTGALVEEEDPFFKVPVNKLAAAVSNFGYDLYRVRSSTSPTTNV
LLSPLSVATALSALSLGAEQRTESIIHRALYYDLISSPDIHGTYKELLDTVTAPQKNLKSASRIVFEKKLRIKSSFVA
PLEKSYGTRPRVLTGNPRLDLQEINNWVQAQMKGKLARSTKEIPDEISILLLGVAHFKGQWVTKFDSRKTSLEDFYLD
EERTVRVPMMSDPKAVLRYGLDSDLSCKIAQLPLTGSMSIIFFLPLKVTQNLTLIEESLTSEFIHDIDRELKTVQAVL
TVPKLKLSYEGEVTKSLQEMKLQSLFDSPDFSKITGKPIKLTQVEHRAGFEWNEDGAGTTPSPGLQPAHLTFPLDYHL
NQPFIFVLRDTDTGALLFIGKILDPRGPRVRRGSGATNFSLLKQAGDVEENPGPMRRLLILALVGAAVADVQITQSPS
YLAASPGETITINCRASKSISKYLAWYQDKPGKTNKLLIYSGSTLQSGIPSRFSGSGSGTDFTLTISSLEPEDFAMYY
CQQHDEYPWTFGGGTKLEIKRGGGGGSGGGGSGGGGSGGGGSQVQLQQSGAELAKPGASVRMSCKASGYTFTNYWIHW
VKQRPGQGLEWIGYINPNTGYNDYNQKFKDKATLTADKSSSTVYMQLSSLTSEDSAVYYCARGGQLGLRRAMDYWGQG
TSVTVSS
[SEQ ID No: 65]

Preferably, in this embodiment, the construct comprises a 2130 nucleotide sequence (as contained within the plasmid IKC093P), which is referred to herein as SEQ ID No: 66, or a fragment or variant thereof, as follows:

ATGCGCCGCCTGGTGCTGCTGCTGTGCATCGGCGCCCTGCTGGGCCACAGCAGCTGCCAGAACCCCGCCAGCCCCCCC
GAGGAGGGCAGCCCCGACCCCGACAGCACCGGCGCCCTGGTGGAGGAGGAGGACCCCTTCTTCAAGGTGCCCGTGAAC
AAGCTGGCCGCCGCCGTGAGCAACTTCGGCTACGACCTGTACCGCGTGCGCAGCAGCACCAGCCCCACCACCAACGTG
CTGCTGAGCCCCCTGAGCGTGGCCACCGCCCTGAGCGCCCTGAGCCTGGGCGCCGAGCAGCGCACCGAGAGCATCATC
CACCGCGCCCTGTACTACGACCTGATCAGCAGCCCCGACATCCACGGCACCTACAAGGAGCTGCTGGACACCGTGACC
GCCCCCCAGAAGAACCTGAAGAGCGCCAGCCGCATCGTGTTCGAGAAGAAGCTGCGCATCAAGAGCAGCTTCGTGGCC
CCCCTGGAGAAGAGCTACGGCACCCGCCCCCGCGTGCTGACCGGCAACCCCCGCCTGGACCTGCAGGAGATCAACAAC
TGGGTGCAGGCCCAGATGAAGGGCAAGCTGGCCCGCAGCACCAAGGAGATCCCCGACGAGATCAGCATCCTGCTGCTG
GGCGTGGCCCACTTCAAGGGCCAGTGGGTGACCAAGTTCGACAGCCGCAAGACCAGCCTGGAGGACTTCTACCTGGAC
GAGGAGCGCACCGTGCGCGTGCCCATGATGAGCGACCCCAAGGCCGTGCTGCGCTACGGCCTGGACAGCGACCTGAGC
TGCAAGATCGCCCAGCTGCCCCTGACCGGCAGCATGAGCATCATCTTCTTCCTGCCCCTGAAGGTGACCCAGAACCTG
ACCCTGATCGAGGAGAGCCTGACCAGCGAGTTCATCCACGACATCGACCGCGAGCTGAAGACCGTGCAGGCCGTGCTG
ACCGTGCCCAAGCTGAAGCTGAGCTACGAGGGCGAGGTGACCAAGAGCCTGCAGGAGATGAAGCTGCAGAGCCTGTTC

GACAGCCCCGACTTCAGCAAGATCACCGGCAAGCCCATCAAGCTGACCCAGGTGGAGCACCGCGCCGGCTTCGAGTGG
AACGAGGACGGCGCCGGCACCACCCCCAGCCCCGGCCTGCAGCCCGCCCACCTGACCTTCCCCCTGGACTACCACCTG
AACCAGCCCTTCATCTTCGTGCTGCGCGACACCGACACCGGCGCCCTGCTGTTCATCGGCAAGATCCTGGACCCCCGC
GGCCCCCGCGTGCGCCGCGGCAGCGGCGCCACCAACTTCAGCCTGCTGAAGCAGGCCGGCGACGTGGAGGAGAACCCC
GGCCCCATGCGCCGCCTGCTGATCCTGGCCCTGGTGGGCGCCGCCGTGGCCGACGTCCAGATCACCCAGAGCCCCAGC
TACCTGGCCGCCAGCCCCGGCGAGACCATCACCATCAACTGCCGCGCCAGCAAGAGCATCAGCAAGTACCTGGCCTGG
TACCAGGACAAGCCCGGCAAGACCAACAAGCTGCTGATCTACAGCGGCAGCACCCTGCAGAGCGGCATCCCCAGCCGC
TTCAGCGGCAGCGGCAGCGGCACCGACTTCACCCTGACCATCAGCAGCCTGGAGCCCGAGGACTTCGCCATGTACTAC
TGCCAGCAGCACGACGAGTACCCCTGGACCTTCGGCGGCGGCACCAAGCTGGAGATCAAGCGCGGCGGCGGCGGCGGC
AGCGGCGGCGGCGGCAGCGGCGGCGGCGGCAGCGGCGGCGGCGGCAGCCAGGTGCAGCTGCAGCAGAGCGGCGCCGAG
CTGGCCAAGCCCGGCGCCAGCGTGCGCATGAGCTGCAAGGCCAGCGGCTACACCTTCACCAACTACTGGATCCACTGG
GTGAAGCAGCGCCCCGGCCAGGGCCTGGAGTGGATCGGCTACATCAACCCCAACACCGGCTACAACGACTACAACCAG
AAGTTCAAGGACAAGGCCACCCTGACCGCCGACAAGAGCAGCAGCACCGTGTACATGCAGCTGAGCAGCCTGACCAGC
GAGGACAGCGCCGTGTACTACTGCGCCCGCGGCGGCCAGCTGGGCCTGCGCCGCGCCATGGACTACTGGGGCCAGGGC
ACCAGCGTGACCGTGAGCAGCTGA

[SEQ ID No: 66]

In another embodiment, the amino acid sequence of PEDF-furin-P2A-anti-Bb SCVF, is referred to herein as SEQ ID No: 67, or a fragment or variant thereof, as follows:

MRRLVLLLCIGALLGHSSCQNPASPPEEGSPDPDSTGALVEEEDPFFKVPVNKLAAAVSNFGYDLYRVRSSTSPTTNV
LLSPLSVATALSALSLGAEQRTESIIHRALYYDLISSPDIHGTYKELLDTVTAPQKNLKSASRIVFEKKLRIKSSFVA
PLEKSYGTRPRVLTGNPRLDLQEINNWVQAQMKGKLARSTKEIPDEISILLLGVAHFKGQWVTKFDSRKTSLEDFYLD
EERTVRVPMMSDPKAVLRYGLDSDLSCKIAQLPLTGSMSIIFFLPLKVTQNLTLIEESLTSEFIHDIDRELKTVQAVL
TVPKLKLSYEGEVTKSLQEMKLQSLFDSPDFSKITGKPIKLTQVEHRAGFEWNEDGAGTTPSPGLQPAHLTFPLDYHL
NQPFIFVLRDTDTGALLFIGKILDPRGPRRSKRSGSGATNFSLLKQAGDVEENPGPMRRLLILALVGAAVADVQITQS
PSYLAASPGETITINCRASKSISKYLAWYQDKPGKTNKLLIYSGSTLQSGIPSRFSGSGSGTDFTLTISSLEPEDFAM
YYCQQHDEYPWTFGGGTKLEIKRGGGGSGGGGSGGGGSGGGGSQVQLQQSGAELAKPGASVRMSCKASGYTFTNYWI
HWVKQRPGQGLEWIGYINPNTGYNDYNQKFKDKATLTADKSSSTVYMQLSSLTSEDSAVYYCARGGQLGLRRAMDYWG
QGTSVTVSS
[SEQ ID No: 67]

Preferably, in this embodiment, the construct comprises a 2136 nucleotide sequence (as contained within the plasmid IKC129P), which is referred to herein as SEQ ID No: 68, or a fragment or variant thereof, as follows:

ATGCGCCGCCTGGTGCTGCTGCTGTGCATCGGCGCCCTGCTGGGCCACAGCAGCTGCCAGAACCCCGCCAGCCCCCCCGAG
GAGGGCAGCCCCGACCCCGACAGCACCGGCGCCCTGGTGGAGGAGGAGGACCCCTTCTTCAAGGTGCCCGTGAACAAGCTG
GCCGCCGCCGTGAGCAACTTCGGCTACGACCTGTACCGCGTGCGCAGCAGCACCAGCCCCACCACCAACGTGCTGCTGAGC
CCCCTGAGCGTGGCCACCGCCCTGAGCGCCCTGAGCCTGGGCGCCGAGCAGCGCACCGAGAGCATCATCCACCGCGCCCTG
TACTACGACCTGATCAGCAGCCCCGACATCCACGGCACCTACAAGGAGCTGCTGGACACCGTGACCGCCCCCCAGAAGAAC

CTGAAGAGCGCCAGCCGCATCGTGTTCGAGAAGAAGCTGCGCATCAAGAGCAGCTTCGTGGCCCCCCTGGAGAAGAGCTAC
GGCACCCGCCCCCGCGTGCTGACCGGCAACCCCCGCCTGGACCTGCAGGAGATCAACAACTGGGTGCAGGCCCAGATGAAG
GGCAAGCTGGCCCGCAGCACCAAGGAGATCCCCGACGAGATCAGCATCCTGCTGCTGGGCGTGGCCCACTTCAAGGGCCAG
TGGGTGACCAAGTTCGACAGCCGCAAGACCAGCCTGGAGGACTTCTACCTGGACGAGGAGCGCACCGTGCGCGTGCCCATG
ATGAGCGACCCCAAGGCCGTGCTGCGCTACGGCCTGGACAGCGACCTGAGCTGCAAGATCGCCCAGCTGCCCCTGACCGGC
AGCATGAGCATCATCTTCTTCCTGCCCCTGAAGGTGACCCAGAACCTGACCCTGATCGAGGAGAGCCTGACCAGCGAGTTC
ATCCACGACATCGACCGCGAGCTGAAGACCGTGCAGGCCGTGCTGACCGTGCCCAAGCTGAAGCTGAGCTACGAGGGCGAG
GTGACCAAGAGCCTGCAGGAGATGAAGCTGCAGAGCCTGTTCGACAGCCCCGACTTCAGCAAGATCACCGGCAAGCCCATC
AAGCTGACCCAGGTGGAGCACCGCGCCGGCTTCGAGTGGAACGAGGACGGCGCCGGCACCACCCCCAGCCCCGGCCTGCAG
CCCGCCCACCTGACCTTCCCCCTGGACTACCACCTGAACCAGCCCTTCATCTTCGTGCTGCGCGACACCGACACCGGCGCC
CTGCTGTTCATCGGCAAGATCCTGGACCCCCGCGGCCCCCGCCGCAGCAAGCGCAGCGGCAGCGGCGCCACCAACTTCAGC
CTGCTGAAGCAGGCCGGCGACGTGGAGGAGAACCCCGGCCCCATGCGCCGCCTGCTGATCCTGGCCCTGGTGGGCGCCGCC
GTGGCCGACGTCCAGATCACCCAGAGCCCCAGCTACCTGGCCGCCAGCCCCGGCGAGACCATCACCATCAACTGCCGCGCC
AGCAAGAGCATCAGCAAGTACCTGGCCTGGTACCAGGACAAGCCCGGCAAGACCAACAAGCTGCTGATCTACAGCGGCAGC
ACCCTGCAGAGCGGCATCCCCAGCCGCTTCAGCGGCAGCGGCAGCGGCACCGACTTCACCCTGACCATCAGCAGCCTGGAG
CCCGAGGACTTCGCCATGTACTACTGCCAGCAGCACGACGAGTACCCCTGGACCTTCGGCGGCGGCACCAAGCTGGAGATC
AAGCGCGGCGGCGGCGGCGGCAGCGGCGGCGGCGGCAGCGGCGGCGGCGGCAGCGGCGGCGGCAGCCAGGTGCAGCTG
CAGCAGAGCGGCGCCGAGCTGGCCAAGCCCGGCGCCAGCGTGCGCATGAGCTGCAAGGCCAGCGGCTACACCTTCACCAAC
TACTGGATCCACTGGGTGAAGCAGCGCCCCGGCCAGGGCCTGGAGTGGATCGGCTACATCAACCCCAACACCGGCTACAAC
GACTACAACCAGAAGTTCAAGGACAAGGCCACCCTGACCGCCGACAAGAGCAGCAGCACCGTGTACATGCAGCTGAGCAGC
CTGACCAGCGAGGACAGCGCCGTGTACTACTGCGCCCGCGGCGGCCAGCTGGGCCTGCGCCGCGCCATGGACTACTGGGGC
CAGGGCACCAGCGTGACCGTGAGCAGCTGA

[SEQ ID No: 68]

In another embodiment, the amino acid sequence of PEDF-furin-P2A-sCD46 is referred to herein as SEQ ID No: 69, or a fragment or variant thereof, as follows:

MRRLVLLLCIGALLGHVFCQNPASPPEEGSPDPDSTGALVEEEDPFFKVPVNKLAAAVSNFGYDLYRVRSSTSPTTNV
LLSPLSVATALSALSLGAEQRTESIIHRALYYDLISSPDIHGTYKELLDTVTAPQKNLKSASRIVFEKKLRIKSSFVA
PLEKSYGTRPRVLTGNPRLDLQEINNWVQAQMKGKLARSTKEIPDEISILLLGVAHFKGQWVTKFDSRKTSLEDFYLD
EERTVRVPMMSDPKAVLRYGLDSDLSCKIAQLPLTGSMSIIFFLPLKVTQNLTLIEESLTSEFIHDIDRELKTVQAVL
TVPKLKLSYEGEVTKSLQEMKLQSLFDSPDFSKITGKPIKLTQVEHRAGFEWNEDGAGTTPSPGLQPAHLTFPLDYHL
NQPFIFVLRDTDTGALLFIGKILDPRGPRRSKRSGSGATNFSLLKQAGDVEENPGPMRRFLTVISFLLYFGCAFACEE
PPTFEAMELIGKPKPYYEIGERVDYKCKKGYFYIPPLATHTICDRNHTWLPVSDDACYRETCPYIRDPLNGQAVPANG
TYEFGYQMHFICNEGYYLIGEEILYCELKGSVAIWSGKPPICEKVLCTPPPKIKNGKHTFSEVEVFEYLDAVTYSCDP
APGPDPFSLIGESTIYCGDNSVWSRAAPECKVVKCRFPVVENGKQISGFGKKFYYKATVMFECDKGFYLDGSDTIVCD
SNSTWDPPVPKCLKVLPPSSTKPPALSHSVSTSSTTKSPASSASGPRPTYKPPVSNYPGYPKPEEGILDSLDV
[SEQ ID No: 69]

Preferably, in this embodiment, the construct comprises a 2328 nucleotide sequence (as contained within the plasmid IKC159P), which is referred to herein as SEQ ID No: 70, or a fragment or variant thereof, as follows:

ATGCGCCGCCTGGTGCTGCTGCTGTGCATCGGCGCCCTGCTGGGCCACGTGTTCTGCCAGAACCCCGCCAGCCCCCCCGAG
GAGGGCAGCCCCGACCCCGACAGCACCGGCGCCCTGGTGGAGGAGGAGGACCCCTTCTTCAAGGTGCCCGTGAACAAGCTG
GCCGCCGCCGTGAGCAACTTCGGCTACGACCTGTACCGCGTGCGCAGCAGCACCAGCCCCACCACCAACGTGCTGCTGAGC
CCCCTGAGCGTGGCCACCGCCCTGAGCGCCCTGAGCCTGGGCGCCGAGCAGCGCACCGAGAGCATCATCCACCGCGCCCTG
TACTACGACCTGATCAGCAGCCCCGACATCCACGGCACCTACAAGGAGCTGCTGGACACCGTGACCGCCCCCCAGAAGAAC
CTGAAGAGCGCCAGCCGCATCGTGTTCGAGAAGAAGCTGCGCATCAAGAGCAGCTTCGTGGCCCCCCTGGAGAAGAGCTAC
GGCACCCGCCCCCGCGTGCTGACCGGCAACCCCCGCCTGGACCTGCAGGAGATCAACAACTGGGTGCAGGCCCAGATGAAG
GGCAAGCTGGCCCGCAGCACCAAGGAGATCCCCGACGAGATCAGCATCCTGCTGCTGGGCGTGGCCCACTTCAAGGGCCAG
TGGGTGACCAAGTTCGACAGCCGCAAGACCAGCCTGGAGGACTTCTACCTGGACGAGGAGCGCACCGTGCGCGTGCCCATG
ATGAGCGACCCCAAGGCCGTGCTGCGCTACGGCCTGGACAGCGACCTGAGCTGCAAGATCGCCCAGCTGCCCCTGACCGGC
AGCATGAGCATCATCTTCTTCCTGCCCCTGAAGGTGACCCAGAACCTGACCCTGATCGAGGAGAGCCTGACCAGCGAGTTC
ATCCACGACATCGACCGCGAGCTGAAGACCGTGCAGGCCGTGCTGACCGTGCCCAAGCTGAAGCTGAGCTACGAGGGCGAG
GTGACCAAGAGCCTGCAGGAGATGAAGCTGCAGAGCCTGTTCGACAGCCCCGACTTCAGCAAGATCACCGGCAAGCCCATC
AAGCTGACCCAGGTGGAGCACCGCGCCGGCTTCGAGTGGAACGAGGACGGCGCCGGCACCACCCCCAGCCCCGGCCTGCAG
CCCGCCCACCTGACCTTCCCCCTGGACTACCACCTGAACCAGCCCTTCATCTTCGTGCTGCGCGACACCGACACCGGCGCC
CTGCTGTTCATCGGCAAGATCCTGGACCCCCGCGGCCCCCGCCGCAGCAAGCGCAGCGGCAGCGGCGCCACCAACTTCAGC
CTGCTGAAGCAGGCCGGCGACGTGGAGGAGAACCCCGGCCCCATGCGCCGCTTCCTGACCGTGATCAGCTTCCTGCTGTAC
TTCGGCTGCGCCTTCGCCTGCGAGGAGCCCCCCACCTTCGAGGCCATGGAGCTGATCGGCAAGCCCAAGCCCTACTACGAG
ATCGGCGAGCGCGTGGACTACAAGTGCAAGAAGGGCTACTTCTACATCCCCCCCCTGGCCACCCACACCATCTGCGACCGC
AACCACACCTGGCTGCCCGTGAGCGACGACGCCTGCTACCGCGAGACCTGCCCCTACATCCGCGACCCCCTGAACGGCCAG
GCCGTGCCCGCCAACGGCACCTACGAGTTCGGCTACCAGATGCACTTCATCTGCAACGAGGGCTACTACCTGATCGGCGAG
GAGATCCTGTACTGCGAGCTGAAGGGCAGCGTGGCCATCTGGAGCGGCAAGCCCCCCATCTGCGAGAAGGTGCTGTGCACC
CCCCCCCCCAAGATCAAGAACGGCAAGCACACCTTCAGCGAGGTGGAGGTGTTCGAGTACCTGGACGCCGTGACCTACAGC
TGCGACCCCGCCCCCGGCCCCGACCCCTTCAGCCTGATCGGCGAGAGCACCATCTACTGCGGCGACAACAGCGTGTGGAGC
CGCGCCGCCCCCGAGTGCAAGGTGGTGAAGTGCCGCTTCCCCGTGGTGGAGAACGGCAAGCAGATCAGCGGCTTCGGCAAG
AAGTTCTACTACAAGGCCACCGTGATGTTCGAGTGCGACAAGGGCTTCTACCTGGACGGCAGCGACACCATCGTGTGCGAC
AGCAACAGCACCTGGGACCCCCCCGTGCCCAAGTGCCTGAAGGTGCTGCCCCCCAGCAGCACCAAGCCCCCCGCCCTGAGC
CACAGCGTGAGCACCAGCAGCACCACCAAGAGCCCCGCCAGCAGCGCCAGCGGCCCCCGCCCCACCTACAAGCCCCCCGTG
AGCAACTACCCCGGCTACCCCAAGCCCGAGGAGGGCATCCTGGACAGCCTGGACGTGTAA
[SEQ ID No: 70]

In another embodiment, the amino acid sequence of sCD46-furin-P2A- PEDF, is referred to herein as SEQ ID No: 71 or a fragment or variant thereof, as follows:

MRRFLTVISFLLYFGCAFACEEPPTFEAMELIGKPKPYYEIGERVDYKCKKGYFYIPPLATHTICDRNHTWLPVSDDA
CYRETCPYIRDPLNGQAVPANGTYEFGYQMHFICNEGYYLIGEEILYCELKGSVAIWSGKPPICEKVLCTPPPKIKNG
KHTFSEVEVFEYLDAVTYSCDPAPGPDPFSLIGESTIYCGDNSVWSRAAPECKVVKCRFPVVENGKQISGFGKKFYYK
ATVMFECDKGFYLDGSDTIVCDSNSTWDPPVPKCLKVLPPSSTKPPALSHSVSTSSTTKSPASSASGPRPTYKPPVSN
YPGYPKPEEGILDSLDVRRSKRSGSGATNFSLLKQAGDVEENPGPMRRLVLLLCIGALLGHVFCQNPASPPEEGSPDP
DSTGALVEEEDPFFKVPVNKLAAAVSNFGYDLYRVRSSTSPTTNVLLSPLSVATALSALSLGAEQRTESIIHRALYYD
LISSPDIHGTYKELLDTVTAPQKNLKSASRIVFEKKLRIKSSFVAPLEKSYGTRPRVLTGNPRLDLQEINNWVQAQMK

GKLARSTKEIPDEISILLLGVAHFKGQWVTKFDSRKTSLEDFYLDEERTVRVPMMSDPKAVLRYGLDSDLSCKIAQLP
LTGSMSIIFFLPLKVTQNLTLIEESLTSEFIHDIDRELKTVQAVLTVPKLKLSYEGEVTKSLQEMKLQSLFDSPDFSK
ITGKPIKLTQVEHRAGFEWNEDGAGTTPSPGLQPAHLTFPLDYHLNQPFIFVLRDTDTGALLFIGKILDPRGP
[SEQ ID No: 71]

Preferably, in this embodiment, the construct comprises a 2328 nucleotide sequence (as contained within the plasmid IKC145P), which is referred to herein as SEQ ID No: 72, or a fragment or variant thereof, as follows:

ATGCGCCGCTTCCTGACCGTGATCAGCTTCCTGCTGTACTTCGGCTGCGCCTTCGCCTGCGAGGAGCCCCCCACCTTC
GAGGCCATGGAGCTGATCGGCAAGCCCAAGCCCTACTACGAGATCGGCGAGCGCGTGGACTACAAGTGCAAGAAGGGC
TACTTCTACATCCCCCCCCTGGCCACCCACACCATCTGCGACCGCAACCACACCTGGCTGCCCGTGAGCGACGACGCC
TGCTACCGCGAGACCTGCCCCTACATCCGCGACCCCCTGAACGGCCAGGCCGTGCCCGCCAACGGCACCTACGAGTTC
GGCTACCAGATGCACTTCATCTGCAACGAGGGCTACTACCTGATCGGCGAGGAGATCCTGTACTGCGAGCTGAAGGGC
AGCGTGGCCATCTGGAGCGGCAAGCCCCCCATCTGCGAGAAGGTGCTGTGCACCCCCCCCCCCAAGATCAAGAACGGC
AAGCACACCTTCAGCGAGGTGGAGGTGTTCGAGTACCTGGACGCCGTGACCTACAGCTGCGACCCCGCCCCCGGCCCC
GACCCCTTCAGCCTGATCGGCGAGAGCACCATCTACTGCGGCGACAACAGCGTGTGGAGCCGCGCCGCCCCCGAGTGC
AAGGTGGTGAAGTGCCGCTTCCCCGTGGTGGAGAACGGCAAGCAGATCAGCGGCTTCGGCAAGAAGTTCTACTACAAG
GCCACCGTGATGTTCGAGTGCGACAAGGGCTTCTACCTGGACGGCAGCGACACCATCGTGTGCGACAGCAACAGCACC
TGGGACCCCCCCGTGCCCAAGTGCCTGAAGGTGCTGCCCCCCAGCAGCACCAAGCCCCCCGCCCTGAGCCACAGCGTG
AGCACCAGCAGCACCACCAAGAGCCCCGCCAGCAGCGCCAGCGGCCCCCGCCCCACCTACAAGCCCCCCGTGAGCAAC
TACCCCGGCTACCCCAAGCCCGAGGAGGGCATCCTGGACAGCCTGGACGTGCGCCGCAGCAAGCGCAGCGGCAGCGGC
GCCACCAACTTCAGCCTGCTGAAGCAGGCCGGCGACGTGGAGGAGAACCCCGGCCCCATGCGCCGCCTGGTGCTGCTG
CTGTGCATCGGCGCCCTGCTGGGCCACGTGTTCTGCCAGAACCCCGCCAGCCCCCCCGAGGAGGGCAGCCCCGACCCC
GACAGCACCGGCGCCCTGGTGGAGGAGGAGGACCCCTTCTTCAAGGTGCCCGTGAACAAGCTGGCCGCCGCCGTGAGC
AACTTCGGCTACGACCTGTACCGCGTGCGCAGCAGCACCAGCCCCACCACCAACGTGCTGCTGAGCCCCCTGAGCGTG
GCCACCGCCCTGAGCGCCCTGAGCCTGGGCGCCGAGCAGCGCACCGAGAGCATCATCCACCGCGCCCTGTACTACGAC
CTGATCAGCAGCCCCGACATCCACGGCACCTACAAGGAGCTGCTGGACACCGTGACCGCCCCCCAGAAGAACCTGAAG
AGCGCCAGCCGCATCGTGTTCGAGAAGAAGCTGCGCATCAAGAGCAGCTTCGTGGCCCCCCTGGAGAAGAGCTACGGC
ACCCGCCCCCGCGTGCTGACCGGCAACCCCCGCCTGGACCTGCAGGAGATCAACAACTGGGTGCAGGCCCAGATGAAG
GGCAAGCTGGCCCGCAGCACCAAGGAGATCCCCGACGAGATCAGCATCCTGCTGCTGGGCGTGGCCCACTTCAAGGGC
CAGTGGGTGACCAAGTTCGACAGCCGCAAGACCAGCCTGGAGGACTTCTACCTGGACGAGGAGCGCACCGTGCGCGTG
CCCATGATGAGCGACCCCAAGGCCGTGCTGCGCTACGGCCTGGACAGCGACCTGAGCTGCAAGATCGCCCAGCTGCCC
CTGACCGGCAGCATGAGCATCATCTTCTTCCTGCCCCTGAAGGTGACCCAGAACCTGACCCTGATCGAGGAGAGCCTG
ACCAGCGAGTTCATCCACGACATCGACCGCGAGCTGAAGACCGTGCAGGCCGTGCTGACCGTGCCCAAGCTGAAGCTG
AGCTACGAGGGCGAGGTGACCAAGAGCCTGCAGGAGATGAAGCTGCAGAGCCTGTTCGACAGCCCCGACTTCAGCAAG
ATCACCGGCAAGCCCATCAAGCTGACCCAGGTGGAGCACCGCGCCGGCTTCGAGTGGAACGAGGACGGCGCCGGCACC
ACCCCCAGCCCCGGCCTGCAGCCCGCCCACCTGACCTTCCCCCTGGACTACCACCTGAACCAGCCCTTCATCTTCGTG
CTGCGCGACACCGACACCGGCGCCCTGCTGTTCATCGGCAAGATCCTGGACCCCCGCGGCCCCTAA
[SEQ ID No: 72]

In another embodiment, the amino acid sequence of [PEDF-furin-P2A-CFHL1], is referred to herein as SEQ ID No: 87, or a fragment or variant thereof, as follows:

MRRLVLLLCIGALLGHVFCQNPASPPEEGSPDPDSTGALVEEEDPFFKVPVNKLAAAVSNFGYDLYRVRSSTSPTTNV
LLSPLSVATALSALSLGAEQRTESIIHRALYYDLISSPDIHGTYKELLDTVTAPQKNLKSASRIVFEKKLRIKSSFVA
PLEKSYGTRPRVLTGNPRLDLQEINNWVQAQMKGKLARSTKEIPDEISILLLGVAHFKGQWVTKFDSRKTSLEDFYLD
EERTVRVPMMSDPKAVLRYGLDSDLSCKIAQLPLTGSMSIIFFLPLKVTQNLTLIEESLTSEFIHDIDRELKTVQAVL
TVPKLKLSYEGEVTKSLQEMKLQSLFDSPDFSKITGKPIKLTQVEHRAGFEWNEDGAGTTPSPGLQPAHLTFPLDYHL
NQPFIFVLRDTDTGALLFIGKILDPRGPRRSKRSGSGATNFSLLKQAGDVEENPGPMRRLLTFISILALVGAFAEDCN
ELPPRRNTEILTGSWSDQTYPEGTQAIYKCRPGYRSLGNVIMVCRKGEWVALNPLRKCQKRPCGHPGDTPFGTFTLTG
GNVFEYGVKAVYTCNEGYQLLGEINYRECDTDGWTNDIPICEVVKCLPVTAPENGKIVSSAMEPDREYHFGQAVRFVC
NSGYKIEGDEEMHCSDDGFWSKEKPKCVEISCKSPDVINGSPISQKIIYKENERFQYKCNMGYEYSERGDAVCTESGW
RPLPSCEEKSCDNPYIPNGDYSPLRIKHRTGDEITYQCRNGFYPATRGNTAKCTSTGWIPAPRCTLKPCDYPDIKHGG
LYHENMRRPYFPVAVGKYYSYYCDEHFETPSGSYWDHIHCTQDGWSPAVPCLRKCYFPYLENGYNQNYGRKFVQGKSI
DVACHPGYALPKAQTTVTCMENGWSPTPRCIR
[SEQ ID No: 87]

Preferably, in this embodiment, the construct comprises a 2670 nucleotide sequence (as contained within the plasmid IKC161P), which is referred to herein as SEQ ID No: 88, or a fragment or variant thereof, as follows:

ATGCGCCGCCTGGTGCTGCTGCTGTGCATCGGCGCCCTGCTGGGCCACGTGTTCTGCCAGAACCCCGCCAGCCCCCCC
GAGGAGGGCAGCCCCGACCCCGACAGCACCGGCGCCCTGGTGGAGGAGGAGGACCCCTTCTTCAAGGTGCCCGTGAAC
AAGCTGGCCGCCGCCGTGAGCAACTTCGGCTACGACCTGTACCGCGTGCGCAGCAGCACCAGCCCCACCACCAACGTG
CTGCTGAGCCCCCTGAGCGTGGCCACCGCCCTGAGCGCCCTGAGCCTGGGCGCCGAGCAGCGCACCGAGAGCATCATC
CACCGCGCCCTGTACTACGACCTGATCAGCAGCCCCGACATCCACGGCACCTACAAGGAGCTGCTGGACACCGTGACC
GCCCCCCAGAAGAACCTGAAGAGCGCCAGCCGCATCGTGTTCGAGAAGAAGCTGCGCATCAAGAGCAGCTTCGTGGCC
CCCCTGGAGAAGAGCTACGGCACCCGCCCCCGCGTGCTGACCGGCAACCCCCGCCTGGACCTGCAGGAGATCAACAAC
TGGGTGCAGGCCCAGATGAAGGGCAAGCTGGCCCGCAGCACCAAGGAGATCCCCGACGAGATCAGCATCCTGCTGCTG
GGCGTGGCCCACTTCAAGGGCCAGTGGGTGACCAAGTTCGACAGCCGCAAGACCAGCCTGGAGGACTTCTACCTGGAC
GAGGAGCGCACCGTGCGCGTGCCCATGATGAGCGACCCCAAGGCCGTGCTGCGCTACGGCCTGGACAGCGACCTGAGC
TGCAAGATCGCCCAGCTGCCCCTGACCGGCAGCATGAGCATCATCTTCTTCCTGCCCCTGAAGGTGACCCAGAACCTG
ACCCTGATCGAGGAGAGCCTGACCAGCGAGTTCATCCACGACATCGACCGCGAGCTGAAGACCGTGCAGGCCGTGCTG
ACCGTGCCCAAGCTGAAGCTGAGCTACGAGGGCGAGGTGACCAAGAGCCTGCAGGAGATGAAGCTGCAGAGCCTGTTC
GACAGCCCCGACTTCAGCAAGATCACCGGCAAGCCCATCAAGCTGACCCAGGTGGAGCACCGCGCCGGCTTCGAGTGG
AACGAGGACGGCGCCGGCACCACCCCCAGCCCCGGCCTGCAGCCCGCCCACCTGACCTTCCCCCTGGACTACCACCTG
AACCAGCCCTTCATCTTCGTGCTGCGCGACACCGACACCGGCGCCCTGCTGTTCATCGGCAAGATCCTGGACCCCCGC
GGCCCCCGCCGCAGCAAGCGCAGCGGCAGCGGCGCCACCAACTTCAGCCTGCTGAAGCAGGCCGGCGACGTGGAGGAG
AACCCCGGCCCCATGCGCCGCCTGCTGACCTTCATCAGCATCCTGGCCCTGGTGGGCGCCTTCGCCGAGGACTGCAAC
GAGCTGCCCCCCCGCCGCAACACCGAGATCCTGACCGGCAGCTGGAGCGACCAGACCTACCCCGAGGGCACCCAGGCC
ATCTACAAGTGCCGCCCCGGCTACCGCAGCCTGGGCAACGTGATCATGGTGTGCCGCAAGGGCGAGTGGGTGGCCCTG
AACCCCCTGCGCAAGTGCCAGAAGCGCCCCTGCGGCCACCCCGGCGACACCCCCTTCGGCACCTTCACCCTGACCGGC

GGCAACGTGTTCGAGTACGGCGTGAAGGCCGTGTACACCTGCAACGAGGGCTACCAGCTGCTGGGCGAGATCAACTAC
CGCGAGTGCGACACCGACGGCTGGACCAACGACATCCCCATCTGCGAGGTGGTGAAGTGCCTGCCCGTGACCGCCCCC
GAGAACGGCAAGATCGTGAGCAGCGCCATGGAGCCCGACCGCGAGTACCACTTCGGCCAGGCCGTGCGCTTCGTGTGC
AACAGCGGCTACAAGATCGAGGGCGACGAGGAGATGCACTGCAGCGACGACGGCTTCTGGAGCAAGGAGAAGCCCAAG
TGCGTGGAGATCAGCTGCAAGAGCCCCGACGTGATCAACGGCAGCCCCATCAGCCAGAAGATCATCTACAAGGAGAAC
GAGCGCTTCCAGTACAAGTGCAACATGGGCTACGAGTACAGCGAGCGCGGCGACGCCGTGTGCACCGAGAGCGGCTGG
CGCCCCCTGCCCAGCTGCGAGGAGAAGAGCTGCGACAACCCCTACATCCCCAACGGCGACTACAGCCCCCTGCGCATC
AAGCACCGCACCGGCGACGAGATCACCTACCAGTGCCGCAACGGCTTCTACCCCGCCACCCGGGGCAACACCGCCAAG
TGCACCAGCACCGGCTGGATCCCCGCCCCCCGCTGCACCCTGAAGCCCTGCGACTACCCCGACATCAAGCACGGCGGC
CTGTACCACGAGAACATGCGCCGCCCCTACTTCCCCGTGGCCGTGGGCAAGTACTACAGCTACTACTGCGACGAGCAC
TTCGAGACCCCCAGCGGCAGCTACTGGGACCACATCCACTGCACCCAGGACGGCTGGAGCCCCGCCGTGCCCTGCCTG
CGCAAGTGCTACTTCCCCTACCTGGAGAACGGCTACAACCAGAACTACGGCCGCAAGTTCGTGCAGGGCAAGAGCATC
GACGTGGCCTGCCACCCCGGCTACGCCCTGCCCAAGGCCCAGACCACCGTGACCTGCATGGAGAACGGCTGGAGCCCC
ACCCCCCGCTGCATCCGC
[SEQ ID No: 88]

In another embodiment, the amino acid sequence of [CFHL1-furin-P2A-PEDF], is referred to herein as SEQ ID No: 89, or a fragment or variant thereof, as follows:

MRRLLTFISILALVGAFAEDCNELPPRRNTEILTGSWSDQTYPEGTQAIYKCRPGYRSLGNVIMVCRKGEWVALNPLR
KCQKRPCGHPGDTPFGTFTLTGGNVFEYGVKAVYTCNEGYQLLGEINYRECDTDGWTNDIPICEVVKCLPVTAPENGK
IVSSAMEPDREYHFGQAVRFVCNSGYKIEGDEEMHCSDDGFWSKEKPKCVEISCKSPDVINGSPISQKIIYKENERFQ
YKCNMGYEYSERGDAVCTESGWRPLPSCEEKSCDNPYIPNGDYSPLRIKHRTGDEITYQCRNGFYPATRGNTAKCTST
GWIPAPRCTLKPCDYPDIKHGGLYHENMRRPYFPVAVGKYYSYYCDEHFETPSGSYWDHIHCTQDGWSPAVPCLRKCY
FPYLENGYNQNYGRKFVQGKSIDVACHPGYALPKAQTTVTCMENGWSPTPRCIRRRSKRSGSGATNFSLLKQAGDVEE
NPGPMRRLVLLLCIGALLGHVFCQNPASPPEEGSPDPDSTGALVEEEDPFFKVPVNKLAAAVSNFGYDLYRVRSSTSP
TTNVLLSPLSVATALSALSLGAEQRTESIIHRALYYDLISSPDIHGTYKELLDTVTAPQKNLKSASRIVFEKKLRIKS
SFVAPLEKSYGTRPRVLTGNPRLDLQEINNWVQAQMKGKLARSTKEIPDEISILLLGVAHFKGQWVTKFDSRKTSLED
FYLDEERTVRVPMMSDPKAVLRYGLDSDLSCKIAQLPLTGSMSIIFFLPLKVTQNLTLIEESLTSEFIHDIDRELKTV
QAVLTVPKLKLSYEGEVTKSLQEMKLQSLFDSPDFSKITGKPIKLTQVEHRAGFEWNEDGAGTTPSPGLQPAHLTFPL
DYHLNQPFIFVLRDTDTGALLFIGKILDPRGP
[SEQ ID No: 89]

Preferably, in this embodiment, the construct comprises a 2670 nucleotide sequence (as contained within the plasmid IKC174P), which is referred to herein as SEQ ID No: 90, or a fragment or variant thereof, as follows:

ATGCGCCGCCTGCTGACCTTCATCAGCATCCTGGCCCTGGTGGGCGCCTTCGCCGAGGACTGCAACGAGCTGCCCCCC
CGCCGCAACACCGAGATCCTGACCGGCAGCTGGAGCGACCAGACCTACCCCGAGGGCACCCAGGCCATCTACAAGTGC
CGCCCCGGCTACCGCAGCCTGGGCAACGTGATCATGGTGTGCCGCAAGGGCGAGTGGGTGGCCCTGAACCCCCTGCGC
AAGTGCCAGAAGCGCCCCTGCGGCCACCCCGGCGACACCCCCTTCGGCACCTTCACCCTGACCGGCGGCAACGTGTTC

GAGTACGGCGTGAAGGCCGTGTACACCTGCAACGAGGGCTACCAGCTGCTGGGCGAGATCAACTACCGCGAGTGCGAC
ACCGACGGCTGGACCAACGACATCCCCATCTGCGAGGTGGTGAAGTGCCTGCCCGTGACCGCCCCCGAGAACGGCAAG
ATCGTGAGCAGCGCCATGGAGCCCGACCGCGAGTACCACTTCGGCCAGGCCGTGCGCTTCGTGTGCAACAGCGGCTAC
AAGATCGAGGGCGACGAGGAGATGCACTGCAGCGACGACGGCTTCTGGAGCAAGGAGAAGCCCAAGTGCGTGGAGATC
AGCTGCAAGAGCCCCGACGTGATCAACGGCAGCCCCATCAGCCAGAAGATCATCTACAAGGAGAACGAGCGCTTCCAG
TACAAGTGCAACATGGGCTACGAGTACAGCGAGCGCGGCGACGCCGTGTGCACCGAGAGCGGCTGGCGCCCCCTGCCC
AGCTGCGAGGAGAAGAGCTGCGACAACCCCTACATCCCCAACGGCGACTACAGCCCCCTGCGCATCAAGCACCGCACC
GGCGACGAGATCACCTACCAGTGCCGCAACGGCTTCTACCCCGCCACCCGGGGCAACACCGCCAAGTGCACCAGCACC
GGCTGGATCCCCGCCCCCCGCTGCACCCTGAAGCCCTGCGACTACCCCGACATCAAGCACGGCGGCCTGTACCACGAG
AACATGCGCCGCCCCTACTTCCCCGTGGCCGTGGGCAAGTACTACAGCTACTACTGCGACGAGCACTTCGAGACCCCC
AGCGGCAGCTACTGGGACCACATCCACTGCACCCAGGACGGCTGGAGCCCCGCCGTGCCCTGCCTGCGCAAGTGCTAC
TTCCCCTACCTGGAGAACGGCTACAACCAGAACTACGGCCGCAAGTTCGTGCAGGGCAAGAGCATCGACGTGGCCTGC
CACCCCGGCTACGCCCTGCCCAAGGCCCAGACCACCGTGACCTGCATGGAGAACGGCTGGAGCCCCACCCCCCGCTGC
ATCCGCCGCCGCAGCAAGCGCAGCGGCAGCGGCGCCACCAACTTCAGCCTGCTGAAGCAGGCCGGCGACGTGGAGGAG
AACCCCGGCCCCATGCGCCGCCTGGTGCTGCTGCTGTGCATCGGCGCCCTGCTGGGCCACGTGTTCTGCCAGAACCCC
GCCAGCCCCCCCGAGGAGGGCAGCCCCGACCCCGACAGCACCGGCGCCCTGGTGGAGGAGGAGGACCCCTTCTTCAAG
GTGCCCGTGAACAAGCTGGCCGCCGCCGTGAGCAACTTCGGCTACGACCTGTACCGCGTGCGCAGCAGCACCAGCCCC
ACCACCAACGTGCTGCTGAGCCCCCTGAGCGTGGCCACCGCCCTGAGCGCCCTGAGCCTGGGCGCCGAGCAGCGCACC
GAGAGCATCATCCACCGCGCCCTGTACTACGACCTGATCAGCAGCCCCGACATCCACGGCACCTACAAGGAGCTGCTG
GACACCGTGACCGCCCCCCAGAAGAACCTGAAGAGCGCCAGCCGCATCGTGTTCGAGAAGAAGCTGCGCATCAAGAGC
AGCTTCGTGGCCCCCCTGGAGAAGAGCTACGGCACCCGCCCCCGCGTGCTGACCGGCAACCCCCGCCTGGACCTGCAG
GAGATCAACAACTGGGTGCAGGCCCAGATGAAGGGCAAGCTGGCCCGCAGCACCAAGGAGATCCCCGACGAGATCAGC
ATCCTGCTGCTGGGCGTGGCCCACTTCAAGGGCCAGTGGGTGACCAAGTTCGACAGCCGCAAGACCAGCCTGGAGGAC
TTCTACCTGGACGAGGAGCGCACCGTGCGCGTGCCCATGATGAGCGACCCCAAGGCCGTGCTGCGCTACGGCCTGGAC
AGCGACCTGAGCTGCAAGATCGCCCAGCTGCCCCTGACCGGCAGCATGAGCATCATCTTCTTCCTGCCCCTGAAGGTG
ACCCAGAACCTGACCCTGATCGAGGAGAGCCTGACCAGCGAGTTCATCCACGACATCGACCGCGAGCTGAAGACCGTG
CAGGCCGTGCTGACCGTGCCCAAGCTGAAGCTGAGCTACGAGGGCGAGGTGACCAAGAGCCTGCAGGAGATGAAGCTG
CAGAGCCTGTTCGACAGCCCCGACTTCAGCAAGATCACCGGCAAGCCCATCAAGCTGACCCAGGTGGAGCACCGCGCC
GGCTTCGAGTGGAACGAGGACGGCGCCGGCACCACCCCCAGCCCCGGCCTGCAGCCCGCCCACCTGACCTTCCCCCTG
GACTACCACCTGAACCAGCCCTTCATCTTCGTGCTGCGCGACACCGACACCGGCGCCCTGCTGTTCATCGGCAAGATC
CTGGACCCCCGCGGCCCC

[SEQ ID No: 90]

Hence, in a preferred embodiment, the construct encodes an amino acid sequence substantially as set out in SEQ ID No: 55, 57, 59, 61, 63, 65, 67, 69, 71, 85, 87 or 89, or a fragment or variant thereof.

Preferably, the construct comprises a nucleotide sequence substantially as set out in SEQ ID No: 56, 58, 60, 62, 64, 66, 68, 70, 72, 86, 88 or 90, or a fragment or variant thereof.

The inventors have created a series of recombinant expression vectors comprising the construct of the invention.

Thus, according to a second aspect, there is provided a recombinant vector comprising the genetic construct according to the first aspect.

In one embodiment, the recombinant vector (e.g. known as "IKC0121V") comprises a nucleotide sequence which is referred to herein as SEQ ID No: 73, or a fragment or variant thereof, as follows:

CGCGCTCGCTCGCTCACTGAGGCCGCCCGGGCAAAGCCCGGGCGTCGGGCGACCTTTGGTCGCCCGGCCTCAGTGAGC
GAGCGAGCGCGCAGAGAGGGAGTGGCCAACTCCATCACTAGGGGTTCCTTGTAGTTAATGATTAACCCGCCATGCTAC
TTATCTACGTAGCCATGCTCTAGACATGGCTCGACAGATCGAGCTCCACCGGGTACCCTAGTTATTAATAGTAATCAA
TTACGGGGTCATTAGTTCATAGCCCATATATGGAGTTCCGCGTTACATAACTTACGGTAAATGGCCCGCCTGGCTGAC
CGCCCAACGACCCCCGCCCATTGACGTCAATAATGACGTATGTTCCCATAGTAACGCCAATAGGGACTTTCCATTGAC
GTCAATGGGTGGACTATTTACGGTAAACTGCCCACTTGGCAGTACATCAAGTGTATCATATGCCAAGTACGCCCCCTA
TTGACGTCAATGACGGTAAATGGCCCGCCTGGCATTATGCCCAGTACATGACCTTATGGGACTTTCCTACTTGGCAGT
ACATCTACGTATTAGTCATCGCTATTACCATGGTCGAGGTGAGCCCCACGTTCTGCTTCACTCTCCCCATCTCCCCCC
CCTCCCCACCCCCAATTTTGTATTTATTTATTTTTTAATTATTTTGTGCAGCGATGGGGGCGGGGGGGGGGGGGGGGC
GCGCGCCAGGCGGGGCGGGGCGGGGCGAGGGGCGGGGCGGGGCGAGGCGGAGAGGTGCGGCGGCAGCCAATCAGAGCG
GCGCGCTCCGAAAGTTTCCTTTTATGGCGAGGCGGCGGCGGCGGCGGCCCTATAAAAAGCGAAGCGCGCGGCGGGCGG
GAGTCGCTGCGACGCTGCCTTCGCCCCGTGCCCCGCTCCGCCGCCGCCTCGCGCCGCCCGCCCCGGCTCTGACTGACC
GCGTTACTCCCACAGGTGAGCGGGCGGGACGGCCCTTCTCCTCCGGGCTGTAATTAGCGCTTGGTTTAATGACGGCTT
GTTTCTTTTCTGTGGCTGCGTGAAAGCCTTGAGGGGCTCCGGGAGGCTAGAGCCTCTGCTAACCATGTTCATGCCTTC
TTCTTTTTCCTACAGCTCCTGGGCAACGTGCTGGTTATTGTGCTGTCTCATCATTTTGGCAAAGAATTCGCGACTCGA
CCCGCTAGCGCCACCATGCGCCGCCTGGTGCTGCTGCTGTGCATCGGCGCCCTGCTGGGCCACGTGTTCTGCCAGAAC
CCCGCCAGCCCCCCCGAGGAGGGCAGCCCCGACCCCGACAGCACCGGCGCCCTGGTGGAGGAGGAGGACCCCTTCTTC
AAGGTGCCCGTGAACAAGCTGGCCGCCGCCGTGAGCAACTTCGGCTACGACCTGTACCGCGTGCGCAGCAGCACCAGC
CCCACCACCAACGTGCTGCTGAGCCCCCTGAGCGTGGCCACCGCCCTGAGCGCCCTGAGCCTGGGCGCCGAGCAGCGC
ACCGAGAGCATCATCCACCGCGCCCTGTACTACGACCTGATCAGCAGCCCCGACATCCACGGCACCTACAAGGAGCTG
CTGGACACCGTGACCGCCCCCCAGAAGAACCTGAAGAGCGCCAGCCGCATCGTGTTCGAGAAGAAGCTGCGCATCAAG
AGCAGCTTCGTGGCCCCCCTGGAGAAGAGCTACGGCACCCGCCCCCGCGTGCTGACCGGCAACCCCCGCCTGGACCTG
CAGGAGATCAACAACTGGGTGCAGGCCCAGATGAAGGGCAAGCTGGCCCGCAGCACCAAGGAGATCCCCGACGAGATC
AGCATCCTGCTGCTGGGCGTGGCCCACTTCAAGGGCCAGTGGGTGACCAAGTTCGACAGCCGCAAGACCAGCCTGGAG
GACTTCTACCTGGACGAGGAGCGCACCGTGCGCGTGCCCATGATGAGCGACCCCAAGGCCGTGCTGCGCTACGGCCTG
GACAGCGACCTGAGCTGCAAGATCGCCCAGCTGCCCCTGACCGGCAGCATGAGCATCATCTTCTTCCTGCCCCTGAAG
GTGACCCAGAACCTGACCCTGATCGAGGAGAGCCTGACCAGCGAGTTCATCCACGACATCGACCGCGAGCTGAAGACC
GTGCAGGCCGTGCTGACCGTGCCCAAGCTGAAGCTGAGCTACGAGGGCGAGGTGACCAAGAGCCTGCAGGAGATGAAG
CTGCAGAGCCTGTTCGACAGCCCCGACTTCAGCAAGATCACCGGCAAGCCCATCAAGCTGACCCAGGTGGAGCACCGC
GCCGGCTTCGAGTGGAACGAGGACGGCGCCGGCACCACCCCCAGCCCCGGCCTGCAGCCCGCCCACCTGACCTTCCCC

CTGGACTACCACCTGAACCAGCCCTTCATCTTCGTGCTGCGCGACACCGACACCGGCGCCCTGCTGTTCATCGGCAAG
ATCCTGGACCCCCGCGGCCCCCGCCGCAGCAAGCGCAGCGGCAGCGGCGCCACCAACTTCAGCCTGCTGAAGCAGGCC
GGCGACGTGGAGGAGAACCCCGGCCCCATGCGCCGCCTGCTGACCTTCATCAGCATCCTGGCCCTGGTGGGCGCCTTC
GCCGACATCCAGATGACCCAGAGCCCCAGCAGCCTGAGCGCCAGCGTGGGCGACCGCGTGACCATCACCTGCCGCGCC
AGCCAGGACGTAAGCACCGCCGTGGCCTGGTACCAGCAGAAGCCCGGCAAGGCCCCCAAGCTGCTGATCTACAGCGCC
AGCTTCCTGTACAGCGGCGTGCCCAGCCGCTTCAGCGGCAGCGGCAGCGGCACCGACTTCACCCTGACCATCAGCAGC
CTGCAGCCCGAGGACTTCGCCACCTACTACTGCCAGCAGAGCTACGCCACCCTGCCCACCTTCGAGCAGGGCACCAAG
GTGGAGATCAAGCGCGGCGGCGGCGGCAGCGGCGGCGGCGGCAGCGGCGGCGGCGGCAGCGGCGGCGGCGGCAGC
GAGGTGCAGCTGGTGGAGAGCGGCGGCGGCCTGGTGCAGCCCGGCGGCAGCCTGCGCCTGAGCTGCGCCGCCAGCGGC
TTCAGCTTCACCAGCAGCAGCGTGAGCCCCGGCAAGGGCCTGGAGTGGGTGGGCCTGATCTACCCCTACAACGGCTTC
AACTACTACGCCGACAGCGTGAAGGGCCGCTTCACCATCAGCGCCGACACCAGCCTGCAGATGAACAGCCTGCGCGCC
GAGGACACCGCCGTGTACTACTGCGCCCGCAACGCCCTGTACGGCAGCGGCGGCTACTACGCCATGGACTACTGGGGC
CAGGGCACCCTGGTGACCGTGAGCAGCTGATATACTACTAGTACGCGGCCGCACCGGTGTACAATCAACCTCTGGATT
ACAAAATTTGTGAAAGATTGACTGGTATTCTTAACTATGTTGCTCCTTTTACGCTATGTGGATACGCTGCTTTAATGC
CTTTGTATCATGCTATTGCTTCCCGTATGGCTTTCATTTTCTCCTCCTTGTATAAATCCTGGTTAGTTCTTGCCACGG
CGGAACTCATCGCCGCCTGCCTTGCCCGCTGCTGGACAGGGGCTCGGCTGTTGGGCACTGACAATTCCGTGGTGTGAA
TTCGAGCTAGGTACAGCTTATCGATACCGTCGACAGCAGACATGATAAGATACATTGATGAGTTTGGACAAACCACAA
CTAGAATGCAGTGAAAAAAATGCTTTATTTGTGAAATTTGTGATGCTATTGCTTTATTTGTAACCATTATAAGCTGCA
ATAAACAAGTTAACAACAACAATTGCATTCATTTTATGTTTCAGGTTCAGGGGGAGGTGTGGGAGGTTTTTTAAAGCA
AGTAAAACCTCTACAAATGTGGTATGCTCGAGGGCATGCAACAACAACAATTGCATTCATTTTATGTTTCAGGTTCAG
GGGGAGATGTGGGAGGTTTTTTAAAGCAAGTAAAACCTCTACAAATGTGGTAAAATCCGATAAGGACTAGAGCATGGC
TACGTAGATAAGTAGCATGGCGGGTTAATCATTAACTACAAGGAACCCCTAGTGATGGAGTTGGCCACTCCCTCTCTG
CGCGCTCGCTCGCTCACTGAGGCCGGGCGACCAAAGGTCGCCCGACGCCCGGGCTTTGCCCGGGCGGCCTCAGTGAGC
GAGCGAGCGCGC

[SEQ ID No: 73]

Accordingly, in one embodiment, the recombinant vector comprises a nucleotide sequence substantially as set out in SEQ ID No: 73, or a fragment or variant thereof.

The recombinant vector may be a recombinant AAV (rAAV) vector. The rAAV may be a naturally occurring vector or a vector with a hybrid AAV serotype. The rAAV may be AAV-1, AAV-2, AAV-2.7m8, AAV-3A, AAV-3B, AAV-4, AAV-5, AAV-6, AAV-7, AAV-8, AAV-9, AAV-10, and AAV-11. Preferably, the rAAV is rAAV serotype-2.

Advantageously, recombinant AAV2 evokes a minimal immune response in host organisms and mediates long-term transgene expression that can persist in the retina for at least one year after vector administration.

The term "recombinant AAV (rAAV) vector" can mean a recombinant AAV-derived nucleic acid. It can contain at least one terminal repeat sequence.

The capsid coat of the AAV and recombinant vectors are known to be composed of three capsid proteins called VP1, VP2 and VP3, which all comprise a significant amount of overlapping amino acids between them, but unique N-terminal sequences. The AAV virus contains 60 subunits with a 1:1:10 ratio of each of the VP1, VP2 and VP3 capsid proteins [62], which together form an icosahedral structure. Many AAV serotypes have been identified which differ in the amino acid compositions and thus confer different binding properties to receptors on host cells. There are several naturally occurring AAV serotypes identified, such as AAV1, AAV2, AAV2.7m8, AAV3, AAV4, AAV5, AAV6, AA7, AAV8, AAV9, AAV10, AAV11 and AAV12 and many artificial variants in which further modifications to the amino acid sequence have been identified through screening DNA variants from libraries of capsid coding sequences [63]. Different serotypes can therefore display tropism and exchanging various amino acids of one pseudotype can change the tropism or infectivity for different target cells. Thus, specific AAV pseudotypes can be designed to target one particular cell type or greatly restrict infectivity to a particular organ. In some embodiments, an rAAV vector is a vector derived from an AAV serotype, including AAV1, AAV2, AAV2.7m8, AAV3, AAV4, AAV5, AAV6, AA7, AAV8, AAV9, AAV10, AAV11, or AAV12. An rAAV particle may comprise a capsid protein derived from any AAV serotype, including AAV1, AAV2, AAV2.7m8, AAV3, AAV4, AAV5, AAV6, AA7, AAV8, AAV9, AAV10, AAVrh10, AAV11, or AAV12 capsid. An rAAV particle can comprise viral proteins and viral nucleic acids of the same serotype or a mixed serotype.

A capsid protein or a recombinant viral particle of the invention may comprise or consist of an amino acid sequence of a naturally occurring protein (for example, a naturally occurring AAV capsid protein, such as capsid protein of AAV serotype AAV1, AAV2, AAV2.7m8, AAV3, AAV4, AAV5, AAV6, AA7, AAV8, AAV9, AAV10, AAVrh10, AAV11, or AAV12), or may be a derivative or chimera of a naturally occurring capsid protein that includes one or more amino acid substitutions, deletions, or additions, compared to the amino acid sequence of the naturally occurring capsid protein, for example to confer tropism for a desired tissue type or cell type (such as retinal ganglion cell, photoreceptor or retinal pigment epithelial cell), or to reduce immunogenicity of the recombinant virus particle.

In some embodiments a capsid protein of a recombinant virus particle of the invention has an amino acid sequence that has at least 60%, 70%, 80%, 90%, 95%, 96%, 97%, 98% or 99% amino acid identity along its entire length to the amino acid sequence of a naturally occurring capsid protein, for example a naturally occurring AAV capsid protein of serotype AAV1, AAV2, AAV2.7m8, AAV3, AAV4, AAV5, AAV6, AA7, AAV8, AAV9, AAV10, AAVrhio, AAV11, or AAV12.

The constructs and expression vectors described herein can be used to treat retinal disorders, particularly dry age-related macular degeneration and geographic atrophy, and more generally reduce complement activation and retinal cell damage and loss.

Hence, according to a third aspect, there is provided the genetic construct according to the first aspect, or the recombinant vector according to the second aspect, for use as a medicament or in therapy.

According to a fourth aspect, there is provided the genetic construct according to the first aspect, or the recombinant vector according to the second aspect, for use in treating, preventing or ameliorating a retinal disorder, or for reducing complement activation and retinal cell damage and loss.

According to a fifth aspect, there is provided a method of treating, preventing or ameliorating a retinal disorder in a subject, or for reducing complement activation and retinal cell damage and loss, the method comprising administering, or having administered, to a subject in need of such treatment, a therapeutically effective amount of the genetic construct according to the first aspect, or the recombinant vector according to the second aspect.

Preferably, the genetic construct or the recombinant vector according to invention are used in a gene therapy technique. The PEDF receptor agonist encoded by the construct or vector, activates the PEDF receptor to thereby preserve RPE and photoreceptor cells, reduce VEGF release, and prevent conversion from dry-AMD to wet-AMD. The anti-complement protein encoded by the construct or vector, neutralises complement factors to thereby reduce complement activation and reduce GA area and retinal cell loss.

In one embodiment, the retinal disorder that is treated may be dry age-related macular degeneration or geographic atrophy. In addition, the retinal disorder that is treated may be any pathophysiological condition which involves retinal damage through complement activation.

In another embodiment, the constructs and vectors may be used to reduce complement activation and retinal cell damage and loss. The constructs and vectors may be used to treat retinal cell damage and loss associated with the following conditions; retinitis pigmentosa, Stargardt disease, diabetic macular degeneration, age-related macular degeneration, and Leber's congenital amaurosis. In another embodiment, the constructs and vectors may be used to treat retinal cell damage and loss associated with glaucoma.

It will be appreciated that the genetic construct according to the first aspect, or the recombinant vector according to the second aspect may be used in a medicament, which may be used as a monotherapy (i.e. use of the genetic construct according to the first aspect or the vector according to the second aspect of the invention), for treating, ameliorating, or preventing a retinal disorder, or for reducing complement activation and retinal cell damage and loss. Alternatively, the genetic construct or the recombinant vector according to the invention may be used as an adjunct to, or in combination with, known therapies for treating, ameliorating, or preventing a retinal disorder, or for reducing complement activation and retinal cell damage and loss.

An effective amount of recombinant viral vector is administered, depending on the objective of treatment. For example, where a low percentage of transduction can achieve the desired therapeutic effect, then the objective of treatment is generally to meet or exceed this level of transduction. In some instances, this level of transduction can be achieved by transduction of only about 1 to 5% of the target cells, in some embodiments at least about 20% of the desired tissue type, in some embodiments at least about 50%, in some embodiments at least about 80%, in some embodiments at least about 95%, in some embodiments at least about 99% of the cells of the desired tissue type. In some embodiments of the invention, the dose of viral particles administered to the subject is between 1 x 10⁸ to 1 x 10¹⁴ genome copies.

The recombinant virus particles may be administered by one or more injections, either during the same procedure or spaced apart by days, weeks, months or years. In some embodiments, multiple vectors may be used to treat the subject. In some embodiments, at least 2%, 5%, 10%, 15%, 20%, 25%, 30%, 35%, 40%, 45%, 50%, 55%, 60%, 65%, 70%, or 75% up to 100% of cells of the target tissue (for example retinal cells of the eye) are transduced. Methods to identify cells transduced by recombinant viral particles comprising a recombinant virus particle capsid are known in the art. For example, immunohistochemistry or the use of a market such as enhanced green fluorescent protein can be used to detect transduction of recombinant virus particles.

In some embodiments, the recombinant vectors are administered (for example by injection or infusion) to one or more locations in the desired tissue (for example the eye). In some embodiments, the recombinant vectors are administered (for example by injection or infusion) to any one of one, two, three, four, five, six, seven, eight, nine or ten, or more than ten locations in the tissue. In some embodiments, the recombinant vectors are administered to more than one location simultaneously or sequentially. In some embodiments, multiple injections of recombinant vectors are no more than one hour, two hours, three hours, four hours, five hours, six hours, nine hours, twelve hours or 24 hours apart.

The genetic construct or the recombinant vector according to the invention may be combined in compositions having a number of different forms depending, in particular, on the manner in which the composition is to be used. Thus, for example, the composition may be in the form of a powder, tablet, capsule, liquid, ointment, cream, gel, hydrogel, aerosol, spray, micellar solution, transdermal patch, liposome suspension or any other suitable form that may be administered to a person or animal in need of treatment. It will be appreciated that the vehicle of medicaments according to the invention should be one which is well-tolerated by the subject to whom it is given.

The genetic construct or the recombinant vector according to the invention may also be incorporated within a slow- or delayed-release device. Such devices may, for example, be inserted on or under the skin, and the medicament may be released over weeks or even months. The device may be located at least adjacent the treatment site. Such devices may be particularly advantageous when long-term treatment with the genetic construct or the recombinant vector is required and which would normally require frequent administration (e.g. at least daily injection).

In a preferred embodiment, medicaments according to the invention may be administered to a subject by injection into the blood stream, a nerve or directly into a site requiring treatment. For example, the medicament may be injected at least adjacent the retina. Injections may be intravitreal, suprachoroidal, subretinal, intraretinal, intravenous (bolus or infusion) or subcutaneous (bolus or infusion), or intradermal (bolus or infusion).

It will be appreciated that the amount of the genetic construct or the recombinant vector that is required is determined by its biological activity and bioavailability, which in turn depends on the mode of administration, the physiochemical properties of the genetic construct or the recombinant vector and whether it is being used as a monotherapy or in a combined therapy. The frequency of administration will also be influenced by the half-life of the transgene proteins within the subject being treated. Optimal dosages to be administered may be determined by those skilled in the art, and will vary with the particular genetic construct or the recombinant vector in use, the strength of the pharmaceutical composition, the mode of administration, and the advancement of the retinal oedema or the resulting damage to the retina or loss in retinal neurones. Additional factors depending on the particular subject being treated will result in a need to adjust dosages, including subject age, weight, gender, diet, and time of administration.

Generally, a daily dose of between 0.001µg/kg of body weight and 10mg/kg of body weight of DNA plasmid, or between 1 x 10⁸ GC/mL and 1 x 10¹³ GC/mL of the viral vector according to the invention may be used for treating, ameliorating, or preventing a retinal disorder, depending upon the genetic construct or recombinant vector used.

The genetic construct or the recombinant vector may be administered before, during or after onset of the retinal disorder or retinal capillary dysfunction. Daily doses may be given as a single administration (e.g. a single daily injection or inhalation of a nasal spray). Alternatively, the genetic construct or the recombinant vector may require administration twice or more times during a day. As an example, the genetic construct or the recombinant vector may be administered as two (or more depending upon the severity of the retinal disorder or retinal capillary dysfunction being treated) daily doses of between 0.001 µg/kg of body weight and 10 mg/kg of body weight of DNA plasmid, or between 1 x 10⁸ GC/mL and 1 x 10¹³ GC/mL of the viral vector (i.e. assuming a body weight of 70 kg). A patient receiving treatment may take a first dose upon waking and then a second dose in the evening (if on a two dose regime) or at 3- or 4-hourly intervals thereafter. Alternatively, a slow release device may be used to provide optimal doses of the genetic construct or the recombinant vector according to the invention to a patient without the need to administer repeated doses.

Known procedures, such as those conventionally employed by the pharmaceutical industry (e.g., *in vivo* experimentation, clinical trials, etc.), may be used to form specific formulations of the genetic construct or the recombinant vector according to the invention and precise therapeutic regimes (such as daily doses of the agents and the frequency of administration). The inventors believe that they are the first to suggest a bi-cistronic genetic construct encoding a promoter operably linked to coding sequences which will improve retinal survival and reducing GA disease progression through boosting PEDF concentrations and attenuating complement cascade.

According to a sixth aspect, there is provided a pharmaceutical composition comprising the genetic construct according to the first aspect, or the recombinant vector according to the second aspect, and a pharmaceutically acceptable vehicle.

According to a seventh aspect, there is provided a method of preparing the pharmaceutical composition according to the sixth aspect, the method comprising contacting the genetic construct according to the first aspect, or the recombinant vector according to the second aspect, with a pharmaceutically acceptable vehicle.

A "subject" may be a vertebrate, mammal, or domestic animal. Hence, compositions and medicaments according to the invention may be used to treat any mammal, for example livestock (e.g. a horse), pets, or may be used in other veterinary applications. Most preferably, however, the subject is a human being.

A "therapeutically effective amount" of the genetic construct, the recombinant vector or the pharmaceutical composition is any amount which, when administered to a subject, is the amount of the aforementioned that is needed to treat dry age-related macular oedema or GA.

For example, the therapeutically effective amount of the genetic construct, the recombinant vector or the pharmaceutical composition used may be from about 1x10⁸ vector particles to about 1x10¹⁵ vector particles, and preferably from about 1x10¹¹ vector particles to about 1x10¹² vector particles.

In some embodiments, the viral titre of a pharmaceutical composition of the invention is between 5 x 10¹⁰, and 5 x 10¹³ genome copies per millilitre.

In some embodiments, the viral titre of a pharmaceutical composition of the invention is between 5 x 10¹⁰, and 5 x 10¹³ transducing units per millilitre. The term "transducing unit" as used in reference to a viral titre, refers to the number of infectious recombinant vector particles that result in the production of a functional transgene product as measured in functional assays, such as described in [64].

A "pharmaceutically acceptable vehicle" as referred to herein, is any known compound or combination of known compounds that are known to those skilled in the art to be useful in formulating pharmaceutical compositions.

In one embodiment, the pharmaceutically acceptable vehicle may be a solid, and the composition may be in the form of a powder or tablet. A solid pharmaceutically acceptable vehicle may include one or more substances which may also act as flavouring agents, lubricants, solubilisers, suspending agents, dyes, fillers, glidants, compression aids, inert binders, sweeteners, preservatives, dyes, coatings, or tablet-disintegrating agents. The vehicle may also be an encapsulating material. In powders, the vehicle is a finely divided solid that is in admixture with the finely divided active agents according to the invention. In tablets, the active agent (e.g. the genetic construct or recombinant vector according to the invention) may be mixed with a vehicle having the necessary compression properties in suitable proportions and compacted in the shape and size desired. The powders and tablets preferably contain up to 99% of the active agents. Suitable solid vehicles include, for example calcium phosphate, magnesium stearate, talc, sugars, lactose, dextrin, starch, gelatin, cellulose, polyvinylpyrrolidine, low melting waxes and ion exchange resins. In another embodiment, the pharmaceutical vehicle may be a gel and the composition may be in the form of a cream or the like.

However, the pharmaceutical vehicle may be a liquid, and the pharmaceutical composition is in the form of a particle suspension in solution. Liquid vehicles are used in preparing solutions, suspensions, emulsions, syrups, elixirs and pressurized compositions. The genetic construct or the recombinant vector according to the invention may be dissolved or suspended in a pharmaceutically acceptable liquid vehicle such as water, ionic buffered solution, an organic solvent, a mixture of both or pharmaceutically acceptable oils or fats. The liquid vehicle can contain other suitable pharmaceutical additives such as solubilisers, emulsifiers, buffers, preservatives, sweeteners, flavouring agents, suspending agents, thickening agents, colours, viscosity regulators, stabilizers or osmo-regulators. Suitable examples of liquid vehicles for oral and parenteral administration include water (partially containing additives as above, e.g. cellulose derivatives, preferably sodium carboxymethyl cellulose solution), alcohols (including monohydric alcohols and polyhydric alcohols, e.g. glycols) and their derivatives, and oils (e.g. fractionated coconut oil and arachis oil). For parenteral administration, the vehicle can also be an oily ester such as ethyl oleate and isopropyl myristate. Sterile liquid vehicles are useful in sterile liquid form compositions for parenteral administration. The liquid vehicle for pressurized compositions can be a halogenated hydrocarbon or other pharmaceutically acceptable propellant.

Liquid pharmaceutical compositions, which are sterile solutions or suspensions, can be utilized by, for example, intravitreal, suprachoroidal, subretinal, intraretinal, intracameral, intramuscular, intrathecal, epidural, intraperitoneal, intravenous and particularly subcutaneous injection. The genetic construct or the recombinant vector may be prepared as a sterile solid composition that may be dissolved or suspended at the time of administration using sterile water, saline, or other appropriate sterile injectable medium.

Pharmaceutically acceptable carriers, excipients, and diluents are relatively inert substances that facilitate administration or a pharmaceutically effective substance and can be supplied as a liquid solutions or suspensions, as emulsions, or as solid forms suitable for dissolution or suspension in liquid prior to use. For example, an excipient can give forms suitable for or consistency, or act as a diluent. Suitable excipients include, but are not limited to stabilizing agents, wetting and emulsifying agents, salts for varying osmolarity, encapsulating agents, pH buffering substances, and buffers. Such excipients include any pharmaceutical agent suitable for direct delivery to the subject (for example intravitreally or sub-retinally) which may be administered without undue toxicity. Pharmaceutical acceptable excipients include, but are not limited to, sorbitol, any of the various TWEEN compounds, and liquids such as water, saline, glycerol and ethanol. Pharmaceutically acceptable salts can be included therein, for example, mineral acid salts such as hydrochlorides, hydrobromides, phosphates, sulfates and the like; and the salts of organic acids such as acetates, propionates, malonates, or benzoates.

In some embodiments, pharmaceutical acceptable excipients may include pharmaceutical acceptable carriers. Such pharmaceutically acceptable carriers can be sterile liquids, such as water and oil, including those of petroleum, animal, vegetable or synthetic origin, such as peanut oil, soybean oil, mineral oil. Saline solutions and aqueous dextrose, polyethylene glycol (PEG) and glycerol solutions can also be employed as liquid carriers, particularly for injectable solutions. Additional ingredients may also be used, for example preservatives, buffers, tonicity agents, antioxidants and stabilizers, non-ionic wetting or clarifying agents, or viscosity-increasing agents. A thorough discussion of pharmaceutical acceptable excipients and carriers is available in Remington's Pharmaceutical Sciences (Ed Remington JP and Gennaro AR; Mack Pub. Co. Easton, Pa 1990).

It will be appreciated that the invention extends to any nucleic acid or peptide or variant, derivative or analogue thereof, which comprises substantially the amino acid or nucleic acid sequences of any of the sequences referred to herein, including variants or fragments thereof. The terms "substantially the amino acid/nucleotide/peptide sequence", "variant" and "fragment", can be a sequence that has at least 40% sequence identity with the amino acid/nucleotide/peptide sequences of any one of the sequences referred to herein, for example 40% identity with the sequence identified as SEQ ID No: 1-90, and so on.

Amino acid/polynucleotide/polypeptide sequences with a sequence identity which is greater than 65%, more preferably greater than 70%, even more preferably greater than 75%, and still more preferably greater than 80% sequence identity to any of the sequences referred to are also envisaged. Preferably, the amino acid/polynucleotide/polypeptide sequence has at least 85% identity with any of the sequences referred to, more preferably at least 90% identity, even more preferably at least 92% identity, even more preferably at least 95% identity, even more preferably at least 97% identity, even more preferably at least 98% identity and, most preferably at least 99% identity with any of the sequences referred to herein.

The skilled technician will appreciate how to calculate the percentage identity between two amino acid/polynucleotide/polypeptide sequences. In order to calculate the percentage identity between two amino acid/polynucleotide/polypeptide sequences, an alignment of the two sequences must first be prepared, followed by calculation of the sequence identity value. The percentage identity for two sequences may take different values depending on:- (i) the method used to align the sequences, for example, ClustalW, BLAST, FASTA, Smith-Waterman (implemented in different programs), or structural alignment from 3D comparison; and (ii) the parameters used by the alignment method, for example, local vs global alignment, the pair-score matrix used (e.g. BLOSUM62, PAM250, Gonnet etc.), and gap-penalty, e.g. functional form and constants.

Having made the alignment, there are many different ways of calculating percentage identity between the two sequences. For example, one may divide the number of identities by: (i) the length of shortest sequence; (ii) the length of alignment; (iii) the mean length of sequence; (iv) the number of non-gap positions; or (iv) the number of equivalenced positions excluding overhangs. Furthermore, it will be appreciated that percentage identity is also strongly length dependent. Therefore, the shorter a pair of sequences is, the higher the sequence identity one may expect to occur by chance.

Hence, it will be appreciated that the accurate alignment of protein or DNA sequences is a complex process. The popular multiple alignment program ClustalW [65,66] is a preferred way for generating multiple alignments of proteins or DNA in accordance with the invention. Suitable parameters for ClustalW may be as follows: For DNA alignments: Gap Open Penalty = 15.0, Gap Extension Penalty = 6.66, and Matrix = Identity. For protein alignments: Gap Open Penalty = 10.0, Gap Extension Penalty = 0.2, and Matrix = Gonnet. For DNA and Protein alignments: ENDGAP = -1, and GAPDIST = 4. Those skilled in the art will be aware that it may be necessary to vary these and other parameters for optimal sequence alignment.

Preferably, calculation of percentage identities between two amino acid/polynucleotide/polypeptide sequences may then be calculated from such an alignment as (N/T)*100, where N is the number of positions at which the sequences share an identical residue, and T is the total number of positions compared including gaps and either including or excluding overhangs. Preferably, overhangs are included in the calculation. Hence, a most preferred method for calculating percentage identity between two sequences comprises (i) preparing a sequence alignment using the ClustalW program using a suitable set of parameters, for example, as set out above; and (ii) inserting the values of N and T into the following formula:- Sequence Identity = (N/T)*100.

Alternative methods for identifying similar sequences will be known to those skilled in the art. For example, a substantially similar nucleotide sequence will be encoded by a sequence which hybridizes to DNA sequences or their complements under stringent conditions. By stringent conditions, we mean the nucleotide hybridises to filter-bound DNA or RNA in 3x sodium chloride/sodium citrate (SSC) at approximately 45°C followed by at least one wash in 0.2x SSC/0.1% SDS at approximately 20-65°C. Alternatively, a substantially similar polypeptide may differ by at least 1, but less than 5, 10, 20, 50 or 100 amino acids from the sequences shown herein.

Due to the degeneracy of the genetic code, it is clear that any nucleic acid sequence described herein could be varied or changed without substantially affecting the sequence of the protein encoded thereby, to provide a functional variant thereof. Suitable nucleotide variants are those having a sequence altered by the substitution of different codons that encode the same amino acid within the sequence, thus producing a silent change. Other suitable variants are those having homologous nucleotide sequences but comprising all, or portions of, sequence, which are altered by the substitution of different codons that encode an amino acid with a side chain of similar biophysical properties to the amino acid it substitutes, to produce a conservative change. For example, small non-polar, hydrophobic amino acids include glycine, alanine, leucine, isoleucine, valine, proline, and methionine. Large non-polar, hydrophobic amino acids include phenylalanine, tryptophan and tyrosine. The polar neutral amino acids include serine, threonine, cysteine, asparagine and glutamine. The positively charged (basic) amino acids include lysine, arginine and histidine. The negatively charged (acidic) amino acids include aspartic acid and glutamic acid. It will therefore be appreciated which amino acids may be replaced with an amino acid having similar biophysical properties, and the skilled technician will know the nucleotide sequences encoding these amino acids. All of the features described herein (including any accompanying claims, abstract and drawings), and/or all of the steps of any method or process so disclosed, may be combined with any of the above aspects in any combination, except combinations where at least some of such features and/or steps are mutually exclusive.

All of the features described herein (including any accompanying claims, abstract and drawings), and/or all of the steps of any method or process so disclosed, may be combined with any of the above aspects in any combination, except combinations where at least some of such features and/or steps are mutually exclusive.

For a better understanding of the invention, and to show how embodiments of the same may be carried into effect, reference will now be made, by way of example, to the accompanying Figure, in which:-
**Figure 1** is an illustration of one embodiment of a viral vector (top of figure) according to the invention, which expresses various transgene proteins, i.e. a PEDF receptor agonist and an anti-complement protein, and their biological effects in reducing the pathophysiology associated with retinal disorders, such as geographic atrophy and dry-AMD.
**Figure 2** shows a schematic drawing of one embodiment of a genetic construct according to the invention. The construct is a bicistronic cassette where a coding sequence for the PEDF receptor agonist is preceded by a signal peptide which directs cell secretion; a coding sequence for an anti-complement protein which is also preceded by a signal peptide which directs cell secretion; both are linked in either orientation by the Enzyme cut-viral-2A linker sequence/skipping site.
**Figure 3** illustrates the intracellular processing of the genetic material from the gene therapy construct in order to generate two mature therapeutic proteins capable of protecting retinal cells and neutralising or attenuating complement activation. Step 1 is the transcription of the messenger RNA via the single promoter. Step 2 is the translational skipping by the ribosome directed by the viral 2A sequences to give rise to two separate proproteins. Step 3 occurs at the level of the Golgi, in which the viral-2A sequences are cleaved from the pro-proteins via the activity of the furin/enzyme at the upstream cleavage site. Step 4 is the removal of the secretory signal peptides, which removes the remaining proline amino acid from the N-terminus of the downstream component, prior to secretion from the target retinal cells.
**Figure 4** shows images of enhanced Green Fluorescent Protein (eGFP) reporter gene expression in HEK293 cells taken 24 hours after transduction with rAAV2 vectors containing different promoter sequences: the small chicken beta-actin promoter/cytomegalovirus enhancer promoter (sCAG), the sCAG promoter followed by the addition of an intron created from fusing a short stretch of nucleotides derived from the 5' and 3' of rabbit beta-globulin intron (sCAG-intron), the cytomegalovirus promoter (CMV), the murine phosphoglycerate kinase promoter (mPGK) and the human synaptin-1 promoter (hSYN1).

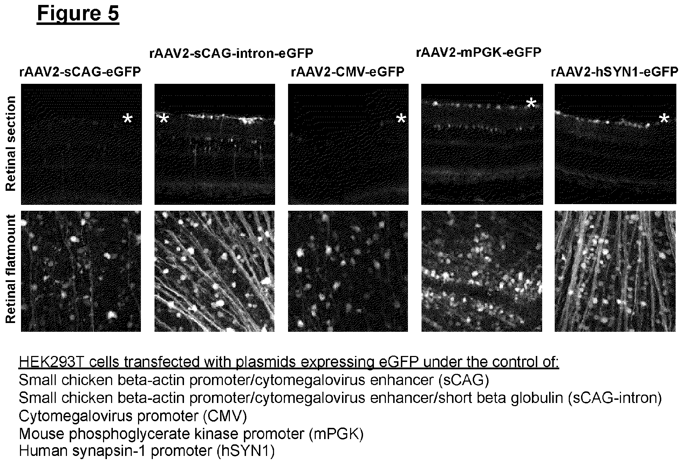

**Figure 5** shows both cross-sectional and flatmount images of the mouse retina, to illustrate the level of eGFP expression three weeks after intravitreal injection with rAAV2 vectors containing different promoters: the small chicken beta-actin promoter/cytomegalovirus enhancer promoter (sCAG); the cytomegalovirus enhancer element plus the chicken beta-actin promoter and a short stretch of nucleotides derived from the 5' and 3' of rabbit beta-globulin intron (sCAG-intron); the cytomegalovirus promoter (CMV); the murine phosphoglycerate kinase-1 promoter (mPGK); and the human synaptin-1 (hSYN1) promoter. The symbol '*' indicates the ganglion cell layer.

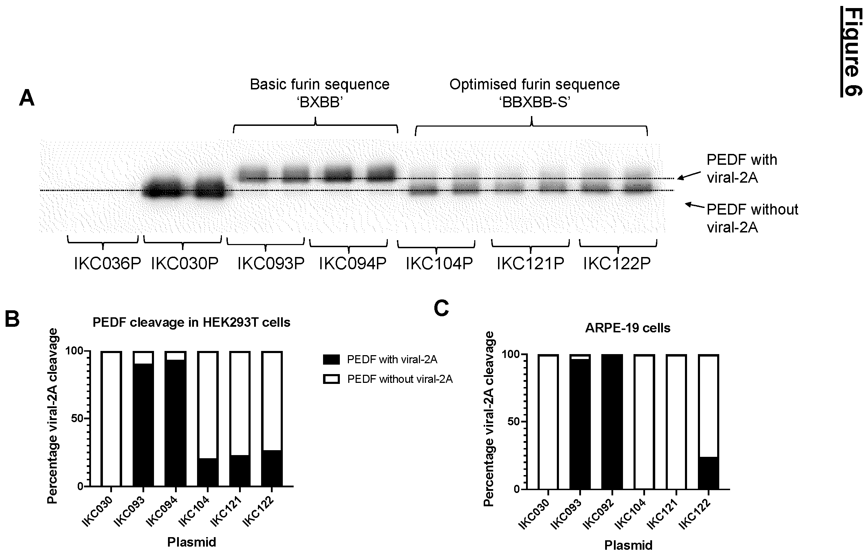

**Figure 6** shows Western blots illustrating the expression of PEDF protein in the supernatant harvested from either HEK293T or ARPE-19 cells 24 hours after transfection with a series of expression plasmids and effective furin cleavage in constructs with the optimised sequence compared to the basic sequence and liberation of the viral-2A linker from the C-terminal of the up-stream protein (Figure 6A). IKC036P [null control], IKC030P [PEDF only], IKC093P [hPEDF-basic furin-viralP2A-anti-Bb SCVF], IKC094P [hPEDF-basic furin-viralP2A-anti-C5 SCVF], IKC104P [hPEDF-optimised furin-viralP2A-anti-C3b SCVF], IKC121P [hPEDF-optimised furin-viralP2A-anti-C3b SCVF], IKC122P [hPEDF-optimised furin-viralP2A-sCD55]. Figure 6B shows percentage furin cleavage in HEK293T supernatants, and Figure 6C shows percentage furin cleavage in ARPE-19 supernatants.

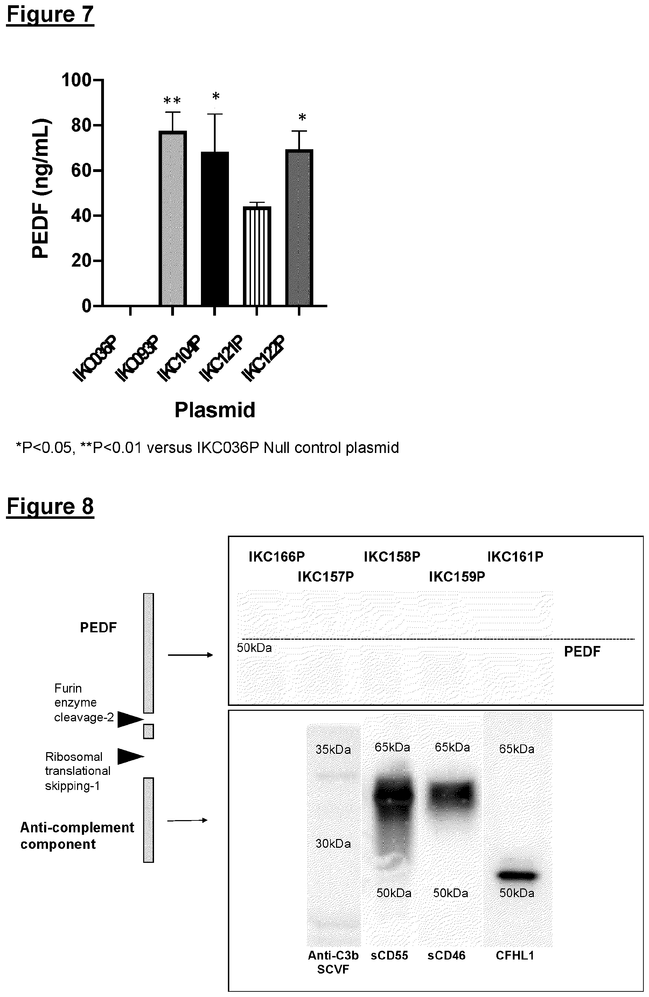

**Figure 7** is a graphical representation of an ELISA assay to illustrate PEDF concentrations released from HEK293T cells in to the cell culture medium 24 hours after transfection with a series of bi-cistronic or control plasmids; IKC036 [null control], IKC093P, IKC104P, IKC121P, and IKC122P.
**Figure 8** is a Western blot to illustrate the intracellular processing and release of the PEDF protein and non-membrane-bound anti-complement factors into the culture medium following transfection of HEK293T cells with the plasmids IKC157P, IKC158P, IKC159P and IKC161P, versus the Null control IKC166 plasmid.

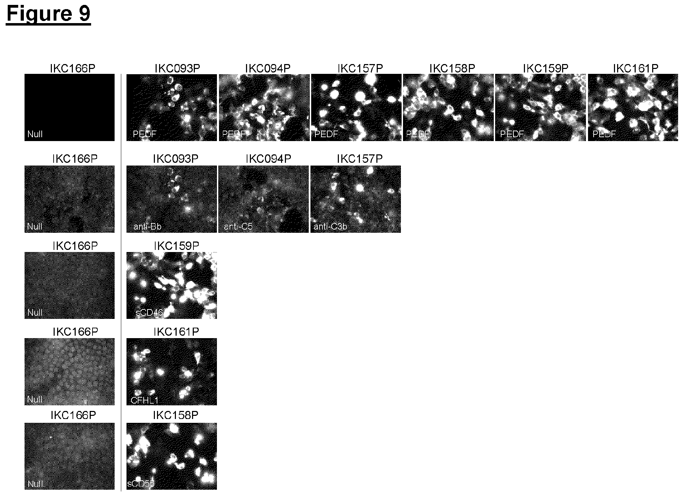

**Figure 9** shows HEK293T cells expressing PEDF and the non-membrane bound anti-complement proteins prior to secretion using immunocytochemistry (light stain) to release into the culture medium following transfection with the plasmids IKC093P, IKC094P, IKC157P, IKC158P, IKC159P and IKC161P, versus the Null control IKC166P plasmid.

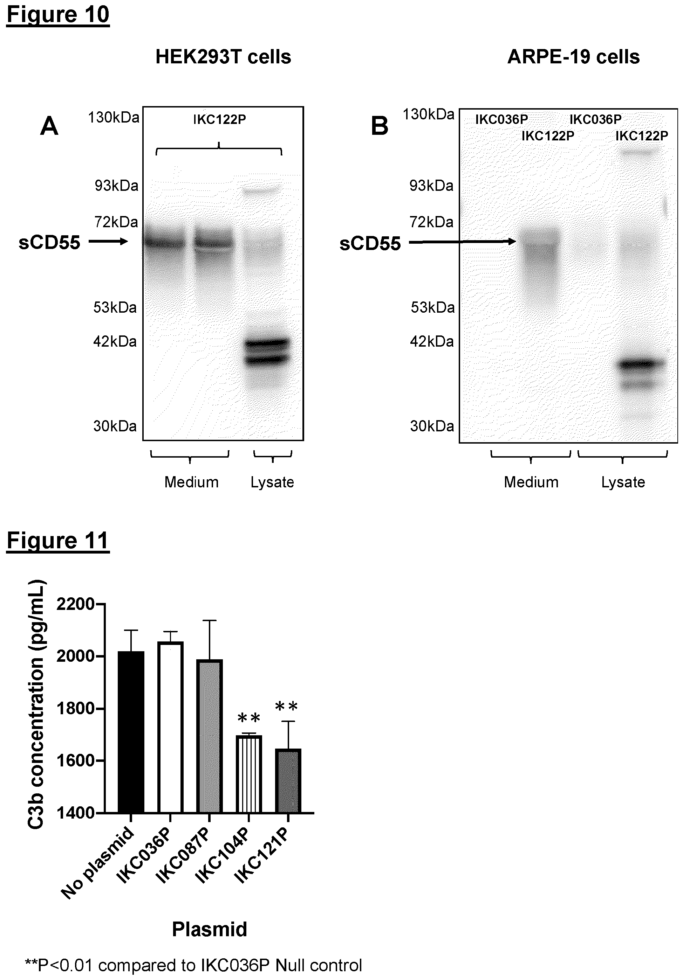

**Figure 10A and B** illustrates the production and release of the soluble sCD55 (DAF) from HEK293T cell culture medium or ARPE-19 cell culture medium respectively, following transfection with the IKC122P plasmid or control IKC036 null plasmid.
**Figure 11** illustrates data showing neutralisation and/or reduction in complement C3b in human serum following incubation of serum with cell growth medium from HEK293T cells transfected with Null control plasmid (IKC036P) or IKC087P, IKC104P IKC0121P, which secreted the single chain variable fragment capable of binding to and neutralising human C3b. Note that the IKC087P has a non-optimised expression cassette and that the ELISA antibody has 80% cross-reactivity with human C3 (constituting approximately 2/3 of the immunoreactivity and so a 30% reduction in the reading will equate to almost 100% neutralisation of C3b as the SCVF does not bind to C3).

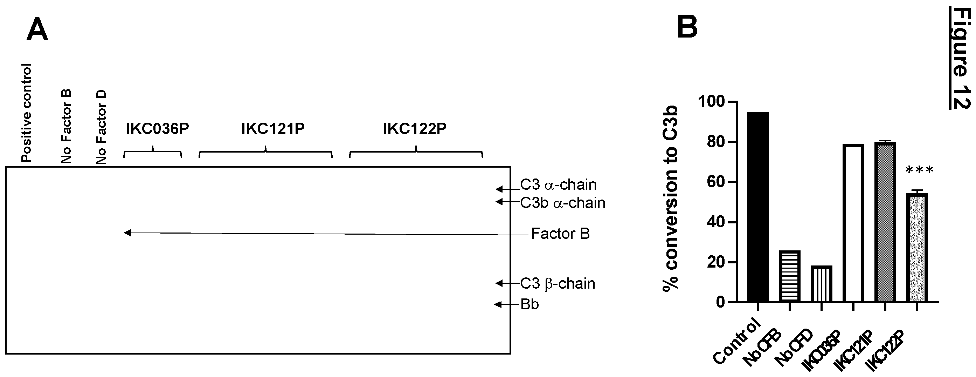

**Figure 12A** demonstrates the ability of supernatant harvested from HEK293T cells transfected with the IKC122P plasmid construct, which produces sCD55, to reduce the generation of recombinant C3b (C3 convertase) from parent C3 in the presence of factor B and D compared to the Null control plasmid (IKC036P) and the IKC121P plasmid construct that expresses the single chain variable fragment capable of binding to and neutralising human C3b. Figure 12B shows that the reduction in the percentage conversion of C3 to C3b in the presence of IKC121P is significant compared to IKC036P control.

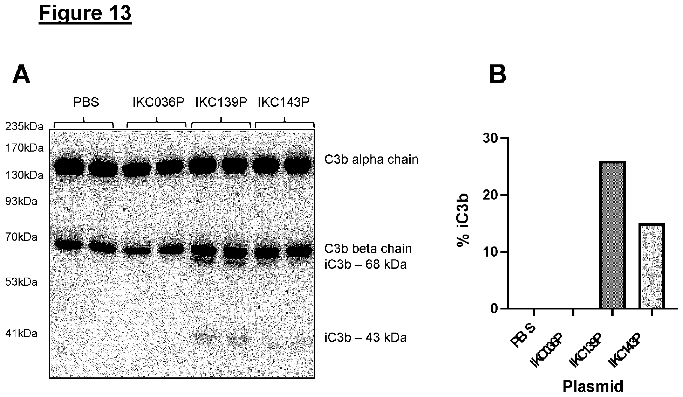

**Figure 13A** illustrates that supernatant harvested from HEK293T cells transfected with the constructs IKC139P and IKC143P, which generate the Factor I co-factors, sCD46 and CFHL1, respectively, can facilitate recombinant C3b breakdown into two iC3b fragments (68 and 43 kDa) in the presence of low concentrations of recombinant CFI. Note no breakdown of C3b produced by the Null control plasmid IKC036P or the PBS control. Figure 13B shows the percentage of iC3b fragments in the presence of IKC139P and IKC143P compared to the IKC036P and PBS controls that showed no breakdown of C3b to iC3b.

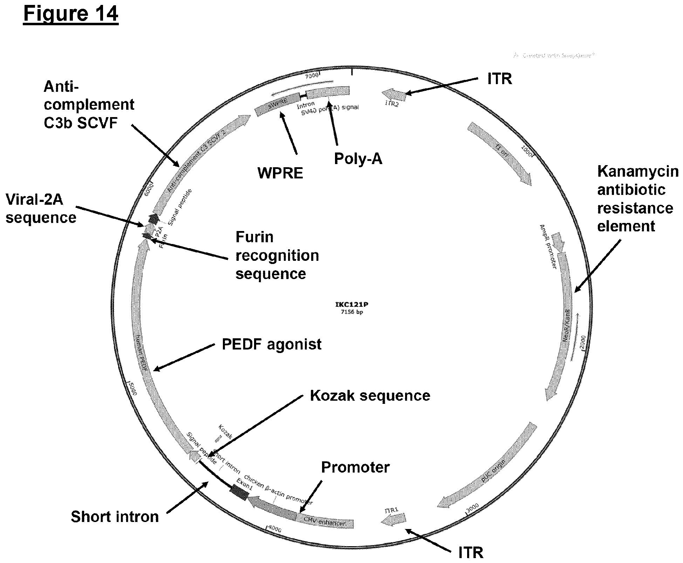

**Figure 14** shows an embodiment of a plasmid map of the "IKC121P" vector of the invention.

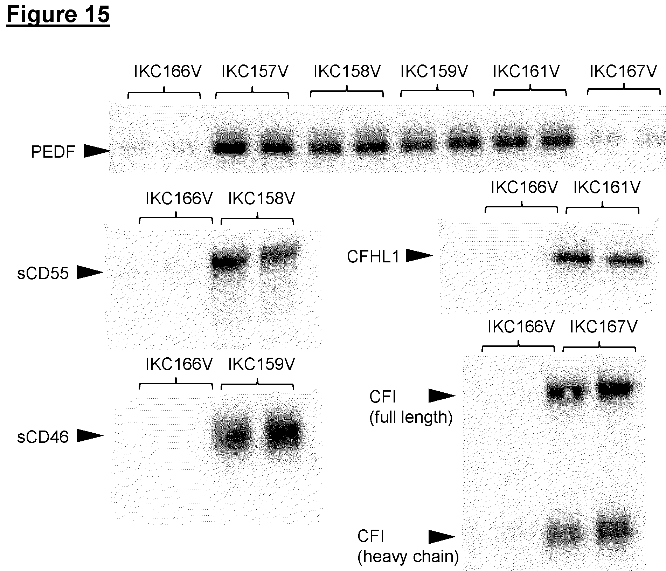

**Figure 15** shows western blot data of PEDF and anti-complement transgene expression which are expressed and secreted in to the culture medium 48 hours following transduction of HEK293T cells with the vectors IKC157V, IKC158V, IKC159V, IKC161V, IKC167V versus the Null control vector IKC166V.

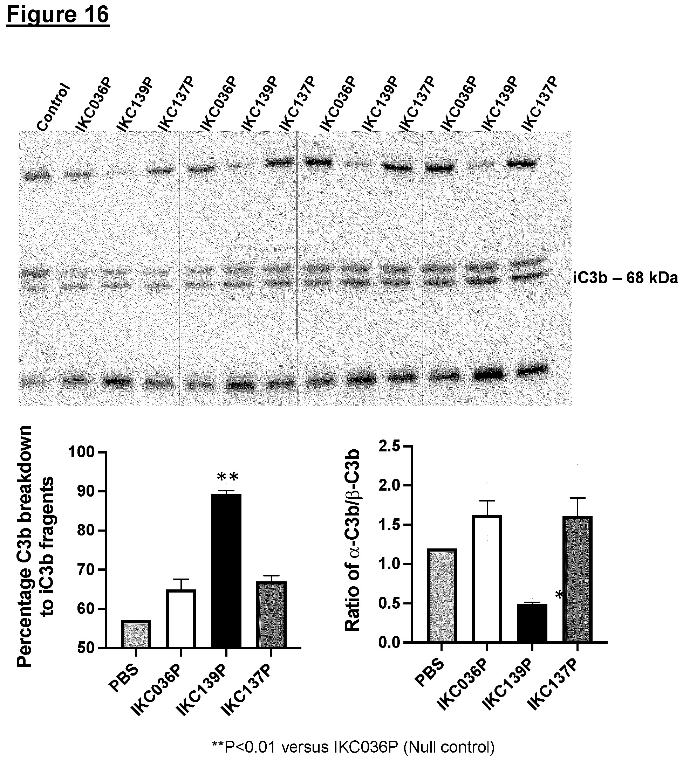

**Figure 16** compares the effects of the addition of HEK293T transfected cells with plasmid expressing either soluble CD46 (IKC137P) or complement I (IKC139P) on complement C3b factor (39nM) breakdown by low concentrations of recombinant complement factor I (11 nM) and factor H (0.5 nM), versus Null control transfected cells (IKC036P). Note that supplementation of the recombinant complement factor I with the cell culture medium harvested from HEK293T cells transfected with IKC137P (complement I) did not increase C3b breakdown, as compared to IKC036P Null controls. In contrast, supplementation with cell culture medium harvested from HEK293T cells transfected with IKC139P (soluble CD46) significantly increased enzymatic C3b breakdown as seen by the decrease in the C3b alpha chain band and the increase in the iC3b (68 kDa and 43 kDa) bands.

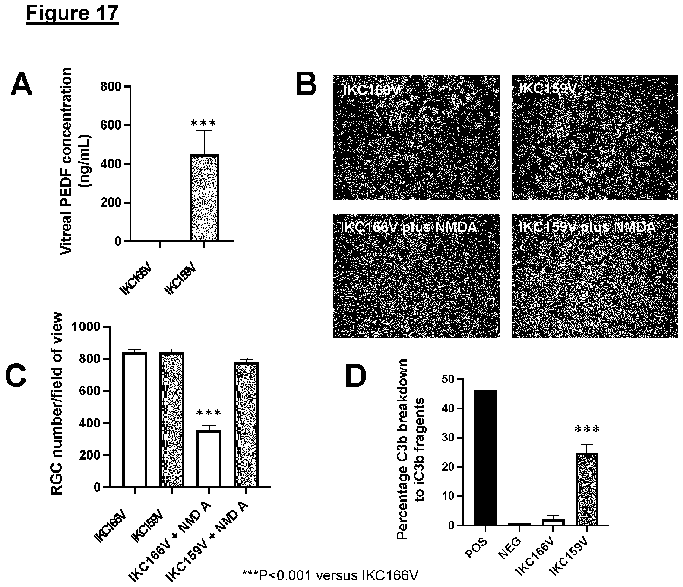

**Figure 17** illustrates that intravitreal injection of IKC159V rAAV2 vector increases vitreal PEDF concentrations (A) and protects retinal ganglion cells (B and C) when challenged with the neurotoxin N-methyl-D-aspartate (NMDA) (8 day challenge 3 weeks after gene therapy delivery). In addition, the rAAV2 vector is able to secrete sufficient soluble CD46 into the vitreous which is able to significantly breakdown recombinant complement C3b (*ex-vivo*) (D) compared to vitreous isolated from animals treated with the Null IKC166V vector.

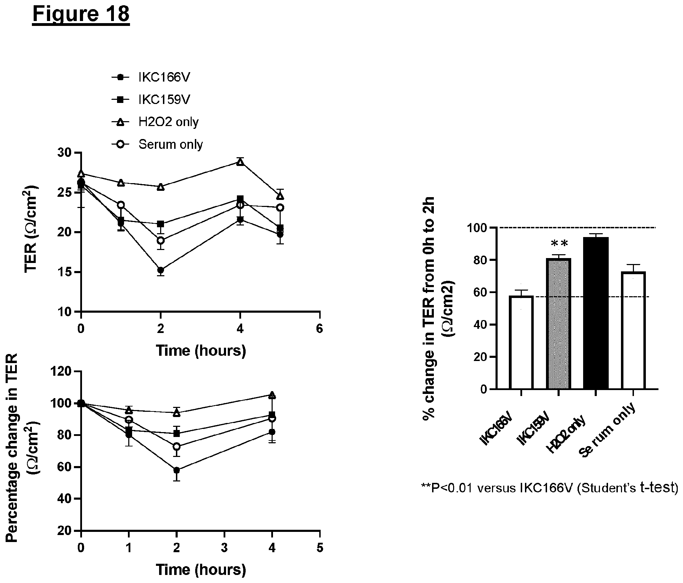

**Figure 18** shows the beneficial effects of IKC159V rAAV2 vector in preventing the transient reduction of ARPE-19 cell transepithelial resistance when challenged with mild oxidative stress (hydrogen peroxide) and complement attack (addition of human serum proteins). Data shows the transient loss in transepithelial resistance at 2 hours and the percentage change from baseline when IKC159V or IKC166V (Null vector) transduced cells were treated with both hydrogen peroxide and human serum. Comparators are ARPE-19 cells treated with hydrogen peroxide or human serum only.

### Examples

Referring to Figures 1 and 2, the inventors have designed and constructed a novel genetic construct, which encodes (i) an agonist of the PEDF receptor and (ii) an anti-complement protein, under the control of a single promoter. As illustrated in Figure 3, the inventors also introduced a spacer sequence into the genetic construct (e.g. a viral-2A peptide spacer sequence), which advantageously enables expression of all of the peptides encoded by the construct to occur under control of a single promoter, as a single mRNA transcript. Additionally, in order to enzymatically remove the viral-2A peptide sequence from the C-terminal of the proteins, the inventors introduced a viral-2A removal sequence into the construct, such as a furin recognition sequence, referring to Figure 6.

As illustrated in Figure 3, the bi-cistronic expression cassette produces two mature therapeutic proteins, a PEDF receptor agonist and an anti-complement protein (Figures 7-11 and 15). The PEDF receptor agonist acts to protect the retinal pigment epithelium (RPE) and other retinal cells, such as photoreceptors, from cytotoxic biochemical insults and cell death. In patients with dry-AMD and geographic atrophy, endogenous concentrations of PEDF in the eye are significantly depleted due to the disease pathology, thereby reducing the retina's ability to function normally. The genetic construct of the invention will supplement retinal PEDF concentrations, thus restoring the retinal defence mechanism against the oxidative damage and other pathophysiological factors at play in dry-AMD. Boosting PEDF concentrations will lead to prevention of further loss to RPE cells and overlying photoreceptors, thereby slowing or halting loss in vision, as illustrated in Figure 17. In addition, the anti-complement protein is capable of reducing complement system activation, which has also been shown to play a significant role in GA, as illustrated in Figures 12, 13, 16-18. Through better protection against retinal cell loss via increased retinal PEDF concentrations coupled to reduced complement activation, the bi-cistronic gene construct is able to attenuate or halt further retinal damage and associated loss in visual acuity.

The inventors then introduced the genetic construct into recombinant expression vectors, such as rAAV2 (for example, see Figure 14).

### Materials and Methods

### DNA plasmid design and production

Codon optimisation of DNA sequences was performed using the tools (http://www.jcat.de) or the Genscript online tool). Synthetic DNA blocks and cloning were performed by using standard molecular biology techniques. All DNA Plasmids were scaled up in SURE competent cells (Agilent Technologies) overnight following maxi-prep purification with minimal endotoxin presence.

IKC036P is a null control. IKC030P comprises PEDF only. IKC093P comprises [hPEDF-basic furin-viralP2A-anti-Bb SCVF], IKC094P comprises [hPEDF-basic furin-viralP2A-anti-C5 SCVF], IKC104P comprises [hPEDF-optimised furin-viralP2A-anti-C3b SCVF], IKC121P comprises [hPEDF-optimised furin-viralP2A-anti-C3b SCVF], and IKC122P comprises [hPEDF-optimised furin-viralP2A-sCD55], IKC157P comprises [hPEDF-optimised furin-viralP2A-anti-C3b SCVF], IKC158P [hPEDF-optimised furin-viralP2A-sCD55], IKC159P [hPEDF-optimised furin-viralP2A-sCD46], IKC161P [hPEDF-optimised furin-viralP2A-CFHL1] and IKC166P [Null control].

### Recombinant AAV vector production

Recombinant AAV2 vectors were manufactured using the DNA plasmids. HEK293 cells (2.5x10⁸) were transduced with a total of 500µg of the three plasmids (Rep-2-Cap2, pHelper and ORF and ITR containing plasmid). Following freeze-thaw of the HEK293 cells to liberate the viral vector particles, followed by iodixanol gradient ultracentrifugation and de-salting. The vectors were suspended in Dulbecco's phosphate-buffered saline (DPBS) buffer from Thermo Fisher/Gibco manufactured to cGMP standard (cat number 14190250 consisting 8g/L NaCl, 1.15g/L of Na₂HPO₄, 0.2 g/L of KCl and 0.2 g/L of K₂HPO₄ with no calcium or magnesium; pH 7.0-7.3, 270-300 mOsm/kg) the following vector titres were obtained by qPCR using primers recognising the ITR region.

Figure 4 illustrates enhanced Green Fluorescent Protein (eGFP) reporter gene expression in HEK293 cells taken 24 hours after transduction with rAAV2 vectors containing different promoter sequences: sCAG, sCAG-intron, CMV, hSYN1 and mPGK.

As illustrated in Figure 4, both sCAG and CMV display high level of eGFP transgene expression in HEK293 cells.

Additionally, Figure 5 shows both cross-sectional and flatmount images of the mouse retina to illustrate the level of eGFP expression three weeks after intravitreal injection with rAAV2 vectors containing different promoter sequences: sCAG, sCAG-intron, CMV, mPGK and hSYN1. As can be seen from Figure 5, the addition of the intron in the sCAG-intron promoter increases retinal expression compared to the same promoter without the intron, i.e. sCAG.

### Example 1 - PEDF concentration in HEK293T cells after cell transfection with various plasmid constructs

Briefly, DNA plasmids were mixed with Opti-MEM (FisherSci; Loughborough, Leics., U.K.) and lipofectamine 3000 (FisherSci) and added to HEK293T cells cultured to 80% confluency in 24-well plates such that each well received 0.5 µg of plasmid DNA and 0.75 µL lipofectamine. Cells were incubated for 24 hours at 37°C, 5% CO₂. The HEK293T cell incubation medium was collected and centrifuged to remove any cell debris and the PEDF concentrations generated from the cells were subsequently measured using a commercial human PEDF ELISA kit (Abcam; Cambridge, U.K.; ab246535) or by Western blot (Abcam ab180711, diluted 1:1000). Control Null plasmid (IKC036P or IKC166P) was not shown to contribute any further PEDF to small amounts generated by HEK293T cells.

Figures 6 and 7 illustrate that the amount of PEDF secreted by the HEK293T cells 24 hours after transfection with a series of plasmids is significantly greater than that of the null control (IKC036P and IKC166P).

### Example 2 - Detection of furin activity and viral-2A peptide cleavage

HEK293T cells were transfected with plasmids as described above. The molecular weights of the test transgenes from the bi-cistronic constructs (PEDF and anti-complement proteins) were compared to transgene constructs which produce only a single transgene. To confirm cleavage of the viral-2A peptide from the C-terminal, a 2A antibody (NBP2-59627) from Bio-Techne (Abingdon, Oxon, U.K.) was used to examine for viral-2A peptide presence.

Figure 6 illustrates the expression of PEDF protein (with and without viral-2A) in the supernatant harvested from HEK293T and ARPE-19 cells, 24 hours after transfection with a series of expression plasmids. Additionally, Figures 6B and 6C illustrate the quantification of effective furin cleavage and liberation of the viral-2A linker from the C-terminal of PEDF in HEK293T and ARPE-19 cells transfected with a series of plasmids to show that the optimised furin sequence in the IKC104P, IKC121P and IKC122P plasmids is significantly more effective than the basic furin sequence in the IKC093P and IKC094P plasmids. The PEDF derived from HEK293T cells transfected with the IKC030P plasmid was used as a control since this does not have a C-terminal furin or viral 2A sequence.

### Example 3 - detection of expressed anti-complement proteins

Figures 8 and 9 demonstrate generation, correct processing and release of the PEDF and downstream anti-complement proteins in HEK293T cells following transfection with plasmids. In Figure 8, the release of both PEDF and anti-complement proteins in to the culture medium from HEK293T cells transfected with either the control IKC166P (Null) or the IKC157P, IKC158P, IKC159P and IKC161P plasmids via Western blot using the same antibodies as used for the immunocytochemistry (below) and all diluted 1:1000. The secondary antibody was a goat-anti-rabbit at 1:10,000 dilution (Abcam, ab6721).

Shown in Figure 9 are the PEDF and anti-complement proteins within the transfected HEK293T cells grown on coverslips prior to release and stained by immunocytochemistry (anti-Bb, anti-C5 and anti-C3b SCVF stained with a custom rabbit polyclonal antibody generated by Genscript (peptide 1); CD55 Abcam ab133684; CD46 Invitrogen PA535311; and CFH Abcam ab133536). In Figure 15, the release of both PEDF and anti-complement proteins in to the culture medium from HEK293T cells transduced with rAAV vectors including the control IKC166V (Null vector) or the IKC157V, IKC158V, IKC159V, IKC161V and IKC167V rAAV2 vectors, was evaluated via Western blotting, using the same antibodies as described above for Figures 8 and 9.

Figure 10 illustrates the production and secretion of a soluble/non-cell membrane-bound form of (DAF) sCD55 in both HEK293T and ARPE-19 cells, 24 hours after transfection with the plasmid IKC122P by Western blot using the CD55 antibody (1:1000 dilution ab-133684 from Abcam (Cambridge, U.K.) and peroxidase-labelled goat anti-rabbit secondary antibody (1:10,000 dilution, abcam, ab6721).

### Example 4 - demonstration of anti-complement protein activity

Activity of the anti-complement proteins is shown in Figures 11, 12, 13 and 16, in which neutralisation of C3b is shown in Figure 11, prevention of C3 convertase (C3bBb) generation is illustrated in Figure 12 and the complement factor I (CFI)-mediated C3b cleavage assay is shown in Figures 13 and 16.

For the C3b neutralisation assay shown in Figure 11, HEK293T cells were transfected with plasmids as described above. After 24 hours the supernatant was collected and clarified by brief centrifugation. The supernatant (190/µL) was incubated with 10/µL of normal human serum (diluted 1:20,000) at room temperature for 30 minutes with gentle agitation. Following incubation, the samples were quantified using a Human Complement C3b ELISA kit (abcam, ab195461) that has 80% cross-reactivity with human C3 (constituting approximately 2/3 of the immunoreactivity and so a 30% reduction in the reading will equate to almost 100% neutralisation of C3b as the SCVF does not bind to C3).

For the C3 convertase assay shown in Figure 12, recombinant proteins (C3, 0.2µM; Complement Factor B; 0.2µM and Complement Factor D, 0.02µM, final concentrations; Complement Technologies Inc., Tyler, TX75703, U.S.A) were incubated in veronal buffer with HEK293T culture medium previously transfected with various plasmids in a total volume of 50µL for 30 min at 37°C. Following incubation, the generation of C3 convertase was measured by SDS-Page electrophoresis and staining the gel using SimplyBlue Safe stain (Thermofisher).

For the C3b cleavage assay illustrated in Figure 13, HEK293T culture medium previously transfected with various plasmids was incubated with C3b substrate (42 nM) and recombinant CFI (1.2 nM) (Complement Technologies Inc.) in a total volume of 60µL for 60 min at 37°C. The C3b breakdown products (iC3b 64kDa and 43kDa) were examined by Western blot using a goat anti-human C3 antibody (AHP1752 dilution 1:2,000; BioRad) and peroxidase-labelled donkey anti-goat secondary antibody (705-035-147 at 1:10,000; Jackson ImmunoResearch Europe, Ely, U.K.).

For the C3b cleavage assay illustrated in Figure 16, medium from transfected HEK293T cells was combined with recombinant complement factors. The concentrations of recombinant complement factor I and factor H used previously were reduced to 11 nM and 0.5 nM, respectively, whilst the substrate C3b was maintained around 39 nM. Note that addition of more complement factor I from the HEK293T culture medium (IKC137P) did not significantly increase C3b breakdown, whereas addition of HEK293T culture medium containing soluble CD46 co-factor was able to significantly boost C3b breakdown (IKC139P). These data would suggest that C3b breakdown is more sensitive to co-factor addition than to factor I supplementation.

### Example 5 - demonstration of anti-complement protein and PEDF activity of a bi-cistronic rAAV vector in vivo.

Activity of both the sCD46 and PEDF proteins following intravitreal delivery of the IKC159V (soluble CD46 vector) is shown in Figure 17.

Mice were intravitreally injected (2 µL) with IKC166V (Null control) or IKC159V. After 21 days, their eyes were dissected free and vitreous samples (between 4 and 5 µL) were extracted and used to assess C3b breakdown *ex-vivo* using the C3b cleavage assay method described above. The results showed a significant breakdown of C3b in vitreous from IKC159V treated eyes compared to the IKC166V (Null) group.

Another set of mice receiving intravitreal injection (2 µL) of IKC166V (Null control) or IKC159V were used in the NMDA study. Twenty one days after vector injection, mice received a further intravitreal injection of NMDA (30 nmol/eye) or vehicle, and 8 days later the animals were terminated. Vitreal samples were obtained from vehicle injected eyes and PEDF concentration measured using a commercial PEDF ELISA kit (Abcam). Retinal flat-mounts were prepared from all eyes and retinal ganglion cell counts were measured using RBPMS immunolabelling. Of note is the approximately 50% loss of retinal ganglion cells in the IKC166V (Null) plus NMDA treatment group compared to an almost complete protection in the IKC159V plus NMDA treatment group.

### Example 6 -Demonstration of the beneficial effects of bi-cistronic rAAV2 vector (IKC159V) on preventing a reduction in ARPE-19 cell transepithelial resistance

For the ARPE-19 cell transepithelial resistance (TER) assay shown in Figure 18, the ARPE-19 cells were grown on transwells (24 well, Greiner) for 2 weeks with regular media change until a stable monolayer and TER was reached. The media was then exchanged for serum free medium for an additional 2 weeks before transduction of the monolayer with rAAV vectors for 48 hours. TER assay readings were taken before and 1, 2 and 4 hours after exposure to 1 mM H₂O₂ and human serum. As illustrated in Figure 18, the IKC159V rAAV2 vector prevented the transient reduction of ARPE-19 cell transepitheliual resistance when challenged with mild oxidative stress.

### Discussions and Conclusions

As illustrated in the Examples, the inventors have demonstrated that it is surprisingly possible to combine the open reading frames (ORFs) which code for a PEDF receptor agonist and an anti-complement protein, in a single genetic construct.

The PEDF receptor agonist restores concentrations of PEDF, thereby reducing inflammation and preserving RPE and photoreceptor cells. Additionally, the anti-complement protein is capable of neutralising or attenuating the alternative complement pathway, thereby arresting further RPE cell loss. Advantageously, the genetic construct of the invention targets the AP pathway, meaning the classical and lectin pathways of the complement system are preserved, thereby maintaining the antimicrobial defence system which can facilitate destruction of an invading pathogen.

### References

1. Tufail A, et al. Objective measurement of reading speed and correlation with patient-reported functional reading independence. Presented at the 15th EURETINA Congress, Nice, France, September 17-20, 2015.
2. The Eye Diseases Prevalence Research Group (2004). Prevalence of age-related macular degeneration in the United States. Arch Ophthalmol., vol.122, PP: 564-572
3. Age-Related Eye Disease Study Research Group. Risk factors associated with age-related macular degeneration. A case-control study in the age-related eye disease study: Age-Related Eye Disease Study Report Number 3. Ophthalmology. 2000, vol.107, PP: 2224-232.
4. Chakravarthy U, Augood C, Bentham GC, de Jong PT, Rahu M, Seland J, Soubrane G, Tomazolli L, Topouzis F, Vingerling JR, Vioque J, Young IS, Fletcher AE (2007). Cigarette smoking and age-related macular degeneration in the EUREYE Study. Ophthalmology vol. 114(6), PP: 1157-63.
5. Smith W, Assink J, Klein R, Mitchell P, Klaver CC, Klein BE et al. Risk factors for age-related macular degeneration: Pooled findings from three continents. Ophthalmology 2001, vol. 108, PP: 697-704.
6. Fraser-Bell S, Donofrio J, Wu J, Klein R, Azen SP, Varma R, Los Angeles Latino Eye Study Group (2005). Sociodemographic factors and age-related macular degeneration in Latinos: the Los Angeles Latino Eye Study. American Journal of Ophthalmology, vol. 139, PP: 30-38.
7. Young RW (1987). Pathophysiology of age-related macular degeneration. Surv. Ophthalmol., vol. 31, PP: 291-306.
8. Pilgrim MG, Lengyel I, Lanzirotti A, Newville M, Fearn S, Emri E, Knowles JC, Messinger JD, Read RW, Guidry C, Curcio CA (2017). Sub retinal pigment epithelial deposition of Drusen components including hydroxyapatite in a primary cell culture model. Invest. Ophthamol. Vis. Sci., vol. 58, PP: 708-719.
9. Schmitz-Valckenberg S, Fleckenstein M, Gobel AP, Hohman TC, Holz FG (2011). Optical coherence tomography and autofluorescense findings in areas with geographic atrophy due to age-related macular degeneration. Invest. Ophthalmol. Cis. Sci. 2011, vol. 52(1), PP: 1-6.
10. Marsiglia M, Boddu S, Bearelly S, Xu L, Breaux BE Jr, Freund KB, Yannuzzi LA, Smith RT (2013). Association between geographic atrophy progression and reticular pseudodrusenin eyes with dry age-related macular degeneration. Invest Ophthalmol Vis Sci., vol. 54, PP: 7362-7369.
11. Kennedy CJ, Raboczy PE, Constable IJ (1995). Lipofuscin of the retinal pigment epithelium: a review. Eye, vol. 9, PP: 763-771.
12. Feeney-Burns L, Hilderbrand ES, Eldridge S (1984). Aging human RPE: morphometric analysis of macular, equatorial and peripheral cells. Invest. Ophthamol. Vis. Sci., vol. 25, PP: 195-200.
13. Dorey CK,Wu G, Ebenstein D, Garsd A, Weiter JJ (1989). Cell loss in the aging retina. Relationship to lipofuscin accumulation and macular degeneration. Invest. Ophthamol. Vis, Sci., 30(8), 1691-1699.
14. Delori FC, Dorey DDK, Staurenghi G, Arend O, Goger DG, Weiter JJ (1995). In vivo fluorescence of the ocular fundus exhibits retinal pigment epithelium lipofuscin characteristics. Invest. Ophthamol. Vis. Sci., vol. 36, PP: 718-729.
15. Delori FC, (1995). RPE lipofuscin in aging and age-related macular degeneration. In: Coscas G, Piccolino FC, eds. Retinal Pigment Epithelium and Macular Disease: Documenta Ophthalmologica. Dordrecht, The Netherlands, Kluwer; vol. 62, PP: 37-45.
16. Holz FG, Pauleikhoff D, Klein R, Bird AC (2004). Pathogenesis of lesions in late age-related macular disease. Am J Ophthalmol, vol. 137(3), PP: 504-510.
17. Eldred G, Lasky MR (1993). Retinal age pigments generated by self-assembling lysosomotrophic detergents. Nature, vol. 361, PP: 724-726.
18. Sakai N, Decatur J, Nakanishi K, Eldered GE (1996). Ocular age pigment "A2E": an unprecedented pyridinium bisretinoid. J. Am. Chem. Soc., vol. 118, PP: 1559-1560.
19. Ren RX-F, Sakai N, Nakanishi K, Eldred GE (1996). Total synthesis of the ocular age pigment A2E: a convergent pathway. J. Am. Chem. Soc., vol. 119, PP: 3619-3620.
20. Parish CA, Hashimoto M, Nakanishi K, Dillon J, Sparrow JR (1998). Isolation and one-step preparation of A2E and iso-A2E, fluorophores from human retinal pigment epithelium. Proc. Natl. Acad. Sci. USA, vol. 95, PP: 14609-14613.
21. Boyer NP, Higbee D, Currin MB, Blakeley LR, Chen C, Ablonczy Z, Crouch RK, Koutalos Y (2012). Lipofuscin and N-retinylidene-N-retinylethaolamine (A2E) accumulate in retinal pigment epithelium in absence of light exposure. J. Biol. Chem., vol. 287(26), PP: 22276-22286.
22. Weiter JJ, Delori FC, Wing GL, Fitch KA (1986). Retinal pigment epithelial lipofuscin and melanin and choroidal melanin in human eyes. Invest. Ophthamol. Vis. Sci., vol. 27, PP: 145-152.
23. Lakkaraju A, Finnemann SC, Rodriguez-Boulan E (2007). The lipofuscin fluorphore A2E perturbs cholesterol metabolism in retinal pigment epithelial cells. Proc. Natl. Acad. Sci. USA, vol. 104(26), PP: 11026-11031.
24. Maeda A, Maeda T, Golczak M, Palczewska G, Dong Z, Golczak M, et al (2008). Retinopathy in mice induced by disrupted all-trans-retinal clearance. J. Biol. Chem., vol. 283(39), PP: 26684-26693.
25. Zhang J, Kiser PD, Badiee M, Palczewska G, Dong Z, Golczak M (2015). Molecular pharmacodynamics of emixustat in protection against retinal degeneration. J. Clin. Invest., vol. 125(7), PP: 2781-2794.
26. Rosenfeld PJ, Dugel PU, Holz FG, Heier JS, Pearlman JA, Novack RL, Csaky KG, Koester JM, Gregory JK, Kubota R (2018). Emixustat hydrochloride for geographic atrophy secondary to age-related macular degeneration. Ophthalmol., vol. 125(10), PP: 1556-1567.
27. Trou LA, Pickering MC, Blom AM (2017). The complement system as a potential therapeutic target in rheumatic disease. Nature Rev., vol. 13, PP: 538-547.
28. Anderson DH, Radeke MJ, Gallo NB, Chapin EA, Johnson PT, Curletti CR, Hancox LS, Hu J, Ebright JN, Malek G, Hauser MA, Rickman CB, Bok D, Hageman GS, Johnson LV (2010). The pivotal role of the complement system in aging and age-related macular degeneration: hypothesis re-visited. Prog. Retin. Eye Res., vol. 29, PP: 95-112.
29. Scholl HPN, Issa PC, Walier M, Janzer S, Pollok-Kopp B, Börncke F, Fritsche LG, Chong NV, Fimmers R, Wienker T, Holz FG, Weber BH, Oppermann M (2008). Systemic complement activation in age related macular degeneration. PLoS ONE, vol. 3(7) P:e2593.
30. Sepp T, Khan JC, Thurlby DA, Shahid H, Clayton DG, Moore AT, Bird AC, Yates JR (2006). Complement factor H variant Y402H is a major risk determinant for geographic atrophy and choroidal neovascularization in smokers and nonsmokers. Invest. Ophthalmol. Vis. Sci., vol. 47(2), PP: 536-540.
31. Cameron DJ, Yang Z, Gibbs D, Chen H, Kaminoh Y, Jorgensen A, Zeng J, Luo L, Brinton E, Brinton G, Brand JM, Bernstein PS, Zabriskie NA, Tang S, Constantine R, Tong Z, Zhang K. (2007). HTRA1 variant confers similar risks to geographic atrophy and neovascular age-related macular degeneration. Cell Cycle 2007, vol. 6(9), PP: 1122-1125.
32. Hageman GS, Luthert PJ, Victor Chong NH, Johnson LV, Anderson DH, Mullins RF (2001). An integrated hypothesis that considers drusen as biomarkers of immune-mediated processes at the RPE-Bruch's membrane interface in aging and age-related macular degeneration. Prog Retin. Eye Res., vol. 20, PP: 705-732.
33. Xu H, Chen M (2016). Targeting the complement system for the management of retinal inflammatory and degenerative diseases. Eur. J. Pharmacol., vol. 787, PP: 94-104.
34. Nilsson SC, Sim RB, Lea SM, Fremeaux-Bacchi V, Blom Am (2011) Complement factor I in health and disease. Mol. Immunol. Vol., 48(14), PP: 1611-1620.
35. Lachmann PJ (2019) The story of complement factor I. Immunology, vol. 224(4), PP: 511-517.
36. Lukacik P, Roversi P, White J, Esser D, Smith GP, Billington J, Williams PA, Rudd PM, Wormald MR, Harvey DJ, Crispin MDM, Radcliffe CM, Dwek RA, Evans DJ, Morgan BP, Smith RAG, Lea SM (2004). Complement regulation at the molecular level: The structure of decay-accelerating factor. Proc. Natl. Acad. Sci. USA, vol. 101(5), PP: 1279-1284.
37. Lubin DM, Atkinson JP (1998). Decay-accelerating factor: Biochemistry, molecular-biology, and function. Ann. Rev. Immunol., vol. 7, PP: 35-58.
38. Williams P, Chaudry Y, Goodfellow IG, Billington J, Powell R, Spiller OB, Evans DJ, Lea S (2003). Mapping CD55 function. J. Biol. Chem., vol. 12, PP: 10691-10696.
39. Dho SH, Lim JC, Kim LK (2018). Beyond the role of CD55 as a complement component. Innune Netw., Vol. 18(1), e11.
40. Skerka C, Chen Q, Fremeaux-Bacchi V, Roumenina LT (2013). Complement factor H related proteins (CFHRs). Mol. Immunol., vol. 56(3), PP: 170-180.
41. Cipriani V, Lores-Motta L, He F, Fathalla D, Tilakaratna V, McHarg S, Bayatti N, Acar IE, Hoyng CB, Fauser S, Moore AT, Yates JRW, de Jong EK, Morgan BP, den Hollander AI, Bishop PN, Clark SJ (2020) Increased circulating levels of Factor H-related Protein 4 are strongly associated with age-related macular degeneration. Nat. Commun. Vol. 11(1), PP778.
42. Lako M, Kavangh D, (2021). Revisiting the role of factor H in age-related macular degeneration: Insights from complement-mediated renal disease and rare genetic variants. Surv. Ophthamol., vol. 66(2), PP: 378-401.
43. Heinen S, Hartmann A, Lauer N, Wiel U, Dahse H-M, Schirmer S, Gropp K, Enghardt T, Wallich R, Hälbich S, Mihlan M, Schlötzer-Schrehardt U, Zipfel PF, Skerka C (2009). Factor H-related protein 1 (CFHR-1) inhibits complement C5 convertase activity and terminal complex formation. Blood, vol. 114(12), PP: 2439-2447.
44. Geller A, Yan J (2019). The role of membrane bound complement regulatory proteins in tumor development and cancer immunotherapy. Front. Immunol., vol. 10; article 1074.
45. Christiansen D, Milland J, Thorley BR, McKenzie IFC, Mottram PL, Purcell LJ, Loveland BE (1996). Engineering of recombinant soluble CD46: an inhibitor of complement activation. Immunology, vol. 87, PP: 348-354.
46. Holz FG, Sadda SR, Busbee B et al (2018). Efficacy and safety of lampalizumab for geographic atrophy due to age-related macular degeneration. JAMA Ophthalmol., vol. 136(6), PP: 666-677.
47. Dreismann AK, MvClements ME, Barnard AR, Orhan E, Hughes JP, Lachmann PJ, MacLaren RE (2021) Functional expression of complement factor I following AAV-mediated gene delivery in the retina of mice and human cells. Gene Ther., https://www.nature.com/articles/s41434-021-00239-9
48. Tombran-Tink J, Chader GG, Johnson LV (1991). PEDF: a pigment epithelium-derived factor with potent neuronal differentiative activity. Exp. Eye Res., vol. 53(3), PP: 411-414.
49. Tombran-Tink J, Johnson LV (1989). Neuronal differentiation of retinoblastoma cells induced by medium conditioned by human RPE cells. Invest. Ophthalmol. Vis. Sci., vol. 30(8), PP: 1700-1707.
50. Subramanian P, Locatelli-Hoops S, Kenealey J, DesJardin J, Notari L, Becerra SP (2013). Pigment epithelium-derived factor (PEDF) prevents retinal cell death via PEDF receptor (PEDF-R). J. Biol. Chem., vol. 288(33), PP: 23928-23942.
51. Kenealey J, Subramanian P, Comitato A, Bullock J, Keehan L, Polato F, Hoover D, Marigo V, Becerra SP (2015). Small retinoprotective peptides reveal a receptor-binding region on pigment epithelium-derived factor. J. Biol. Chem., vol. 290(42), PP: 25241-25253.
52. Filleur S, Volz K, Nelius T, Mirochnik Y, Huang H, Zaichuk TA, Aymerich MS, Becerra SP, Yap R, Veliceasa D, Shroff EH, Volpert OV (2005). Two functional epitopes of pigment epithelial-derived factor block angiogenesis and induce differentiation in prostate cancer. Cancer Res., vol. 65(12), PP: 5144-5152.
53. Tombran-Tink J, Barnstable CJ (2003). PEDF: a multifaceted neurotrophic factor". Nature Reviews. Neuroscience, vol. 4(8), PP: 628-636.
54. Dawson DW, Volpert OV, Gillis P, Crawford SE, Xu H, Benedict W, Bouck NP ( 1999). Pigment epithelium-derived factor: a potent inhibitor of angiogenesis. Science, vol. 285(5425), PP: 245-248.
55. Cao W, Tombran-Tink J, Chen W, Mrazek D, Elias R, McGinnis JF (1999). Pigment epithelial-derived factor protects cultured retinal neurons against hydrogen peroxide-induced cell death. J. Neurosci. Res., vol. 57, PP: 789-800.
56. Cao W, Tombran-Tink J, Elias R, Sezate S, Mrazek D, McGinnis JF (2001). In vivo protection of photoreceptors from light damage by pigment epithelium-derived factor. Invest. Ophthamol. Vis. Sci., vol. 42, PP: 1646-1652.
57. Aymerich MS, Alberdi EM, Martinez A, Becerra SP (2001). Evidence for the pigment epithelial-derived factor receptors in the neural retina. Invest. Ophthamol. Vis. Sci., vol. 42(13), PP: 3287-3293.
58. Wang X, Liu X, Ren Y, Liu Y, Han S, Zhao J, Gou X, He Y (2019). PEDF protects human retinal pigment epithelial cells against oxidative stress via upregulation of UCP2 expression. Mol. Med. Report, vol. 19, PP: 59-74.
59. Callis, J., Fromm, M., and Walbot, V. (1987). Introns increase gene expression in cultured maize cells. Genes Dev. 1, 1183-1200.
60. Rose AB (2019). Introns as gene regulators: a brick on the accelerator. Front. Genet., vol. 9; 672.
61. Haut DD, Pintel DJ (1998). Intron definition is required for excision of the minute virus of mice small intron and definition of the upstream exon. J. Virol., vol. 72(3), PP: 1834-1843.
62. Samulski RJ, Chang L-S, Shenk T (1987). A recombinant plasmid from which an infectious adeno-associated virus genome can be excised in vitro and its use to study viral replication. J. Virol., vol., 61(10), PP: 3096-3101.
63. Li C, Samulski RJ (2020). Engineering adeno-associated virus vectors for gene therapy. Nat. Rev. Genetics, vol. 21, PP: 255-272.
64. Fisher KJ, Gao GP, Weiitzman MD, DeMatteo R, Burda. JF, Wilson JM (1996). Transduction with recombinant adeno-associated virus for gene therapy is limited by leading-strand synthesis. J. Virol., vol. 70(1), PP: 520-532.
65. Thompson JD, Higgins DG, Gibson TJ (1994). CLUSTAL W: improving the sensitivity of progressive multiple sequence alignment through sequence weighting, position-specific gap penalties and weight matric choice. Nucleic Acids Res., 22(22), 4673-4680.
66. Thompson JD, Gibson TJ, Plewniak F, Jeanmougin F, Higgins DG (1997). The CLUSTAL_X windows interface: flexible strategies for multiple sequence alignment aided by quality analysis tools. Nucleic Acids Res., 25(24), 4876-4882

### Clauses

1. A genetic construct comprising a promoter operably linked to a first coding sequence, which encodes an agonist of the PEDF receptor, and a second coding sequence, which encodes an anti-complement protein.
2. A genetic construct according to clause 1, wherein the promoter is the cytomegalovirus (CMV) promoter, a fusion of the CMV early enhancer element and the first intron of chicken beta-actin gene (CAG), the vitelliform macular dystrophy protein-2 (VMD2) promoter, the human phosphoglycerate kinase-1 (PGK-1) promoter, or the EF1α promoter, optionally wherein the promoter comprises a nucleotide sequence substantially as set out in SEQ ID No: 1, 2, 3, 4, 5, 6 7, 8 or 9, or a fragment or variant thereof.
3. A genetic construct according to either clause 1 or clause 2, wherein the first coding sequence comprises a nucleotide sequence encoding a PEDF protein.
4. A genetic construct according to any preceding clause, wherein the first coding sequence comprises a nucleotide sequence substantially as set out in any one of SEQ ID No: 11, 12 or 13, or a fragment or variant thereof, and/or wherein the first coding sequence encodes an amino acid sequence substantially as set out in SEQ ID No: 10, or a fragment or variant thereof.
5. A genetic construct according to any preceding clause, wherein the anti-complement protein is capable of neutralising or attenuating complement activation.
6. A genetic construct according to any preceding clause, wherein the anti-complement protein is capable of targeting the alternative pathway (AP) of the complement system, preferably wherein the anti-complement protein minimally affects the classical pathway (CP) and/or the lectin pathway (LP) of the complement system.
7. A genetic construct according to any preceding clause, wherein the anti-complement protein is an anti-C3b, anti-Bb or anti-C5 antibody, or antigen-binding fragment thereof, optionally wherein the anti-complement protein is a single chain variable fragment (SCVF).
8. A genetic construct according to any one of clauses 1-6, wherein the anti-complement protein is CD55, preferably soluble CD55 (sCD55).
9. A genetic construct according to any one of clauses 1-6, wherein the anti-complement protein is complement factor H related protein-1 (CFHR1).
10. A genetic construct according to any one of clauses 1-6, wherein the anti-complement protein is CD46, preferably soluble CD46 (sCD46).
11. A genetic construct according to any one of clauses 1-6, wherein the anti-complement protein is complement factor H-like protein 1 (CFHL1).
12. A genetic construct according to clause 7, wherein the second coding sequence comprises a nucleotide sequence substantially as set out in SEQ ID No: 15, 17 or 83, or a fragment or variant thereof, and/or wherein the second coding sequence encodes an amino acid sequence substantially as set out in SEQ ID No: 14, 16 or 82, or a fragment or variant thereof.
13. A genetic construct according to clause 8, wherein the second coding sequence comprises a nucleotide sequence substantially as set out in SEQ ID No: 19, or a fragment or variant thereof, and/or wherein the second coding sequence encodes an amino acid sequence substantially as set out in SEQ ID No: 18, or a fragment or variant thereof.
14. A genetic construct according to clause 9, wherein the second coding sequence comprises a nucleotide sequence substantially as set out in SEQ ID No: 21 or 22, or a fragment or variant thereof, and/or wherein the second coding sequence encodes an amino acid sequence substantially as set out in SEQ ID No: 20, or a fragment or variant thereof.
15. A genetic construct according to clause 10, wherein the second coding sequence comprises a nucleotide sequence substantially as set out in SEQ ID No: 24, or a fragment or variant thereof, and/or wherein the second coding sequence encodes an amino acid sequence substantially as set out in SEQ ID No: 23, or a fragment or variant thereof.
16. A genetic construct according to clause 11, wherein the second coding sequence comprises a nucleotide sequence substantially as set out in SEQ ID No: 81, or a fragment or variant thereof, and/or wherein the second coding sequence encodes an amino acid sequence substantially as set out in SEQ ID No: 80, or a fragment or variant thereof.
17. A genetic construct according to any preceding clause, wherein the genetic construct comprises a spacer sequence disposed between the first and second coding sequences, which spacer sequence encodes a peptide spacer that is configured to produce the PEDF receptor agonist and the anti-complement protein as separate molecules.
18. A genetic construct according to clause 17, wherein the spacer sequence comprises and encodes a viral peptide spacer sequence, most preferably a viral-2A peptide spacer sequence.
19. A genetic construct according to clause 18, wherein the viral-2A peptide spacer sequence comprises a F2A, E2A, T2A or P2A sequence.
20. A genetic construct according to any one of clauses 17-19, wherein the spacer sequence comprises a nucleotide sequence substantially as set out in SEQ ID No: 26, 28, 30 or 32, or a fragment or variant thereof, and/or wherein the spacer sequence encodes an amino acid sequence substantially as set out in SEQ ID No: 25, 27, 29 or 31, or a fragment or variant thereof.
21. A genetic construct according to any one of clauses 17-20, wherein the genetic construct comprises a viral-2A removal sequence, optionally wherein the viral-2A removal sequence is disposed 5' of the viral-2A sequence.
22. A genetic construct according to clause 21, wherein the viral-2A removal sequence is separated from the viral-2A peptide spacer sequence by a linker sequence comprising a tripeptide glycine-serine-glycine sequence (G-S-G).
23. A genetic construct according to either clause 21 or 22, wherein the viral-2A removal sequence is a furin recognition sequence, optionally wherein the viral-2A removal sequence encodes an amino acid sequence substantially as set out in SEQ ID No: 33, or a fragment or variant thereof.
24. A genetic construct according to clause 23, wherein the viral-2A removal sequence comprises a nucleotide sequence substantially as set out in SEQ ID No: 35 or 37, or a fragment or variant thereof, and/or wherein the viral-2A removal sequence encodes an amino acid sequence substantially as set out in SEQ ID No: 34 or 36, or a fragment or variant thereof.
25. A genetic construct according to either clause 21 or 22, wherein the viral-2A removal sequence is a gelatinase MMP-2 recognition sequence, optionally wherein the viral-2A removal sequence comprises a nucleotide sequence substantially as set out in SEQ ID No: 39, or a fragment or variant thereof, and/or wherein the viral-2A removal sequence encodes an amino acid sequence substantially as set out in SEQ ID No: 38, or a fragment or variant thereof.
26. A genetic construct according to either clause 21 or 22, wherein the viral-2A removal sequence is a renin recognition sequence, optionally wherein the viral-2A removal sequence comprises a nucleotide sequence substantially as set out in SEQ ID No: 41, or a fragment or variant thereof, and/or wherein the viral-2A removal sequence encodes an amino acid sequence substantially as set out in SEQ ID No: 40, or a fragment or variant thereof.
27. A genetic construct according to any preceding clause, wherein the genetic construct comprises a nucleotide sequence encoding Woodchuck Hepatitis Virus Post-transcriptional Regulatory Element (WPRE), optionally wherein the WPRE comprises a nucleic acid sequence substantially as set out in SEQ ID No: 42 or 43, or a fragment or variant thereof.
28. A genetic construct according to any preceding clause, wherein the genetic construct comprises a nucleotide sequence encoding a polyA tail, optionally wherein the polyA tail comprises a nucleic acid sequence substantially as set out in SEQ ID No: 44, 45 or 84, or a fragment or variant thereof.
29. A genetic construct according to any preceding clause, wherein the genetic construct comprises a nucleotide sequence encoding left and/or right Inverted Terminal Repeat sequences (ITRs), optionally wherein the left and/or right Inverted Terminal Repeats comprise a nucleic acid sequence substantially as set out in SEQ ID No: 46 or 47, or a fragment or variant thereof.
30. A genetic construct according to any preceding clause, wherein the genetic construct comprises a non-coding intron, optionally wherein the non-coding intron is located between the promoter and the first coding sequence.
31. A genetic construct according to clause 30, wherein the non-coding intron comprises a nucleic acid sequence substantially as set out in SEQ ID No: 48, 49 or 50, or a fragment or variant thereof.
32. A genetic construct according to any preceding clause, wherein the genetic construct comprises a signal peptide coding sequence, optionally wherein the signal peptide coding sequence comprises a nucleotide sequence substantially as set out in any one of SEQ ID No: 52 or 54, or a fragment or variant thereof, and/or wherein the signal peptide coding sequence encodes an amino acid sequence substantially as set out in SEQ ID No: 51 or 53, or a fragment or variant thereof.
33. A genetic construct according to any preceding clause, wherein the genetic construct encodes an amino acid sequence substantially as set out in SEQ ID No: 55, 57, 59, 61, 63, 65, 67, 69, 71, 85, 87 or 89, or a fragment or variant thereof, and/or wherein the construct comprises a nucleotide sequence substantially as set out in SEQ ID No: 56, 58, 60, 62, 64, 66, 68, 70, 72, 86, 88 or 90, or a fragment or variant thereof.
34. A recombinant vector comprising the genetic construct according to any one of clauses 1-33.
35. A recombinant vector according to clause 34, wherein the vector is a recombinant AAV (rAAV) vector.
36. A recombinant vector according to clause 35, wherein the rAAV is AAV-1, AAV-2, AAV-2.7m8, AAV-3A, AAV-3B, AAV-4, AAV-5, AAV-6, AAV-7, AAV-8, AAV-9, AAV-10 or AAV-11.
37. A recombinant vector according to clause 36, wherein the rAAV is rAAV serotype-2.
38. A recombinant vector according to any one of clauses 34-37, wherein the recombinant vector comprises a nucleotide sequence substantially as set out in SEQ ID No: 67, or a fragment or variant thereof.
39. The genetic construct according to any one of clauses 1-33, or the recombinant vector according to any one of clauses 34-38, for use as a medicament or in therapy.
40. The genetic construct according to any one of clauses 1-33, or the recombinant vector according to any one of clauses 34-38, for use in treating, preventing or ameliorating a retinal disorder, or for reducing complement activation and retinal cell damage and loss.
41. The genetic construct or vector, for use according to clause 40, wherein the retinal disorder that is treated is: dry age-related macular degeneration, geographic atrophy, and/or any pathophysiological condition which involves retinal damage through complement activation.
42. The genetic construct or vector, for use according to clause 41, wherein the retinal disorder is dry age-related macular degeneration.
43. The genetic construct or vector, for use according to clause 41, wherein the retinal disorder is geographic atrophy.
44. The genetic construct or vector, for use according to clause 40, wherein the construct or vector is used to reduce complement activation and retinal cell damage and loss associated with any one of the following conditions: retinitis pigmentosa, Stargardt disease, diabetic macular degeneration, age-related macular degeneration, and/or Leber's congenital amaurosis.
45. A pharmaceutical composition comprising the genetic construct according to any one of clauses 1-33, or the recombinant vector according to any one of clauses 34-38, and a pharmaceutically acceptable vehicle.
46. A method of preparing the pharmaceutical composition according to clause 45, the method comprising contacting the genetic construct according to any one of clauses 1-33, or the recombinant vector according to any one of clauses 34-38, with a pharmaceutically acceptable vehicle.
47. The genetic construct or vector, for use according to clause 40, wherein the construct or vector is used to reduce complement activation and retinal cell damage and loss associated with glaucoma.

## Claims

1. A genetic construct comprising a promoter operably linked to a first coding sequence, which comprises a nucleotide sequence encoding a PEDF protein, and a second coding sequence, which encodes an anti-complement protein, wherein the anti-complement protein is CD46.

2. A genetic construct according to claim 1, wherein:
(i) CD46 is soluble CD46 (sCD46);
(ii) the promoter is the cytomegalovirus (CMV) promoter, a fusion of the CMV early enhancer element and the first intron of chicken beta-actin gene (CAG), the vitelliform macular dystrophy protein-2 (VMD2) promoter, the human phosphoglycerate kinase-1 (PGK-1) promoter, or the EF1α promoter, optionally wherein the promoter comprises a nucleotide sequence substantially as set out in SEQ ID No: 1, 2, 3, 4, 5, 6 7, 8 or 9, or a fragment or variant thereof;
(iii) the first coding sequence comprises a nucleotide sequence substantially as set out in any one of SEQ ID No: 11, 12 or 13, or a fragment or variant thereof, and/or wherein the first coding sequence encodes an amino acid sequence substantially as set out in SEQ ID No: 10, or a fragment or variant thereof; and/or
(iv) the second coding sequence comprises a nucleotide sequence substantially as set out in SEQ ID No: 24, or a fragment or variant thereof, and/or wherein the second coding sequence encodes an amino acid sequence substantially as set out in SEQ ID No: 23, or a fragment or variant thereof.

3. A genetic construct according to claim 1 or claim 2, wherein the genetic construct comprises a spacer sequence disposed between the first and second coding sequences, which spacer sequence encodes a peptide spacer that is configured to produce the PEDF protein and the anti-complement protein as separate molecules,
optionally wherein the spacer sequence comprises and encodes a viral peptide spacer sequence, most preferably a viral-2A peptide spacer sequence,
optionally wherein the viral-2A peptide spacer sequence comprises a F2A, E2A, T2A or P2A sequence.

4. A genetic construct according to claim 3, wherein:
(i) the spacer sequence comprises a nucleotide sequence substantially as set out in SEQ ID No: 26, 28, 30 or 32, or a fragment or variant thereof, and/or wherein the spacer sequence encodes an amino acid sequence substantially as set out in SEQ ID No: 25, 27, 29 or 31, or a fragment or variant thereof; and/or
(ii) the genetic construct comprises a viral-2A removal sequence, optionally wherein the viral-2A removal sequence is disposed 5' of the viral-2A sequence,
optionally wherein the viral-2A removal sequence is separated from the viral-2A peptide spacer sequence by a linker sequence comprising a tripeptide glycine-serine-glycine sequence (G-S-G).

5. A genetic construct according to claim 4, wherein:
(i) the viral-2A removal sequence is a furin recognition sequence, optionally wherein the viral-2A removal sequence encodes an amino acid sequence substantially as set out in SEQ ID No: 33, or a fragment or variant thereof,
optionally wherein the viral-2A removal sequence comprises a nucleotide sequence substantially as set out in SEQ ID No: 35 or 37, or a fragment or variant thereof, and/or wherein the viral-2A removal sequence encodes an amino acid sequence substantially as set out in SEQ ID No: 34 or 36, or a fragment or variant thereof;
(ii) the viral-2A removal sequence is a gelatinase MMP-2 recognition sequence, optionally wherein the viral-2A removal sequence comprises a nucleotide sequence substantially as set out in SEQ ID No: 39, or a fragment or variant thereof, and/or wherein the viral-2A removal sequence encodes an amino acid sequence substantially as set out in SEQ ID No: 38, or a fragment or variant thereof; or
(iii) the viral-2A removal sequence is a renin recognition sequence, optionally wherein the viral-2A removal sequence comprises a nucleotide sequence substantially as set out in SEQ ID No: 41, or a fragment or variant thereof, and/or wherein the viral-2A removal sequence encodes an amino acid sequence substantially as set out in SEQ ID No: 40, or a fragment or variant thereof.

6. A genetic construct according to any preceding claim, wherein:
(i) the genetic construct comprises a nucleotide sequence encoding Woodchuck Hepatitis Virus Post-transcriptional Regulatory Element (WPRE), optionally wherein the WPRE comprises a nucleic acid sequence substantially as set out in SEQ ID No: 42 or 43, or a fragment or variant thereof;
(ii) the genetic construct comprises a nucleotide sequence encoding a polyA tail, optionally wherein the polyA tail comprises a nucleic acid sequence substantially as set out in SEQ ID No: 44, 45 or 84, or a fragment or variant thereof;
(iii) the genetic construct comprises a nucleotide sequence encoding left and/or right Inverted Terminal Repeat sequences (ITRs), optionally wherein the left and/or right Inverted Terminal Repeats comprise a nucleic acid sequence substantially as set out in SEQ ID No: 46 or 47, or a fragment or variant thereof; and/or
(iv) the genetic construct comprises a non-coding intron, optionally wherein the non-coding intron is located between the promoter and the first coding sequence,
optionally wherein the non-coding intron comprises a nucleic acid sequence substantially as set out in SEQ ID No: 48, 49 or 50, or a fragment or variant thereof.

7. A genetic construct according to any preceding claim, wherein:
(i) the genetic construct comprises a signal peptide coding sequence, optionally wherein the signal peptide coding sequence comprises a nucleotide sequence substantially as set out in any one of SEQ ID No: 52 or 54, or a fragment or variant thereof, and/or wherein the signal peptide coding sequence encodes an amino acid sequence substantially as set out in SEQ ID No: 51 or 53, or a fragment or variant thereof; and/or
(ii) the genetic construct encodes an amino acid sequence substantially as set out in SEQ ID No: 55, 57, 59, 61, 63, 65, 67, 69, 71, 85, 87 or 89, or a fragment or variant thereof, and/or wherein the construct comprises a nucleotide sequence substantially as set out in SEQ ID No: 56, 58, 60, 62, 64, 66, 68, 70, 72, 86, 88 or 90, or a fragment or variant thereof.

8. A recombinant vector comprising the genetic construct according to any one of claims 1-7.

9. A recombinant vector according to claim 8, wherein:
(i) the vector is a recombinant AAV (rAAV) vector,
optionally wherein the rAAV is AAV-1, AAV-2, AAV-2.7m8, AAV-3A, AAV-3B, AAV-4, AAV-5, AAV-6, AAV-7, AAV-8, AAV-9, AAV-10 or AAV-11,
optionally wherein the rAAV is rAAV serotype-2; and/or
(ii) the recombinant vector comprises a nucleotide sequence substantially as set out in SEQ ID No: 67, or a fragment or variant thereof.

10. The genetic construct according to any one of claims 1-7, or the recombinant vector according to claim 8 or claim 9, for use as a medicament or in therapy.

11. The genetic construct according to any one of claims 1-7, or the recombinant vector according to claim 8 or claim 9, for use in treating, preventing or ameliorating a retinal disorder, or for reducing complement activation and retinal cell damage and loss.

12. The genetic construct or vector, for use according to claim 11, wherein:
(i) the retinal disorder that is treated is: dry age-related macular degeneration, geographic atrophy, and/or any pathophysiological condition which involves retinal damage through complement activation, optionally
wherein the retinal disorder is dry age-related macular degeneration, or
wherein the retinal disorder is geographic atrophy;
(ii) the construct or vector is used to reduce complement activation and retinal cell damage and loss associated with any one of the following conditions: retinitis pigmentosa, Stargardt disease, diabetic macular degeneration, age-related macular degeneration, and/or Leber's congenital amaurosis; or
(iii) the genetic construct or vector is administered by intravitreal, suprachoroidal or subretinal injection.

13. A pharmaceutical composition comprising the genetic construct according to any one of claims 1-7, or the recombinant vector according to claim 8 or claim 9, and a pharmaceutically acceptable vehicle.

14. A method of preparing the pharmaceutical composition according to claim 13, the method comprising contacting the genetic construct according to any one of claims 1-7, or the recombinant vector according to claim 8 or claim 9, with a pharmaceutically acceptable vehicle.

15. The genetic construct or vector, for use according to claim 12, wherein the construct or vector is used to reduce complement activation and retinal cell damage and loss associated with glaucoma.
